(19) Europäisches Patentamt
European Patent Office
Office européen des brevets

(11) EP 3 531 131 A1

(12) **EUROPEAN PATENT APPLICATION**
published in accordance with Art. 153(4) EPC

(43) Date of publication:
**28.08.2019 Bulletin 2019/35**

(21) Application number: **17864143.7**

(22) Date of filing: **13.10.2017**

(51) Int Cl.:
***G01N 33/543*** (2006.01) ***G01N 27/327*** (2006.01)
***G01N 27/414*** (2006.01)

(86) International application number:
**PCT/JP2017/037226**

(87) International publication number:
**WO 2018/079314 (03.05.2018 Gazette 2018/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.10.2016 JP 2016207707**
**29.05.2017 JP 2017105259**

(71) Applicant: **Toray Industries, Inc.**
**Tokyo 103-8666 (JP)**

(72) Inventors:
- **NAITO, Kojiro**
**Otsu-shi**
**Shiga 520-8558 (JP)**
- **NAGAO, Kazumasa**
**Otsu-shi**
**Shiga 520-8558 (JP)**
- **MURASE, Seiichiro**
**Otsu-shi**
**Shiga 520-8558 (JP)**

(74) Representative: **Hoefer & Partner Patentanwälte mbB**
**Pilgersheimer Straße 20**
**81543 München (DE)**

# (54) SEMICONDUCTOR SENSOR AND MANUFACTURING METHOD FOR SAME, AND COMPOUND SENSOR

(57) In order to selectively detect a target substance in high sensitivity, a semiconductor sensor includes a substrate, a first electrode, a second electrode, and a semiconductor layer located between the first electrode and the second electrode, in which the semiconductor layer includes a semiconducting component and a substructure of immunoglobulin, and the substructure of immunoglobulin is bonded or attached to the semiconducting component through a linking group $L^1$ in a hinge region of a heavy chain. Moreover, a method for producing a semiconductor sensor includes a step of forming the semiconductor layer, the step including a step of applying a semiconducting component between the first electrode and the second electrode.

FIG.3

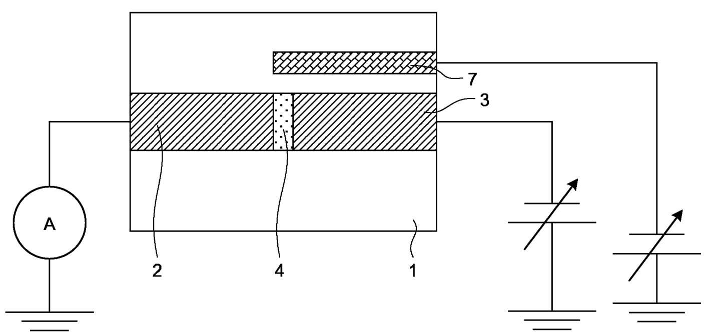




Printed by Jouve, 75001 PARIS (FR)

## Description

### Field

The present invention relates to a semiconductor sensor onto which target recognition molecules are immobilized, a method for producing the same, and a combined sensor.

### Background

In recent years, requirement for early detection of diseases and confirmation of therapeutic effects conducted by a sensor that detects biological substances with high sensitivity has been increasing more and more. In order to satisfy this requirement, development of biosensors for detecting biological substances has been actively carried out.

In the development of the biosensor, a biological substance selectively interacting with a target substance is generally arranged in a detection part of the sensor in order to selectively detect the target substance. As such a biological substance, for example, a protein such as immunoglobulin (also referred to as an "antibody") and an enzyme, a nucleic acid (also referred to as a "polynucleotide"), an aptamer, or a sugar chain is used. Of various substances selectively interacting with the target substance, the substructure of immunoglobulin (for example, Fab) that is small and can arrange the binding site to capture the target substance at a position closer to the semiconducting component in higher density has attracted attention (for example, refer to Non Patent Literature 1). As a method for applying immunoglobulin or the substructure of immunoglobulin to the biosensor, various methods exist. Examples of the application include a fluid pressure sensor, a photochemical reaction sensor, and an electrochemical reaction sensor utilizing immunoglobulin (for example, refer to Patent Literature 1).

In addition, among sensors using biological substances selectively interacting with the target substance, there is a field effect transistor (FET)-type sensor as a sensor which has particularly attracted attention (for example, refer to Patent Literature 2 and Patent Literature 3). The FET-type sensor is a sensor for detecting the target substance by change in the current flowing through a semiconductor or the voltage applied to a semiconductor. In this detection method, labeling of the target substance by the fluorescent material or the like is not required. In addition, the method also has an advantage that conversion of electrical signals is fast and connection to the integrated circuit is easy.

### Citation List

#### Patent Literature

Patent Literature 1: Published Japanese Translation of PCT International Publication for Patent Application No. H2-501860
Patent Literature 2: Japanese Laid-open Patent Publication No. 2017-9612
Patent Literature 3: WO 2006/103872

#### Non Patent Literature

Non Patent Literature 1: Japanese Journal of Applied Physics (Japan), 2012, Vol. 51, p. 06FD08-1-06FD08-4

### Summary

#### Technical Problem

In the conventional FET type biosensor, however, detection sensitivity and detection selectivity are insufficient and thus the conventional FET type biosensor has not been put to practical use.

The present invention has been made in view of the above problems, and an object of the present invention is to provide a semiconductor sensor having excellent detection sensitivity and detection selectivity, a method for producing the same, and a combined sensor.

#### Solution to Problem

To solve the problem described above and to achieve the object, a semiconductor sensor according to an embodiment of a first invention group includes: a substrate; a first electrode; a second electrode; and a semiconductor

layer formed between the first electrode and the second electrode. The semiconductor layer comprises a semiconducting component and a substructure of immunoglobulin, and the substructure of immunoglobulin is bonded or attached to the semiconducting component through a linking group $L^1$ in a hinge region of a heavy chain.

A semiconductor sensor according to an embodiment of a second invention group includes: a substrate; a first electrode; a second electrode; and a semiconductor layer located between the first electrode and the second electrode. The semiconductor layer includes a semiconducting component to which target recognition molecules are bonded or attached, the target recognition molecule includes at least a target capture body X and a linking group $L^2$, the target capture body X is a protein or nucleic acid having a molecular weight of 20,000 or higher and 200,000 or lower, and number of atom(s) N from the atom bonded to the semiconducting component or from the atom bonded to the group attached to the semiconducting component to the atom bonded to the atom originated from the target capture body X in the linking group $L^2$ is 5 or more and 30 or less.

Advantageous Effects of Invention

According to the semiconductor sensor, the method for producing the same, and the combined sensor according to the present invention, a semiconductor sensor exhibiting high detection selectivity and high detection sensitivity can be obtained. In addition, according to the semiconductor sensor, the method for producing the same, and the combined sensor according to the present invention, variations in performance among semiconductor sensors can be reduced and the long-term storage stability of the semiconductor sensor can be improved.

Brief Description of Drawings

FIG. 1A is a schematic plan view illustrating a semiconductor sensor that is one embodiment of the present invention.
FIG. 1B is a schematic sectional view illustrating the semiconductor sensor that is one embodiment of the present invention.
FIG. 2A is a schematic plan view illustrating a semiconductor sensor that is one embodiment of the present invention.
FIG. 2B is a schematic sectional view illustrating the semiconductor sensor that is one embodiment of the present invention.
FIG. 3 is a schematic plan view illustrating a semiconductor sensor that is one embodiment of the present invention.
FIG. 4A is a schematic plan view illustrating a semiconductor sensor that is one embodiment of the present invention.
FIG. 4B is a schematic sectional view illustrating the semiconductor sensor that is one embodiment of the present invention.
FIG. 5A is a schematic plan view illustrating a semiconductor sensor that is one embodiment of the present invention.
FIG. 5B is a schematic sectional view illustrating the semiconductor sensor that is one embodiment of the present invention.
FIG. 6A is a schematic plan view illustrating a semiconductor sensor that is one embodiment of the present invention.
FIG. 6B is a schematic sectional view illustrating the semiconductor sensor that is one embodiment of the present invention.
FIG. 7A is a schematic view illustrating the structure of immunoglobulin.
FIG. 7B is a schematic view illustrating the structure of immunoglobulin.
FIG. 7C is a schematic view illustrating the structure of immunoglobulin.
FIG. 8A is a schematic view illustrating the substructure of immunoglobulin.
FIG. 8B is a schematic view illustrating the substructure of immunoglobulin.
FIG. 9A is a schematic view illustrating an example of a state in which a precursor of a target recognition molecule is attached on a semiconducting component.
FIG. 9B is a schematic view illustrating an example of a state in which a target recognition molecule is attached on a semiconducting component.

Description of Embodiments

Hereinafter, suitable embodiments of the semiconductor sensor, the method for producing the same, and the combined sensor including the semiconductor sensor according to the present invention will be described in detail. The present invention is not construed as being limited to the following embodiments and various modifications may be carried out in accordance with purposes and applications within the range that achieves the object of the present invention and does not depart from the scope of the present invention.

<Semiconductor sensor>

The semiconductor sensor according to an embodiment of a first invention group of the present invention includes a substrate, a first electrode, a second electrode, and a semiconductor layer located between the first electrode and the second electrode, in which the semiconductor layer includes a semiconducting component and a substructure of immunoglobulin. The substructure of immunoglobulin is bonded or attached to the semiconducting component through a linking group $L^1$ in the hinge region of a heavy chain.

The semiconductor sensor according to an embodiment of a second invention group of the present invention includes a substrate, a first electrode, a second electrode, and a semiconductor layer located between the first electrode and the second electrode, in which the semiconductor layer includes a semiconducting component to which target recognition molecules are bonded or attached. The target recognition molecule includes at least a target capture body X and a linking group $L^2$. The target capture body X is a protein or nucleic acid having a molecular weight of 20,000 or higher and 200,000 or lower. The number of atom(s) N from the atom bonded to the semiconducting component or from the atom bonded to the group attached to the semiconducting component to the atom bonded to the atom originated from the target capture body X in the linking group $L^2$ is 5 or more and 30 or less.

As a modification example of the semiconductor sensor according to the embodiment of the present invention, the semiconductor sensor preferably further includes a third electrode. In other words, the semiconductor sensor is a semiconductor sensor including the substrate, the first electrode, the second electrode, the third electrode, and the semiconductor layer located between the first electrode and the second electrode. In the semiconductor sensor according to the first invention group, the semiconductor layer includes the semiconducting component and the substructure of immunoglobulin and the substructure of immunoglobulin is bonded or attached to the semiconducting component through the linking group $L^1$ in the hinge region of the heavy chain. In the semiconductor sensor according to the second invention group, the semiconductor layer includes the semiconducting component to which the target recognition molecules are bonded or attached, the target recognition molecule includes at least a target capture body X and a linking group $L^2$, the target capture body X is a protein or a nucleic acid having a molecular weight of 20,000 or higher and 200,000 or lower, and the number of atom(s) N from the atom bonded to the semiconducting component or from the atom bonded to the group attached to the semiconducting component to the atom bonded to atom originated from the target capture body X in the linking group $L^2$ is 5 or more and 30 or less.

In any of the semiconductor sensors, the detection sensitivity can be improved by applying voltage to the semiconductor layer through the third electrode to change electrical characteristics of the semiconductor layer.

FIG. 1A, FIG. 2A, and FIG. 3 are schematic plan views illustrating semiconductor sensors that are first, second, and third embodiments according to the present invention, respectively. FIG. 1B and FIG. 2B are schematic sectional views taken along line AA' in FIG. 1A and line BB' in FIG. 2A, respectively.

As illustrated in FIG. 1A and FIG. 1B, in the semiconductor sensor according to the first embodiment, a first electrode 2 and a second electrode 3 are located on a substrate 1 and a semiconductor layer 4 connected to the first electrode 2 and the second electrode 3 is arranged between the first electrode 2 and the second electrode 3.

As illustrated in FIG. 2A and FIG. 2B, in the semiconductor sensor according to the second embodiment, a third electrode 5 and an insulating layer 6 are located on the substrate 1 and the first electrode 2 and the second electrode 3 are located on the insulating layer 6. The semiconductor layer 4 connected to the first electrode 2 and the second electrode 3 is arranged between the first electrode 2 and the second electrode 3. In the semiconductor sensor illustrated in FIG. 2A and FIG. 2B, the first electrode 2, the second electrode 3, and the third electrode 5 correspond to a source electrode, a drain electrode, and a gate electrode, respectively, and the insulating layer 6 corresponds to a gate insulating layer. Consequently, the semiconductor sensor has a function as a field effect transistor (FET).

As illustrated in FIG. 3, in the semiconductor sensor according to the third embodiment, the first electrode 2 and the second electrode 3 are located on the substrate 1. The semiconductor layer 4 connected to the first electrode 2 and the second electrode 3 is located between the first electrode 2 and the second electrode 3. A third electrode 7 is arranged apart from the first electrode 2 and the second electrode 3 on the substrate 1.

In a common FET, the current flowing between the source electrode and the drain electrode can be controlled by changing the gate voltage applied to the gate electrode. A mobility in an FET can be calculated using the following formula (a).

$$\mu = (\delta Id/\delta Vg) L \cdot D/(W \cdot \varepsilon_r \cdot \varepsilon \cdot Vsd) \qquad (a)$$

where Id represents the current between the source electrode and the drain electrode, Vsd the voltage between the source electrode and the drain electrode, Vg the gate voltage, D the thickness of the insulating layer, L the channel length, W the channel width, $\varepsilon_r$ the relative dielectric constant of the gate insulating layer, $\varepsilon$ the dielectric constant in a

vacuum (8.85 × $10^{-12}$ F/m), and δ the change amount of the corresponding physical quantity. In addition, an on/off ratio can be determined from the ratio of the maximum value of Id and the minimum value of Id, that is, the ratio of the maximum value of Id to the minimum value of Id.

The principle of the FET type sensor will be described below. In other words, the capture of the target substance serving as the detection target by the target recognition molecule causes change in the electric field in the semiconductor layer 4 of the FET due to the electric charge of the captured target substance. The target substance can be detected by detecting this change. Here, the term "target substance" used the present specification refers to a target substance that is captured by the target recognition molecules included in the sensor.

In the semiconductor sensor according to the first embodiment illustrated in FIG. 1A and FIG. 1B, a current value flowing through the semiconductor layer 4 between the first electrode 2 and the second electrode 3 or an electric resistance value is changed when the target substance, or a solution, gas, or solid including the target substance is arranged in the vicinity of the semiconductor layer 4. The target substance can be detected by measuring this change. In the first embodiment, although the gate electrode does not exist, change in electric potential in the semiconductor layer 4 caused by an electric field generated by an electric charge of the target substance may be regarded as δVg. Consequently, the semiconductor sensor according to the first embodiment illustrated in FIG. 1A and FIG. 1B is classified into the FET-type sensor.

In addition, in the semiconductor sensor according to the second embodiment illustrated in FIG. 2A and FIG. 2B, a current value flowing through the semiconductor layer 4 between the first electrode 2 and the second electrode 3 is also changed when the target substance, or a solution, gas, or solid including the target substance is arranged in the vicinity of the semiconductor layer 4. The target substance can be detected by measuring this change.

In addition, in the semiconductor sensor according to the third embodiment illustrated in FIG. 3, the current value flowing through the semiconductor layer 4 can be controlled by the potential of the third electrode 7. Therefore, a two-dimensional graph (Vg-Id graph) is obtained by measuring the current value flowing between the first electrode 2 and the second electrode 3 (the current Id between the source electrode and the drain electrode) at the time of changing the potential of the third electrode 7 (the gate voltage Vg).

Characteristic values such as shift in Vg direction and shift in Id direction in the Vg-Id graph and a sub-threshold coefficient can be read from the Vg-Id graph. The target substance may be detected by using some or all of these characteristic values or the target substance may be detected by using the ratio of the maximum value to the minimum value of Id, that is, the on/off ratio. Moreover, known electrical characteristics obtained from the semiconductor element such as a resistance value, threshold voltage change, impedance, mutual conductance, and capacitance may be used.

The target substance may be used singly or in combination with other substances or solvents. In the case where the measurement target is a solution including the target substance, change in an electric field is transmitted to the semiconductor layer 4 through the solution by the electric charge of the target substance captured by the target recognition molecules. The principle that the information of the electric charge of the target substance is transmitted through the solution will be described below. Existence of a substance having an electric charge in the solution causes an electric double layer to be formed around the substance and the electric field around the semiconductor layer 4 to be changed through the electric double layer. Generally, the thickness of the electric double layer is about several nm and thus the size of the target recognition molecule is preferably several nm or smaller.

(Substrate)

The material used for the substrate is not particularly limited. Examples of the material include inorganic materials such as a silicon wafer, glass, and an alumina sintered product and organic materials such as an aliphatic polyester, a polyethylene terephthalate, a polybutylene terephthalate, a polycarbonate, a polysulfone, a polyether sulfone, a polyethylene, a polypropylene, a polystyrene, a polyphenylene sulfide, a polyparaxylene, a polyimide, a polyvinyl alcohol, a polyvinyl chloride, a polyvinylidene fluoride, a polysiloxane, a polyvinylphenol, and a polyaramid, or mixtures of inorganic material powder and the organic material. These materials may be used singly or may be used by mixing or laminating a plurality of materials selected from these materials.

The surface of the substrate 1 may be processed. In the case where the semiconductor sensor according to the embodiment of the present invention is used for the detection of the target substance in a solution, attachment of the target substances such as proteins and other substances in the measurement sample solution to the substrate 1 can be reduced by processing the surface of the substrate 1. Attachment of the target substance to the substrate 1 instead of the detection unit reduces the concentration of the target substance in the solution and thus correct measurement values cannot be determined. In addition, attachment of substances other than the target substance to the substrate 1 in the vicinity of the detection unit causes the measured value to be disturbed due to the electric charge of the substance and thus correct measurement values cannot be determined.

As a processing method, for example, a method for providing hydrophilic groups having no electric charge such as oligoethylene glycol chains or oligo(3,4-dihydroxyphenylalanine) and hydrophilic groups having both positive charge

and negative charge such as phosphorylcholine groups to the surface of the substrate 1 is preferable.

(Electrodes)

Examples of the material used in the first electrode 2, the second electrode 3, and the third electrode 5 or 7 include conductive metal oxides such as tin oxide, indium oxide, indium tin oxide (ITO); metals such as platinum, gold, silver, copper, iron, tin, zinc, aluminum, indium, chromium, lithium, sodium, potassium, cesium, calcium, magnesium, palladium, and molybdenum and alloys thereof; inorganic conductive substances such as copper iodide and copper sulfide; organic conductive substance such as a polythiophene, a polypyrrole, a polyaniline, and a complex of a polyethylene dioxythiophene and a polystyrene sulfonic acid; nano-carbon material such as carbon nanotubes (CNT) and graphene; and conductive carbon black. The material, however, is not limited to these examples. In the first electrode 2, the second electrode 3, and the third electrode 5 or 7, these materials may be used singly or may be used by mixing or laminating a plurality of materials selected from these materials.

In the case where these electrodes are used as electrodes in the semiconductor sensor, the material of the first electrode 2 and second electrode 3 is preferably selected from gold, silver, platinum, palladium, the organic conductive materials, and the nano-carbon materials from the viewpoint of stability to the aqueous solution to be contacted.

Each of the first electrode 2, the second electrode 3, and the third electrode 5 or 7 may be directly closely contacted to the substrate 1 or an adhesive layer may be located between the substrate 1 and each of the electrodes. In the case where the adhesive layer also serves as a role of the insulating layer 6, metallic or semiconducting materials may be used as the substrate 1.

The width, thicknesses, distances, and arrangements of the first electrode 2, the second electrode 3, and the third electrode 5 or 7 can be designed arbitrarily. The width of each of the electrodes 2, 3, 5, and 7 is preferably 1 μm or larger and to 1 mm or smaller. The thickness of each electrode 2, 3, 5, and 7 is preferably 1 nm or larger and 1 μm or smaller. The distance between the first electrode 2 and the second electrode 3 is preferably 1 μm or longer and 10 mm or shorter. The planar shape of each of the electrodes 2, 3, 5, and 7 is not limited to a rectangle. The planar shape may include curves and may be a comb-like shape and other shapes. In addition, the width and thickness of each of the electrodes 2, 3, 5, and 7 are not necessarily equal.

The distance between the third electrode 5 or 7 and the semiconductor layer 4 is preferably 100 nm or longer and 10 cm or shorter. Arrangement of the third electrode 5 or 7 is not limited to just above the substrate 1 and third electrode 5 or 7 may be an upper layer on another member arranged on the substrate 1. Examples of the arrangement include a constitution in which the third electrode 5 or 7 having a width of 100 μm and a thickness of 500 nm is arranged at a distance of 2 mm away from the semiconductor layer 4 may be exemplified. The arrangement, however, is not limited to this example. As illustrated in FIG. 2A, the third electrode 5 may be arranged so that the insulating layer 6 exists between the first electrode 2, the second electrode 3, and the semiconductor layer 4, and the third electrode 5. In addition, as illustrated in FIG. 3, the third electrode 7 may be arranged so that third electrode 7 exists in the same plane as the first electrode 2, the second electrode 3, and the semiconductor layer 4. In the semiconductor sensor illustrated in FIG. 3, the third electrode 7 is arranged in parallel to the second electrode 3. The third electrode 7, however, may be arranged vertically or in any angles other than the right angle. As another embodiment, the third electrode 7 may exist at a position where the third electrode 7 is arranged away from the plane in which the first electrode 2, the second electrode 3, and the semiconductor layer 4 exist.

In the case where the measurement target is a solution, the third electrode 5 or 7 may be in contact with the solution with which the semiconductor layer 4 is in contact. In this case, the third electrode 5 or 7 may be used as an electrode applying voltage to the semiconductor layer 4 through the solution or may be used as a reference electrode for regulating the difference in potential between the first electrode 2 and the solution or the second electrode 3 and the solution.

In addition, any one of a gas layer, a liquid layer, and a solid layer or a combination thereof may exist between the first electrode 2 and the third electrode 5 or 7, between the second electrode 3 and the third electrode 5 or 7, or between the semiconductor layer 4 and the third electrode 5 or 7 or the location between them may be in a vacuum.

(Insulating layer)

The material used for the insulating layer 6 is not particularly limited. Examples of the material include inorganic materials such as glass, silicon oxide, and alumina and organic materials such as a polyimide, a polyvinyl alcohol, a polyvinyl chloride, a polyethylene terephthalate, a polyvinylidene fluoride, a polysiloxane, and a polyvinyl phenol, or mixtures of inorganic material powder and the organic material.

The film thickness of the insulating layer 6 is preferably 10 nm or larger, more preferably 50 nm or larger, and further preferably 100 nm or larger. In addition, the film thickness of the insulating layer 6 is preferably 5 μm or smaller, more preferably 3 μm or smaller, and further preferably 1 μm or smaller. The thickness of the insulating layer 6 can be

measured by, for example, an atomic force microscope (AFM) or an ellipsometric method.

(Covering member)

In the semiconductor sensor according to the embodiment of the present invention, a covering member 8 that covers at least a part of the substrate 1 may be located on the substrate 1. FIG. 4A is a schematic plan view illustrating a first modification example according to the third embodiment of the present invention. FIG. 4B is a schematic sectional view taken along line CC' in FIG. 4A. As illustrated in FIG. 4A and FIG. 4B, the semiconductor sensor of the first modification example according to the third embodiment preferably includes the covering member 8 on the substrate 1. The covering member 8 forms an inner space 9 between the covering member 8 and the substrate 1. As illustrated in FIG. 4A, dotted lines i and j in the covering member 8 represent boundaries between the covering member 8 and the inner space 9 and the inner space 9 is located on the side of the substrate 1 at the part existing between the dotted lines i and j.

FIG. 5A is a schematic plan view illustrating a second modification example according to the third embodiment of the present invention. FIG. 5B is a schematic sectional views taken along line DD' in FIG. 5A. As illustrated in FIG. 5A and FIG. 5B, in the semiconductor sensor of the second modification example according to the third embodiment, the covering member 8 forming the inner space 9 so as to surround the semiconductor layer 4 is located on the substrate 1. In the semiconductor sensor according to the second modification example, the semiconductor layer 4 and the liquid including the target substance can be effectively contacted.

FIG. 6A is a schematic plan view illustrating a third modification example according to the third embodiment of the present invention. FIG. 6B is a schematic sectional views taken along line EE' in FIG. 6A. As illustrated in FIG. 6A and FIG. 6B, in the semiconductor sensor according to the third modification example, the first electrode 2 and the second electrode 3 are located on the substrate 1 and the semiconductor layer 4 connected to the first electrode 2 and the second electrode 3 is arranged between the first electrode 2 and the second electrode 3. In the semiconductor sensor according to the third modification example, the first electrode 2, the second electrode 3, and the semiconductor layer 4 arranged on the substrate 1 and the covering member 8 are arrange on the same side. The third electrode 7 on the covering member 8 is arranged on the part opposite to the semiconductor layer 4. The arrangement of the third electrode 7 on the covering member 8 is not limited to just above the semiconductor layer 4 and the arrangement may be diagonally upper side. In addition, the arrangement of the third electrode 7 is not necessarily limited to the upper part in the covering member 8 seen from the semiconductor layer 4 and the third electrode 7 may be located on the side surface. Dotted lines p and q in the covering member 8 illustrated in FIG. 6A represent boundaries between the covering member 8 and the inner space 9 and the inner space 9 is located on the side of the substrate 1 at the part existing between the dotted lines p and q in FIG. 6A. Any one of a gas layer, a liquid layer, and a solid layer or a combination layer thereof may exist in the inner space 9 between the first electrode 2, the second electrode 3, and the semiconductor layer 4 and the third electrode 7 or the location between them may be in a vacuum.

Examples of the material of the covering member 8 include inorganic materials such as a silicon wafer, silicone rubber, glass, and an alumina sintered product and organic materials such as a polyimide, a polyester, a polycarbonate, a polysulfone, a polyether sulfone, a polyethylene, a polyphenylene sulfide, and a polyparaxylene.

(Semiconductor layer)

The film thickness of the semiconductor layer 4 is not particularly limited and is preferably 1 nm or larger and 100 nm or smaller. The semiconductor layer 4 having the film thickness within this range allows change in electrical characteristics due to the interaction between the target recognition molecules and the target substance to be sufficiently taken out as an electric signal. The film thickness of the semiconductor layer 4 is more preferably 1 nm or larger and 50 nm or smaller and further preferably 1 nm or larger and 20 nm or smaller.

The thickness of the semiconductor layer 4 can be measured by known methods, for example, secondary ion mass spectrometry (SIMS) or AFM. In the case where the semiconductor layer 4 is not uniform, for example, in the case where the semiconducting component such as the nano-carbon material described below is used as the semiconductor layer 4 and the target recognition molecules sparsely exist in a state where the target recognition molecules are attached to the semiconducting component, the thickness of the nano-carbon material to which the target recognition molecules are attached is considered to be the thickness of the semiconductor layer 4.

As a method for forming the semiconductor layer 4, dry methods such as resistance heating evaporation, electron beams, sputtering, CVD, transfer from another substrate may be used. However, wet methods such as an application method are preferably used from the viewpoint of production cost and suitability for a large area. The application method includes a step of forming the semiconductor layer 4 by applying the semiconducting component. Specific examples of the application method include a spin coating method, a blade coating method, a slit die coating method, a screen printing method, a bar coater method, a casting method, a transfer printing method, an immersion and withdrawal

method, and an inkjet method. From these methods, a preferable method can be selected depending on the characteristics of the coating film to be obtained such as control of the coating film thickness and control of orientation. To the formed coating film, annealing treatment may be applied under atmospheric air, under reduced pressure, or under atmosphere in inert gases such as nitrogen ($N_2$) and argon (Ar).

(Target recognition molecule)

The target recognition molecule used in the present invention is bonded or attached to the semiconducting component in the semiconductor layer 4. In the present specification, the term "bond" used in the case where the target recognition molecule is bonded to the semiconducting component refers to the bond formed by sharing an electron pair between two atoms to energetically stabilize, that is, what is called a "covalent bond". In addition, the term "attach" used in the case where the target recognition molecule is attached to the semiconducting component refers to a state where dissimilar substances are in contact with each other and the substances are not easily separated due to intermolecular interaction. Examples of such intermolecular interaction include hydrophobic interaction, $\pi$-$\pi$ electron interaction, cation-$\pi$ interaction, and a plurality of types of electrostatic interaction, or a plurality of hydrogen bonds.

(Target capture body X)

Of the target recognition molecules, the target capture body X is a protein or a nucleic acid and captures the target substance by recognizing the shape of the target substance and the configuration of chargeable groups. In order to recognize the target substance, the target capture body X is required to have a size larger than a certain degree of size. From such a viewpoint, the molecular weight of the target capture body X is 20,000 or higher. In addition, in the FET-type sensor, as the distance between the semiconductor layer 4 and the target substance captured by the target recognition molecule becomes larger, change in the electric field due to the electric charge of the target substance becomes smaller. Consequently, the molecular weight of the target capture body X is 200,000 or lower from the viewpoint of obtaining effective detection sensitivity.

Examples of the protein include immunoglobulin, a receptor, an enzyme, a structural protein, a transport protein, a storage protein, or a motor protein. As the protein, the immunoglobulin, the substructure of immunoglobulin, and an enzyme are preferable from the viewpoint of high target recognition ability and high versatility. The immunoglobulin and the substructure of immunoglobulin are more preferable and the substructure of immunoglobulin is particularly preferable from the viewpoint of high detection sensitivity. Preferable examples of the substructure of immunoglobulin include a structure in which a partial structure including the target binding site of the immunoglobulin is taken out.

In the case where the target substance is a substance detected by the selective interaction with the target recognition molecule, the target substance is preferably detected by modifying the sensitive part of the sensor with the target recognition molecules. Consequently, at the time of producing the sensor, the target recognition molecules may be immobilized to the sensitive part of the sensor by exposing the semiconductor layer 4 to the solution into which the target recognition molecules are dissolved. In this case, the surface of the substrate 1 has been preferably adequately processed as described above. By this processing, the attachment of the target recognition molecules to the part other than the sensitive part can be reduced and the target recognition molecules are selectively immobilized to the sensitive part. As described above, the capture of the target substance by the target recognition molecules at the position other than the sensitive part is reduced and the target substance can be selectively detected at the sensitive part, resulting in improving the detection sensitivity.

(Linking group L)

In the first invention group, the substructure of immunoglobulin is bonded or attached to the semiconducting component through the linking group $L^1$. In addition, in the second invention group, the target recognition molecule includes the target capture body X and the linking group $L^2$ described above. The linking group $L^1$ and/or $L^2$ in the target recognition molecule is a group that links the bonded part or the attached part of the target recognition molecule and the semiconducting component and the substructure of immunoglobulin in the first invention group or the target capture body X in the second invention group. The length of the linking group $L^1$ and/or $L^2$ is preferably a length to such an extent that the target recognition molecules are anchored close to the semiconducting component, while the target recognition molecules ensure mobility. Impairing the mobility of the target recognition molecules results in losing the original function of the target recognition molecules of capturing the target substance. In addition, excessively long linking group leads to an excessive distance between the target substance and the semiconducting component when the target substance is captured by the target recognition molecules. As a result, the electric field generated by the electric charge of the target substance is attenuated before the electric field reaches the semiconducting component and thus the current value change in the semiconducting component caused by capturing the target substance on the semiconductor layer

4 becomes small. That is, the detection sensitivity is deteriorated.

Specifically, the number of atom(s) N from the atom bonded to the semiconducting component or from the atom bonded to the group attached to the semiconducting component to the atom bonded to the atom originated from the target capture body X, in particular the substructure of immunoglobulin, in the linking group $L^1$ and/or $L^2$ is 5 or more and 30 or less. The number of atom(s) N is more preferably 8 or more and further preferably 9 or more. In addition, the number of atom(s) N is more preferably 16 or less and further preferably 13 or less. In this range, mobility of the target capture body X, in particular the substructure of immunoglobulin, is sufficiently improved and the target capture body X can capture the target substance at a position closer to the semiconducting component. These effects allow sensitivity to be improved.

Such an effect is observed in a protein or nucleic acid having a molecular weight of 10,000 or higher, generally referred to as high molecular weight substance and is particularly observed in the target capture body having a molecular weight of 20,000 or higher.

In order to improve the mobility of the linking group in a sample solution, a bond having greater affinity to water is preferably contained in the linking group $L^1$ and/or $L^2$. Examples of such a bond include an ether bond, a thioether bond, an ester bond, an amide bond, a thioester bond, a dithioester bond, an acid anhydride bond, an imide bond, a urea bond, and a urethane bond. Of these bonds, from the viewpoint of stability and affinity to water, the linking group $L^1$ and/or $L^2$ preferably contains at least one structure selected from the group consisting of the ether bond, the ester bond, the amide bond, the imide bond, the urea bond, and the urethane bond and particularly preferably contains at least one structure selected from the group consisting of the ether bond, the amide bond, and the imide bond.

In addition, in order to prevent excessive folding of the linking group, the linking group $L^1$ and/or $L^2$ preferably contains a five-membered ring structure. Examples of such a five-membered ring structure include the following structures.

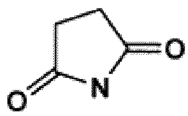

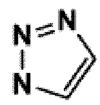

Moreover, the target recognition molecule including the linking group described above allows deterioration of performance of the sensor to be reduced when the sensor is stored for a long period of time. Leaving molecules having high molecular weight such as a protein and nucleic acid in a state of being immobilized with a short linking group for a long period of time causes gradual denaturation of the protein and the nucleic acid due to no escape of thermal energy. As a result, the function of the sensor is also deteriorated. The linking group described above retains the mobility of the protein and the nucleic acid and thus thermal energy is released as the motion of the linking group, resulting in lowering the degeneration rate of the protein or the nucleic acid.

Examples of a method for identifying a group attached to the semiconducting component include an isothermal titration calorimetry (ITC) method. Whether the compound corresponding to the noticed group and the semiconducting component are merely in a mixed state or in an attached state can be determined by measuring enthalpy change and entropy change at the time of mixing the compound corresponding to the noticed group and the semiconducting component. In the present specification, the group that is clearly found to be attached to the semiconducting component by ITC is determined to be a "group attached to the semiconducting component". In addition, the atom that is bonded to the group attached to the semiconducting component and belongs to the group that is bonded to the group attached to the semiconducting component and not attached alone to the semiconducting component is determined to be an "atom bonded to the group attached to the semiconducting component".

In the case where the semiconducting component is a nano-carbon material described below, examples of the group attached to the semiconducting component include an aromatic heterocyclic group and an aromatic hydrocarbon group described below. In addition, groups formed by linking a plurality of aromatic hydrocarbon groups or aromatic heterocyclic groups such as a pyrenylphenyl group may be also included.

In the first invention group, the linking group $L^1$ described above preferably has a substituted or unsubstituted aromatic hydrocarbon group and/or aromatic heterocyclic group. The linking group $L^1$ described above particularly preferably has a substituted or unsubstituted aromatic hydrocarbon group and the number of carbon atoms in the aromatic hydrocarbon group not containing the substituent is 14 or more and 22 or less.

In the first invention group, the hinge region of the substructure of immunoglobulin described above preferably contains a sulfur atom and the sulfur atom preferably forms a bond to the linking group $L^1$ described above. As the bond in the hinge region, the linking group $L^1$ described above and the hinge region of the substructure of immunoglobulin described above particularly preferably form a thioether bond through the sulfur atom.

In the case where the substructure of immunoglobulin is used as the target capture body X in the first invention group or the second invention group, examples of the bond between the substructure of immunoglobulin in the first

invention group or the target capture body X in the second invention group and the linking group $L^1$ and/or $L^2$ include a thioester bond, a dithioester bond, a sulfonic acid ester bond, a disulfide bonds, and a thioether bond. The disulfide bond or the thioether bond is preferable and the thioether bond is particularly preferable from the viewpoint of ease of bond formation and stability of the bond. That is, the linking group preferably contains the thioether bond.

Similarly, in the second invention group, the target capture body X is preferably the substructure of immunoglobulin and the substructure of immunoglobulin preferably forms a bond to the linking group $L^2$ in the hinge region of the heavy chain. This bonding form allows a binding site 13 for bonding to the target molecule to be oriented so as to facilitate contact with the target substance.

(Example of target recognition molecule)

Of the various target recognition molecules, the target recognition molecule is preferably a compound represented by a general formula (1) or a macromolecular compound having a structure represented by a general formula (2) as a repeating unit from the viewpoint that the target recognition molecule can be easily bonded or attached to the semiconducting component and that semiconductor characteristics are assisted by the conjugated system of the rings.

$$Ar^1\text{-}L^2\text{-}X \qquad (1)$$

In the general formula (1), $Ar^1$ is a substituted or unsubstituted aromatic heterocyclic group or a substituted or unsubstituted aromatic hydrocarbon group. $L^2$ is the linking group described above and has the number of atom(s) $N^1$ from the atom bonded to the atom originated from $Ar^1$ to the atom bonded to the atom originated from X of 5 or more and 30 or less. X is the target capture body described above and is made of a protein or nucleic acid having a molecular weight of 20,000 or higher and 200,000 or lower.

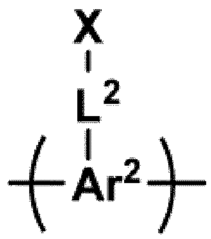

(2)

In the general formula (2), $Ar^2$ is a substituted or unsubstituted aromatic heterocyclic group or a substituted or unsubstituted aromatic hydrocarbon group. $L^2$ is a linking group described above and has the number of atom(s) $N^2$ from the atom bonded to the atom originated from $Ar^2$ to the atom bonded to the atom originated from X of 5 or more and 30 or less. X is a target capture body described above and is a protein or nucleic acid having a molecular weight of 20,000 or higher and 200,000 or lower.

The number of atom(s) N is determined to be counted along the shortest path from the atom bonded to the semiconducting component or the atom bonded to the group attached to the semiconducting component to the atom bonded to the atom originated from the target capture body X. At the time of counting the number of atoms from the atom bonded to the semiconducting component or the atom bonded to the group attached to the semiconducting component to the atom bonded to the atom originated from the target capture body X, the number of atoms in a ring structure part is determined to be counted along the shortest path in the ring structure part in the case where the ring structure exists in the shortest path. In addition, in the case where the target recognition molecule has a plurality of points bonded to or attached to the semiconducting component, the number of atoms is determined to be counted along the shortest path to reach the target capture body X among the paths from these points. The numbers of atom(s) $N^1$ and $N^2$ correspond to the number of atom(s) N and the counting method of atoms and the preferable range are also the same as those for the number of atom(s) N. The number of atom(s) $N^1$ and/or $N^2$ is particularly preferably 8 or more and 16 or less.

For example, in the target recognition molecule represented by the following structural formula, the bond between $Ar^1$ and X is connected so as to be $Ar^1$-C-C-C-N-C-C-C-C-X. Consequently, the number of carbon atoms from the atom bonded to atoms originated from $Ar^1$ (here, the carbon atom adjacent to $Ar^1$) to the atom bonded to the atom originated from X (here, the carbon atom next to X) is 8.

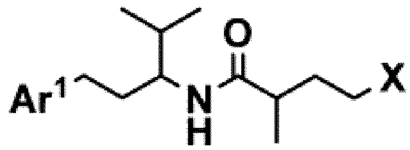

In addition, as the structural formula illustrated below, also in the case where the ring structure is included in the linking group between $Ar^1$ and X, the number of atoms from the atom bonded to the atom originated from $Ar^1$ to the atom bonded to the atom originated from X is 7 by counting the number of atoms along the shortest path between $Ar^1$ and X.

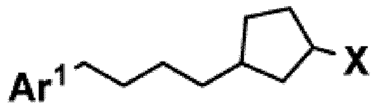

In addition, the following structural formula is one example of the target recognition molecule in which $Ar^1$ is pyrene. This molecule is considered to be attached to the semiconducting component with a plurality of pyrene rings. In this case, as described above, the number of atoms is counted from X to the bonded point or attached point that can be reached along the shortest path. In other words, in the following structural formula, the number of atoms from the atom bonded to the atom originated from $Ar^1$ to the atom bonded to the atom originated from X is 2.

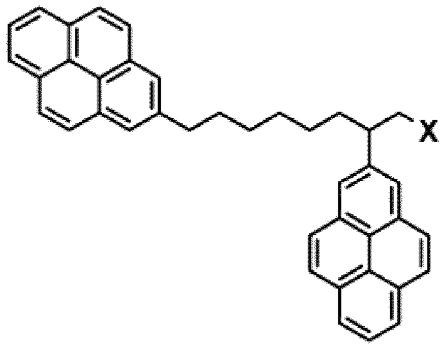

Linking group $L^2$ has a role of anchoring the target capture body X to the semiconducting component. The target capture body X generally has high water solubility and thus anchoring the target capture body X by the linking group $L^2$ is required so as not to diffuse into the solution.

In the compounds represented by the general formula (1) or in the macromolecular compound including the repeating unit structure represented by the general formula (2) described above, the target capture body X is preferably immobilized to $Ar^1$ or $Ar^2$ through the linking group $L^2$.

Generally, a method for immobilizing the target recognition molecule to the insulating layer 6 on the semiconductor layer 4, for example, to a silicon dioxide ($SiO_2$) layer on a silicon (Si) semiconductor layer has been known. In this immobilization method, however, the detection sensitivity is deteriorated by shielding an electric field generated by the target recognition molecule due to the insulating layer 6. Consequently, the target capture body X is preferably anchored to the semiconducting component.

Moreover, so as not to inhibit the conductivity of the semiconducting component, the target recognition molecules are preferably anchored to the semiconducting component by attachment.

$Ar^1$ and $Ar^2$ in the target recognition molecule have hydrophobic interaction with the semiconducting component and can be attached to the semiconducting component. In the case where the semiconducting component is a nano-carbon material, the $\pi$-$\pi$ electron interaction in addition to the hydrophobic interaction is added and thus the target recognition molecule can be particularly strongly and stably attached. In this case, the $\pi$-$\pi$ electron interaction is assumed to be interaction caused by overlapping the $\pi$ electron cloud of the aromatic heterocyclic group or the aromatic hydrocarbon group and the $\pi$ electron cloud of the nano-carbon material.

Examples of the aromatic heterocyclic group include a furanyl group, a benzofuranyl group, a dibenzofuranyl group, a thiophenyl group, a benzothiophenyl group, a dibenzothiophenyl group, an isothiazolyl group, a thiadiazolyl group, a thiazolyl group, a pyridinyl group, a quinolinyl group, a pyrimidinyl group, a pyrazinyl group, a triazinyl group, a quinoxalinyl group, a quinazolinyl group, an indolyl group, and a porphyrinil group. The thiophenyl group, the isothiazolyl group, the thiadiazolyl group, or the thiazolyl group is preferable and the thiophenyl group, or the thiadiazolyl group is

particularly preferable from the viewpoint of particularly strong interaction with the semiconducting component and stable attachment.

Examples of the aromatic hydrocarbon group include a fluorenyl group, a phenanthrenyl group, an anthracenyl group, a pyrenyl group, a triphenyl group, a para-terphenyl group, a benzo[a]anthracenyl group, a fluoranthenyl group, a dibenzo[a,h]anthracenyl group, a dibenzo[a,h]pyrenyl group, a 3,4-benzopyrenyl group, a chrysenyl group, a naphthacenyl group, a pentacenyl group, a naphtho[2,3-a]pyrenyl group, a corannulenyl group, and a coronenyl group. Of these groups, the phenanthrenyl group, the anthracenyl group, the pyrenyl group, the triphenylenyl group, the para-terphenyl group, the benzo[a]anthracenyl group, the fluoranthenyl group, the dibenzo[a,h]anthracenyl group, the 3,4-benzopyrenyl group, the chrysenyl group, the naphthacenyl group, the pentacenyl group, or the corannulenyl group is preferable from the viewpoint of easy handling. Of these groups, the group based on the pyrenyl group, the triphenylenyl group, or the fluoranthenyl group is preferable and the pyrenyl group is particularly preferable from the viewpoint of the particularly strong interaction with the semiconducting component and stable attachment.

$Ar^1$, $Ar^2$, and the linking group $L^2$ may further include a substituent. The substituent in the case where these groups have a substituent is preferably selected from the group consisting of an alkyl group, a cycloalkyl group, an alkenyl group, a cycloalkenyl group, an alkynyl group, an alkoxy group, an alkylthio group, an aryl ether group, an aryl thioether group, a halogen atom, a cyano group, an amino group, a mercapto group, a carbonyl group, a carboxy group, and an oxycarbonyl group. $Ar^1$, $Ar^2$, and linking groups $L^2$ may further include a substituted or unsubstituted aromatic heterocyclic group or aromatic hydrocarbon group or may include both substituted or unsubstituted aromatic heterocyclic group and aromatic hydrocarbon group. The substituent in the case where the aromatic heterocyclic group or the aromatic hydrocarbon group has a substituent is preferably selected from the group consisting of the substituents described above.

As the macromolecular compound including the repeating unit structure represented by the general formula (2), a polyphenylene, a polyphenylenevinylene, a polythiophene, a polyfluorene, a polythiadiazole, and a polybenzothiadiazole are preferable and a polythiophene, a polyfluorene, and a polybenzothiadiazole are particularly preferable from the viewpoint that the semiconducting component and the macromolecular compound have hydrophobic interaction in an aqueous solution and that the macromolecular compound retains a state of being stably bonded or attached to the semiconducting component.

From the viewpoint of stability of the compound, strength of interaction, and easy handling, the target recognition molecule is preferably the compound represented by the general formula (1) described above, $Ar^1$ is preferably a substituted or unsubstituted aromatic hydrocarbon group, and the number of carbon atoms of the aromatic hydrocarbon group not containing a substituent is preferably 14 or more and 22 or less. More specifically, $Ar^1$ is particularly preferably the pyrenyl group.

Specific examples of the preferable structure of the target recognition molecule include the following molecules. In the formulas, k is an integer of 1 or more and 24 or less and m is an integer of 1 or more and 9 or less.

(101)

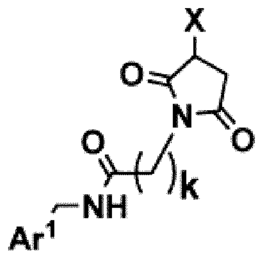

(102)

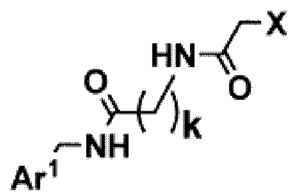

(103)

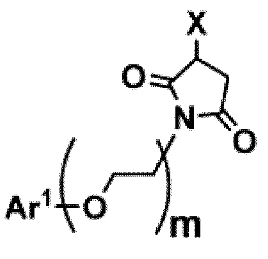

(104)

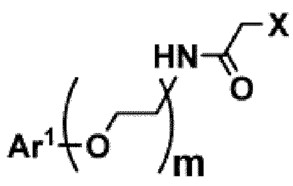

(Immunoglobulin)

Immunoglobulin is generally referred to as an antibody. Immunoglobulin that can be used in the present invention may include any types, classes, and subclasses. Examples of such immunoglobulin include IgG, IgE, IgM, IgD, IgA, IgY, IgG1, IgG2, IgG3, IgG4, IgA1, and IgA2. Of these types of immunoglobulin, IgG, IgE, and IgD are preferable and IgG, which is easily available, is particularly preferable from the viewpoint of existence as a monomer and easy handling.

IgG originated from any animals may be used. Example of IgG include IgG originated from crocodiles, ducks, humans, monkeys, goats, rabbits, sheep, cows, horses, dogs, cats, pigs, rats, mice, and dolphins. Of these types of IgG, IgG originated from goats, rabbits, sheep, or mice is more preferable from the viewpoint of higher immune system and easy availability.

In the present specification, as the description of the antibody, the originated animal and the target substance are described in this order. Specifically, for example, in the case where an antibody originated from a goat that selectively interacts with human hemoglobin, the antibody is referred to as a "goat anti-human hemoglobin antibody."

IgG used in the present invention may be IgG that is bonded to IgG of an animal different from the originated animal. Examples of IgG include a goat anti-rabbit IgG antibody, a rabbit anti-sheep IgG antibody, a goat antimouse IgG antibody, a mouse anti-goat IgG antibody, a goat anti-human IgG antibody, a rabbit anti-human IgG antibody, a sheep anti-human IgG antibody, or a mouse anti-human IgG antibody.

The structure of immunoglobulin will be described with reference to IgG being a monomer. IgA may be regarded as the dimer of IgG and IgM may be regarded as the pentamer of IgG.

As illustrated in FIG. 7A, in immunoglobulin 10, two polypeptide chains referred to as the heavy chains 11 and two polypeptide chains referred to as light chains 12 are linked by disulfide bonds to form one macromolecule.

As illustrated in FIG. 7B, in the immunoglobulin 10, regions referred to as the Fab regions 15 and a region referred to as the Fc region 16 exist. At the tip of the Fab regions 15, binding sites 13 for bonding to the target molecule exist.

As illustrated in FIG. 7B and FIG. 7C, in the boundary regions between the Fab regions 15 and the Fc region 16, two heavy chains 11 are linked each other by two disulfide bonds. This region is referred to as the hinge region 14. The hinge region 14 is a flexible amino acid sequence existing between the Fab regions 15 and the Fc region 16. In the present specification, even when the heavy chains 11 are separated from each other to eliminate the hinge region 14, the region where the hinge region 14 is formed is referred to as the hinge region for convenience.

Action of a proteolytic enzyme to the immunoglobulin 10 causes decomposition of the Fc region 16, and substructures made of the hinge region 14 and the Fab region 15 remain. This substructure is referred to as $F(ab')_2$. For this decomposition reaction, pepsin is generally used. Other proteolytic enzymes, however, may also be used if the reaction conditions are sufficiently studied.

Action of a reducing agent having a suitable reducing power to $F(ab')_2$ results in cutting the disulfide bond linking the heavy chains 11 to each other. As illustrated in FIG. 8A, this generates a substructure 17 made of the Fab region 15 including one light chain 12 and one heavy chain 11 and a part included in the hinge region 14. The substructure 17 is referred to as Fab'. As the reducing agent, an alkyl thiol, 2-mercaptoethylamine, or the like is used.

Action of Fab' to the functional group capable of reacting with a mercapto group allows a bond between Fab' and the functional group to be formed. In the semiconductor sensor according to the embodiment of the present invention, Fab' is bonded or attached to the semiconducting component through the linking group and thus this reaction can be used.

As another example, action of a reducing agent to the immunoglobulin 10 results in cutting the disulfide bond linking the heavy chains 11 to each other as illustrated in FIG. 8B. This generates a substructure 18 made of one light chain 12 and one heavy chain 11 and having mercapto groups in the hinge region 14. The substructure 18 is referred to as a "reduced immunoglobulin half-molecule" in the present specification. The reduced immunoglobulin half-molecule can also form a bond to the functional group by acting on the functional group capable of reacting with the mercapto group.

Here, the method for acquiring the substructure by processing the natural type immunoglobulin 10 has been described. The method for acquiring the substructure of immunoglobulin in the present invention is not limited thereto. Other methods may be used as long as substantially the same substructure can be obtained. Examples of other methods include a method for acquiring the substructure by introducing the vector encoding the Fab' and the reduced immunoglobulin half-molecule into *E. coli.*

The immunoglobulins 10 can be made smaller by the above processes. As a result, the electric field generated by the electric charge of the target substance can be effectively transmitted to the semiconductor layer 4, resulting in improving the detection sensitivity.

Either a monoclonal antibody or a polyclonal antibody may be used as the starting material of the substructure of immunoglobulin 10. From the viewpoint of reduction in characteristics fluctuation among sensors, the monoclonal antibody is preferable. Even in the case where the polyclonal antibody is used as the starting material, a specific antibody can be obtained by what is called an absorption method including a step of bonding the antibody to an affinity column bonding the purified polypeptide.

In the case where the monoclonal antibody is used as the starting material, a chimeric antibody (for example,

a human antibody having the variable region of a mouse monoclonal antibody) and a humanized antibody (for example, a human antibody having the complementarity determining region of a mouse monoclonal antibody) may be used as its antibody. In addition, the antibody may be an antibody introducing an intentional mutation in a part of immunoglobulin by using known genetic engineering techniques or protein engineering techniques.

The molecular weight of the substructure of immunoglobulin to be used also affects the performance of the sensor. As the molecular weight of the substructure is smaller, the advantage that the target substance can be captured near the semiconductor layer 4 and the advantage that the larger number of substructures can be introduced to the semiconductor layer 4 can be obtained. On the other hand, as the original immunoglobulin conformation is preserved as much as possible, binding capacity to the target substance is retained more. From the viewpoint of balancing these two effects, the molecular weight of the substructure of immunoglobulin used as the target capture body X is preferably 20,000 or higher, more preferably 30,000 or higher, and particularly preferably 35,000 or higher. In addition, the molecular weight of the substructure of immunoglobulin is preferably 120,000 or lower, more preferably 100,000 or lower, and particularly preferably 58,000 or lower.

The molecular weight of the substructure of immunoglobulin in the present invention is a value determined by analyzing a sample using matrix assisted laser desorption ionization-time-of-flight mass spectrometry (MALDI-TOFMS).

(Semiconducting component)

Examples of the semiconducting component that can be used in the present invention include element semiconductors, compound semiconductors, organic semiconductors, and nano-carbon materials.

As the element semiconductors, Group IV elements such as silicon and germanium are used. Examples of the form of silicon to be used include amorphous silicon, a polysilicon (polycrystalline silicon), and single crystal silicon. In the case where silicon semiconductor is used, the silicon semiconductor may be used in the form of a plate or in the form of a silicon nanowire. The silicon nanowire refers to a fibrous silicon semiconductor having a diameter of 1 nm or larger and 999 nm or smaller. As the diameter becomes narrower, the sensitivity becomes better because the silicon nanowire can sensitively feel the environment. Consequently, in the case where silicon nanowire is used, the silicon nanowire having a diameter of 1 nm or larger and 100 nm or smaller is preferable.

Examples of a method for producing the silicon nanowire include a solid phase method, a liquid phase method, a gas phase method, a chemical vapor deposition (CVD) method, a VLS (Vapor-Liquid-Solid) method, and a method by etching the substrate.

The compound semiconductor can be further classified into Group II-VI semiconductors, Group III-V semiconductors, Group IV compound semiconductors, Group I-III-VI semiconductors, and Group II-IV-V semiconductors. Examples of the Group II-VI semiconductors include ZnSe, CdS, and ZnO. Examples of the Group III-V semiconductors include GaAs, InP, and GaN. Examples of the Group IV compound semiconductors include SiC and SiGe. Examples of the Group I-III-VI semiconductors include $CuInSe_2$ and $AgGaTe_2$. Examples of the Group II-IV-V semiconductors include $ZnSiAs_2$ and $CdGeAs_2$.

Examples of the organic semiconductors include polycyclic aromatic hydrocarbons such as pentacene, anthracene, rubrene, perylene, coronene, and phthalocyanine and polymers such as a polythiophene, a polyparaphenylenevinylene, a polyacetylene, a polypyrrole, and a polyaniline.

Examples of the nano-carbon materials include fullerene, CNT, graphene, and carbon nanohorn. Each of the nano-carbon materials will be described below. In the present invention, the nano-carbon materials may be used in combination with each other. As an example, carbon peapod in which fullerene is encapsulated inside CNT may be exemplified.

Fullerene is a compound having a polyhedral structure formed by bonding carbon atoms to each other by $sp^2$ hybrid orbital interaction. The polyhedron is constituted of five-membered rings and six-membered rings. The number of the carbon atoms constituting the polyhedron is 60, 70, 74, 76, 78, or the like. Fullerene may be used as one molecule or may be used in the form of fullerene nanowhiskers formed by assembling a plurality of fullerene molecules or in the form of fullerene nanofibers prepared by forming a hollow structure in the fullerene nanowhiskers.

Graphene is also referred to as a graphene sheet. Ideally, all carbon atoms are bonded to each other with $sp^2$ hybrid orbital interaction and form a hexagonal lattice structure. Many stacked layers of graphene form graphite. Graphene is a special semiconductor that does not have band gap. Graphene exhibits extremely high electron mobility and thus is promising as a semiconducting component in the semiconductor layer 4.

The material made by laminating several carbon layers up to several atomic layers is also referred to as graphene. Graphene used in the present invention preferably includes carbon layers formed of 10 atomic layers or less and more preferably formed of 3 atomic layers or less, and is particularly preferably made of a single atomic layer.

A method for synthesizing graphene is not particularly limited. Examples of the method include a mechanical peeling method, a chemical peeling method, a silicon carbide heating method (hereinafter, referred to as a SiC heating method), or a thermal chemical vapor deposition method (hereinafter, referred to as a thermal CVD method).

Existence of graphene on the substrate 1 can be simply determined by an optical microscope. Observation by an optical microscope is a simple method and can distinguish a single layer, two layers, or three layers of graphene by careful observation. Raman spectroscopy is used for more detailed analysis.

Examples of CNT include single-walled CNT formed by winding one sheet of a carbon film (a graphene sheet) in a cylindrical shape, double-walled CNT formed by concentrically winding two sheets of graphene sheets, and multi-walled CNT formed by concentrically winding a plurality of graphene sheets. In the present invention, any types of CNT may be used. In order to obtain high semiconductor characteristics, the single-walled CNT is preferably used. CNT can be obtained by, for example, an arc discharge method, a chemical vapor deposition method (CVD method), and a laser ablation method.

CNT preferably includes semiconductor-type CNT in an amount of 80% by weight or larger. More preferably, CNT includes semiconductor-type CNT in an amount of 95% by weight or larger. As methods for obtaining CNT including semiconductor-type CNT in an amount of 80% by weight or larger, known methods can be used. Examples of the method include a method for carrying out ultracentrifugation in the co-existence of a density gradient material, a method for selectively attaching a specific compound onto the surface of semiconductor-type or metal-type CNT and separation by utilizing difference in solubility, and a method for separation by electrophoresis or the like utilizing difference in electric properties. The content of the semiconductor-type CNT in the present invention is a value calculated from the ratio of the peak area of metal-type CNT and the peak area of semiconductor-type CNT in the Raman spectrum.

In the present invention, the length of the CNT is preferably shorter than the distance between the first electrode 2 and the second electrode 3 in the sensor to be applied. Specifically, the average length of CNT depends on the channel length and is preferably 2 μm or shorter and more preferably 1 μm or shorter. The average length of the CNT refers to the average length of twenty CNT that are randomly selected. Examples of the method for measuring the CNT average length include a method for randomly selecting twenty CNT from the obtained image of, for example, an atomic force microscope, a scanning electron microscope, and a transmission electron microscope and determining the average length of the twenty CNT.

Generally, commercially available CNT has a distribution of length and may include longer CNT than the distance between the electrodes and thus the step of making the CNT shorter than the distance between the electrodes is preferably added. For example, a method for cutting the CNT into short fibers by acid treatment with, for example, nitric acid and sulfuric acid, ultrasonic treatment, a freeze pulverization method, or the like is effective. In addition, using the method together with separation by a filter is more preferable from the viewpoint of improving purity.

In addition, the diameter of CNT is not particularly limited and is preferably 1 nm or larger and 100 nm or smaller and more preferably 1 nm or larger and 50 nm or smaller.

In the present invention, a step of uniformly dispersing CNT in a solvent and filtering the dispersion liquid through a filter is preferably included. CNT shorter than the distance between the electrodes can be efficiently obtained by obtaining CNT smaller than the filter pore size from the filtrate. In this case, a membrane filter is preferably used as the filter. The pore size of the filter used for the filtration may be smaller than the channel length and is preferably 0.5 μm or longer and 10 μm or shorter. Examples of other method for shortening the length of CNT include acid treatment and freeze pulverization treatment.

The carbon nanohorn has a structure in which graphene is rounded in a conical shape. The carbon nanohorn can be synthesized by irradiating graphite with carbon dioxide laser at room temperature in an argon gas atmosphere. The diameter of the carbon nanohorn is preferably about 2 nm or larger and about 5 nm or smaller. The carbon nanohorn forms aggregates in the case where the carbon nanohorn is not subjected to a separation process. In the present invention, the aggregate may be used as they are or each carbon nanohorn may be used by separating the aggregate one by one.

In the sensor according to the embodiment of the present invention, at least one substance selected from the group consisting of fullerene, CNT, graphene, and carbon nanohorn is preferable as the semiconducting component from the viewpoint of large mobility and a large specific surface area. Of these substances, CNT is particularly preferable from the viewpoint of production cycle time.

In the sensor according to the embodiment of the present invention, the surface of the semiconducting component in the semiconductor layer 4 may be covered with an extremely thin film that does not exhibit semiconductor characteristics. This is because, at the time of direct exposure of the semiconducting component to a solution, the exposure may lead to unexpected change in electric characteristics. A conjugated organic compound may be attached on the extremely thin film covering the semiconducting component. The thickness of the film covering the semiconducting component, however, is preferably a film thickness of such an extent that the film does not inhibit the electrical detection of the sensor when the semiconductor element of the present invention is used as the sensor. Specifically, the film thickness is preferably 300 nm or smaller, more preferably 200 nm or smaller, and particularly preferably 100 nm or smaller.

(Disperse reagent)

In the case where a nano-carbon material composite is used as the semiconducting component, a disperse reagent is preferably attached to at least a part of the nano-carbon material surface. This allows the nano-carbon material to be uniformly dispersed in the solution without impairing the high electrical characteristics possessed by the nano-carbon material. In addition, a uniformly dispersed nano-carbon material film can be formed by a coating method from a solution in which the nano-carbon material is uniformly dispersed. This allows high semiconductor characteristics to be achieved.

Examples of the method for attaching the disperse reagent to the nano-carbon material include (I) a method for adding the nano-carbon material into the molten disperse reagent to mix the mixed system, (II) a method for dissolving the disperse reagent into a solvent and adding the nano-carbon material into this solution to mix the mixed system, (III) a method for previously pre-dispersing the nano-carbon material by, for example, ultrasonic wave and adding a disperse reagent thereto to mix the mixed system, and (IV) a method for adding the disperse reagent and the nano-carbon material into a solvent and irradiating the mixed system with ultrasonic wave to mix the mixed system. In the present invention, any one of these methods may be used or any methods may be used in combination.

The disperse reagent is not particularly limited. Specific examples of the disperse reagent include polyvinyl alcohol, celluloses such as carboxymethyl cellulose, polyalkylene glycols such as polyethylene glycol, acrylic resins such as polyhydroxymethyl methacrylate, conjugated polymers such as poly-3-hexylthiophene, polycyclic aromatic compounds such as anthracene derivatives and pyrene derivatives, and long-chain alkyl organic salts such as sodium dodecylsulfate and sodium cholate. The compound having a hydrophobic group such as an alkyl group and an aromatic hydrocarbon group or the compound having a conjugated structure is preferable and the conjugated polymer is particularly preferable from the viewpoint of interaction with the nano-carbon material. A conjugated polymer may further improve the effect of uniformly dispersing the nano-carbon material in a solution and the effect of providing high electrical characteristics without impairing high electrical characteristics possessed by the nano-carbon material.

(Conjugated polymer)

In the sensor according to the embodiment of the present invention, the conjugated polymer is preferably attached to at least a part of the semiconducting component. The term "attachment" as used herein refers to the same phenomenon as in the case where the target recognition molecule is attached to the semiconductor layer 4. The conjugated polymer is a polymer in which the conjugated systems of multiple bonds between atoms are continuously linked in a monomer unit or in a monomer unit and between adjacent monomer units. The conjugated polymer prevents an unexpected change in electrical characteristics due to direct touching of the semiconducting component to a sample solution and also plays a role of assisting the electron transfer of the semiconducting component by the conjugated system.

Examples of the conjugated polymers include polythiophene-based polymers, polypyrrole-based polymers, polyaniline-based polymers, polyacetylene-based polymers, poly-p-phenylene-based polymers, and poly-p-phenylenevinylene-based polymers. The conjugated polymers, however, are not particularly limited to these examples. As the polymer described above, polymers in which a single monomer unit is arranged are preferably used. However, polymers made by block copolymerization, random copolymerization or graft copolymerization of different monomer units are also used.

Of the polymers described above, the conjugated polymer having a heterocyclic ring containing a sulfur atom existing in the repeating unit is preferable from the viewpoint of the large electron orbital of the conjugation system and large interaction with the semiconducting component. Of these conjugated polymers, the thiophene-based polymer, the thiadiazole-based polymer, and the benzothiadiazole-based polymer are particularly preferable due to strong attachment to the semiconducting component and a high electron conductivity assisting effect.

The preferable molecular weight of the conjugated polymer is 800 or higher and 100,000 or lower in term is of the number average molecular weight. In addition, the polymers described above do not necessarily have a high molecular weight and may be an oligomer including a straight chain conjugated system.

In addition, the conjugated polymer preferably contains side chains. The side chain preferably contains at least one functional group selected from the group consisting of a hydroxy group, a carboxy group, an amino group, a mercapto group, a sulfo group, a phosphonic acid group and organic or inorganic salts thereof, a formyl group, a maleimide group, and a succinimide group and particularly preferably contains at least one functional group selected from the group consisting of an amide group, an ester group, and an imide group. These functional groups may be bonded to each other to form a ring.

In the present specification, the term "side chain" refers to a molecular chain containing at least one carbon atom linked by substituting the atom constituting the main chain of the conjugated polymer. In addition, the phrase "containing a functional group in the side chain" refers to containing the functional group described above at the end of the side chain or including the functional group described above branched from the side chain. The term "chain" refers

to a structure in which two or more atoms are serially linked. At this time, one of the atoms contained in the functional groups described above may be included in the atom constituting the molecular chain. Therefore, for example, in the case where a group represented by $CH_2$-COOH is linked in the main chain, this group is a side chain containing a carboxy group.

The side chain preferably contains an alkylene group in at least a part of the molecular chain. In addition, the side chain of the conjugated polymer preferably contains a functional group described above through an alkylene group. The alkylene group may be directly bonded to the atom constituting the conjugated polymer being the main chain may be bonded through, for example, an ether bond or an ester bond.

Examples of the alkylene group include divalent saturated aliphatic hydrocarbon groups such as a methylene group, an ethylene group, a n-propylene group, an isopropylene group, a n-butylene group, a sec-butylene group, a tert-butylene group, a cyclopropylene group, a cyclohexylene group, and a norbornylene group. The alkylene group may have or does not necessarily have a substituent. The additional substituent in the case where the alkylene group has a substituent is not particularly limited. Examples of the additional substituents include an alkyl group and an alkoxy group such as a methoxy group and an ethoxy group. In addition, the number of carbon atoms in the alkylene group is not particularly limited and is preferably 1 or more and 20 or less and more preferably 1 or more and 8 or less from the viewpoint of easy availability and cost.

As the conjugated polymer having the structure described above, the following structures may be specifically exemplified. Here, n in each structure indicates the repeating number in the range of 2 or more and 1,000 or less. In addition, the conjugated polymer may be a homopolymer having each of the structures or a copolymer. In addition, the conjugated polymer may also be a copolymer of each of the structures and each of the structures having no side chain in its structure.

(1) 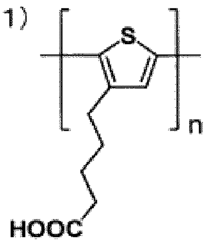 (2) 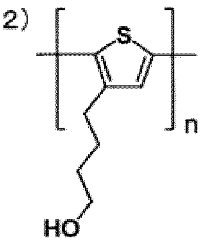 (3) 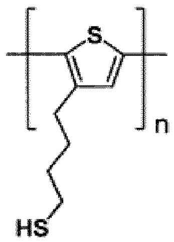 (4) 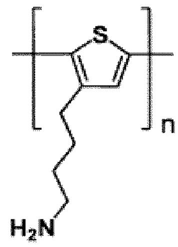

(5) 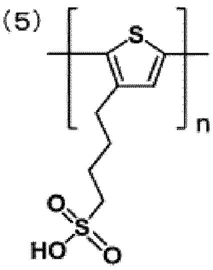 (6) 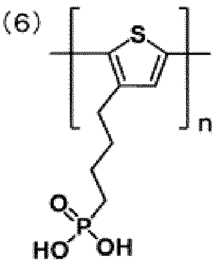 (7) 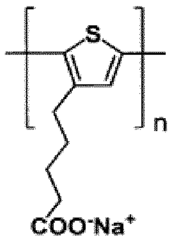 (8) 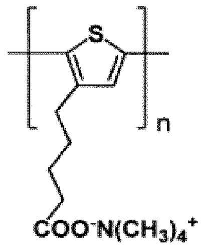

(9) 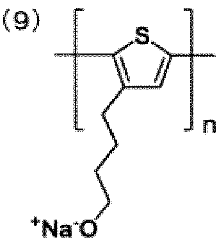 (10) 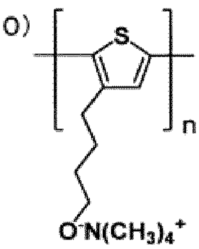 (11) 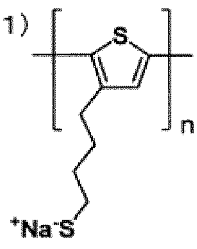 (12) 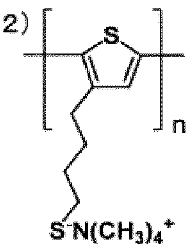

(13) 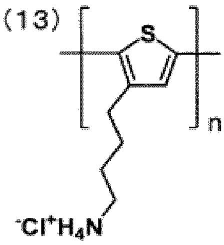

(14) 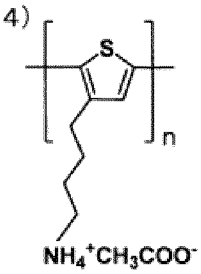

(15) 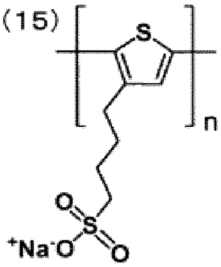

(16) 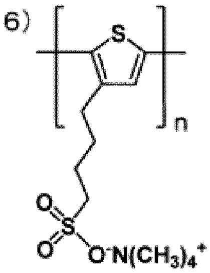

(17) 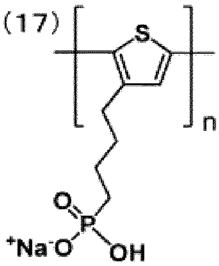

(18) 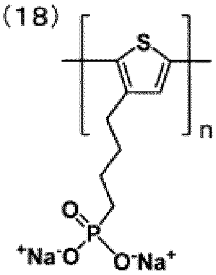

(19) 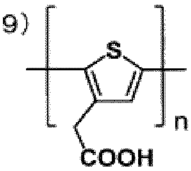

(20) 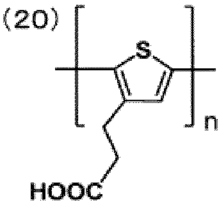

(21) 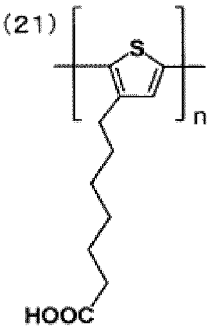

(22) 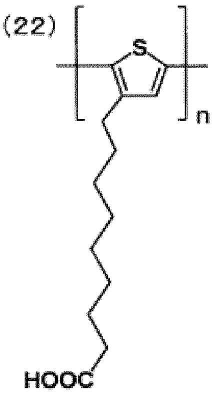

(23) 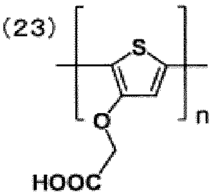

(24) 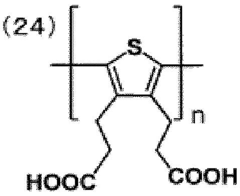

(25) 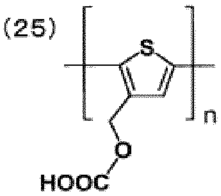

(26) 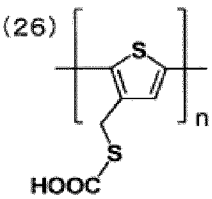

(27) 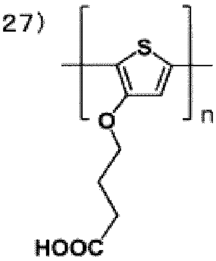

(28) 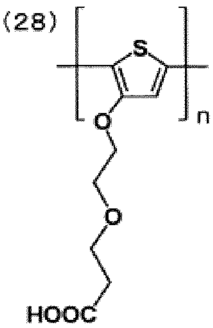

(29) 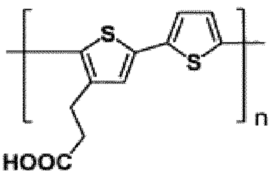

(30) 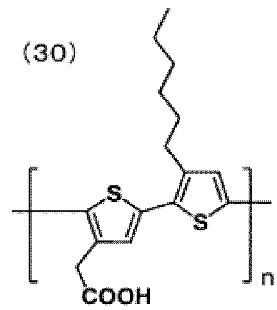

(31) 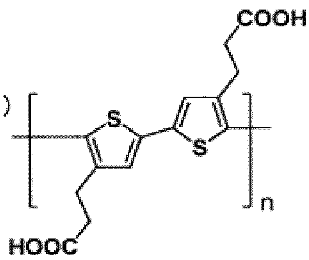

(32) 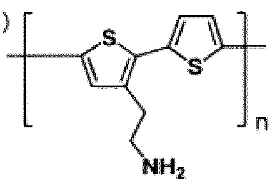

(33) 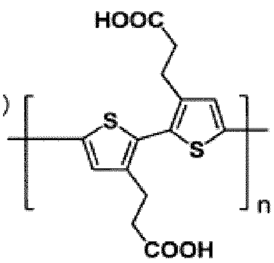

(34)
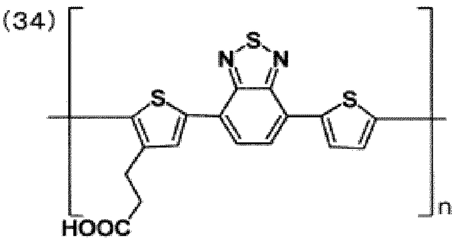
(35)
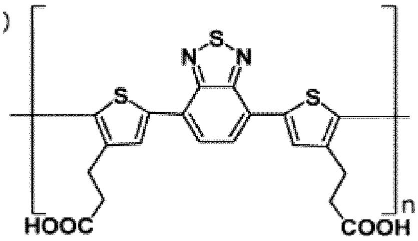

(36)
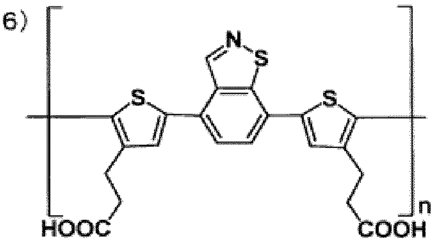
(37)
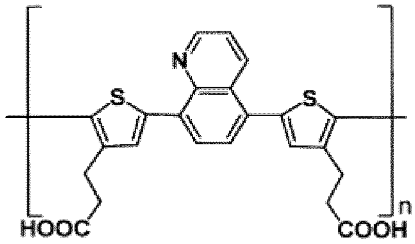

(38)
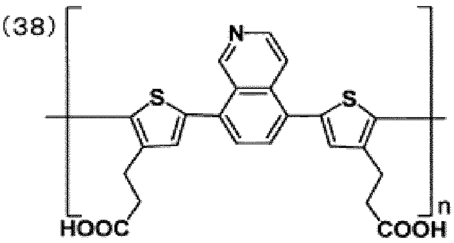
(39)
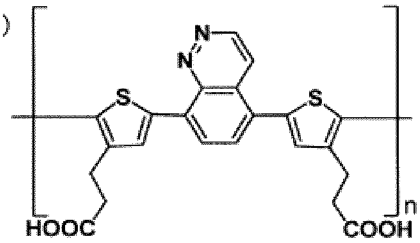

(40)
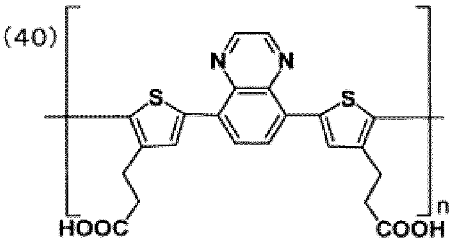
(41)
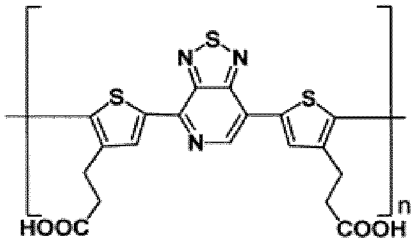

(42)
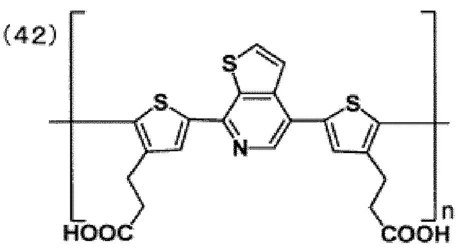
(43)
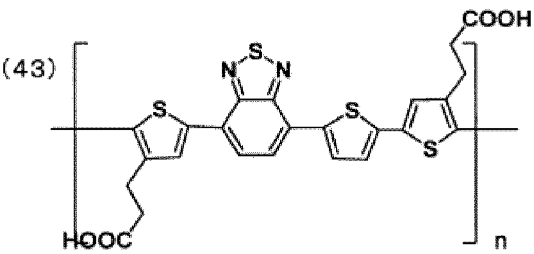

(44)
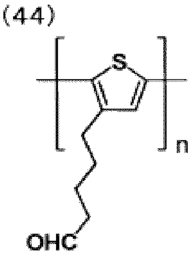
(45)
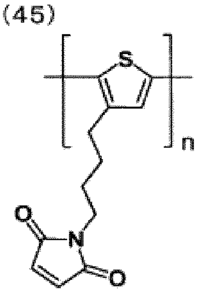
(46)
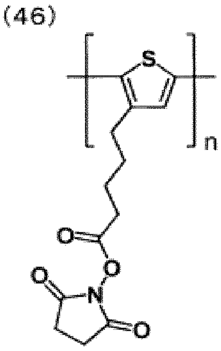
(47)
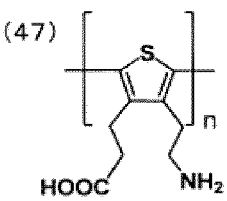

(48)

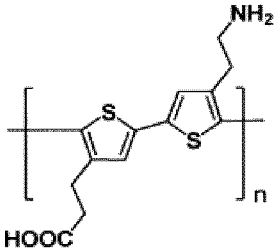

(49)

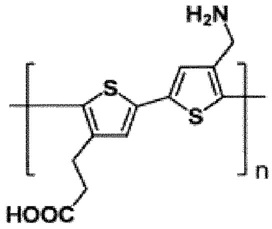

(50)

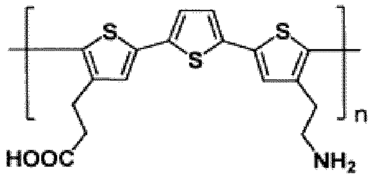

(51)

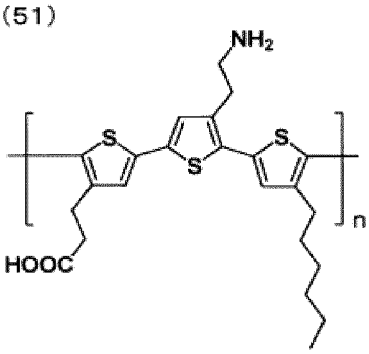

(52)

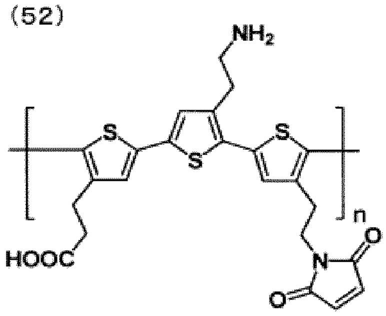

(53)

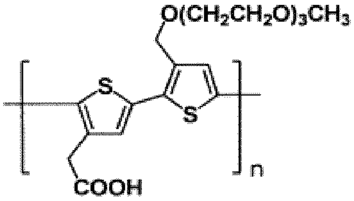

(54)

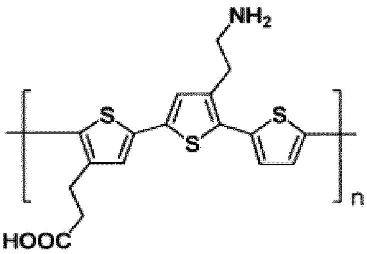

(55)

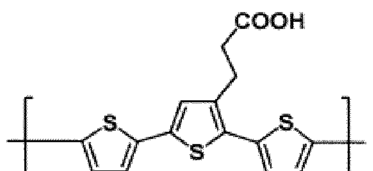

(56)

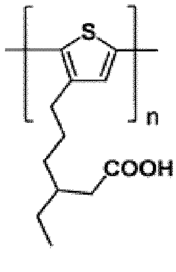

(57)

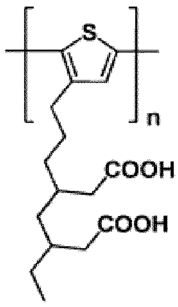

(58)

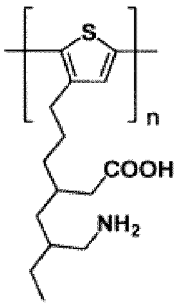

(59)

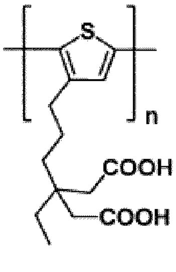

(60)

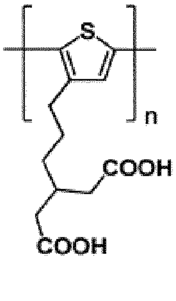

(61)

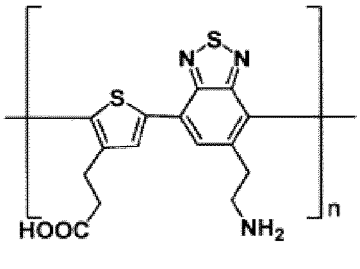

(62)

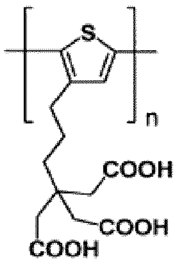

(63)

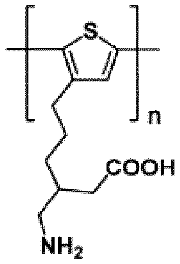

(64)

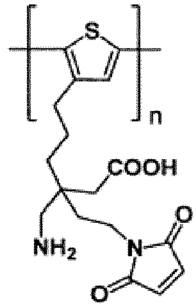

(65)

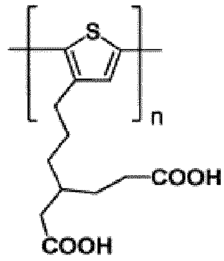

(66)

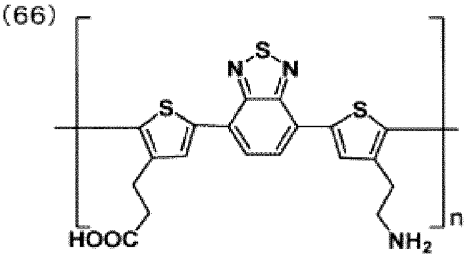

(67)

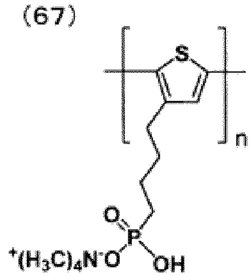

(68)

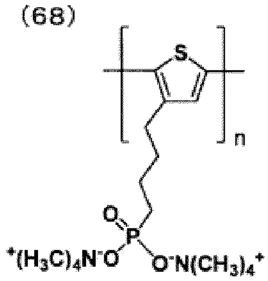

(69)

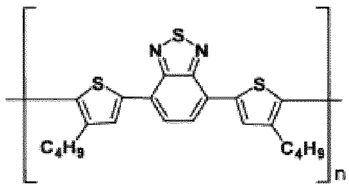

(70)

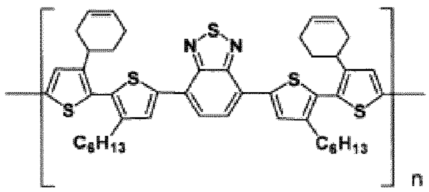

(71)

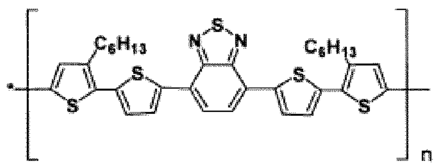

(72)

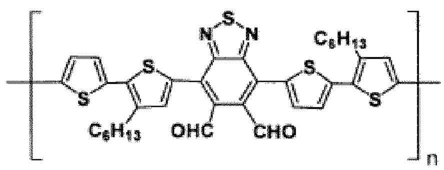

(73)

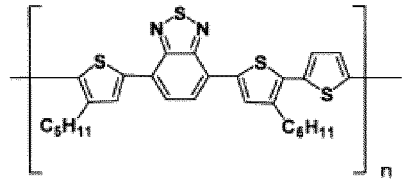

(74)

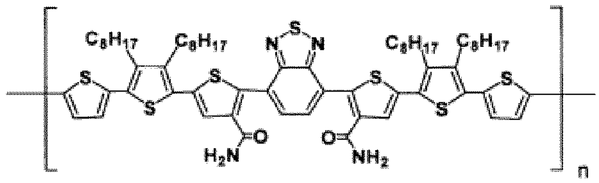

(75)

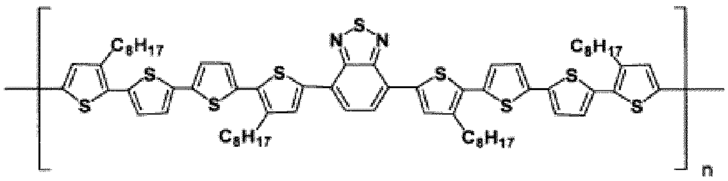

(76)

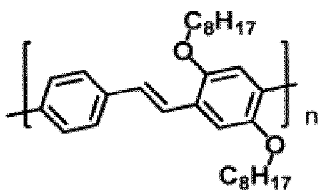

(77)

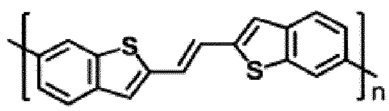

(78)

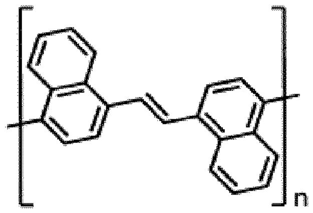

(79)

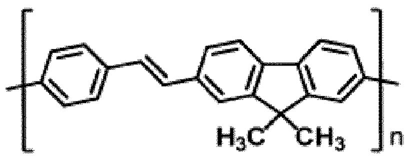

(80)

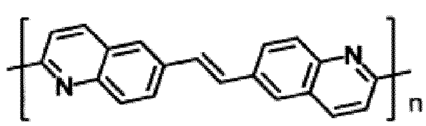

(81)

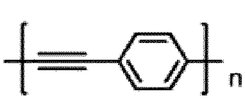

(82)

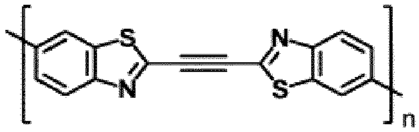

The conjugated polymer used in the present invention can be synthesized by known methods. Examples of the method for synthesizing the monomer include a method for coupling thiophene and a thiophene derivative obtained by introducing an alkyl group having a carboxy group at the end. Specific examples of the method include a method for coupling a halogenated thiophene derivative and a thiophene boronic acid or a thiophene boronic acid ester in the presence of a palladium catalyst and a method for coupling a halogenated thiophene derivative and a thiophene Grignard reagent in the presence of a nickel or palladium catalyst. A polythiophene-based polymer to which an alkyl chain having a terminal carboxy group as a side chain is introduced is obtained by carrying out the polymerization reaction using such monomers. In addition, a compound obtained by coupling a conjugated unit other than thiophene and thiophene by the same method may be served as a monomer unit. A conjugated polymer including a conjugated unit other than thiophene can be obtained by introducing a polymerizable substituent at the end of the thus obtained monomer unit and promoting the polymerization in the presence of a palladium catalyst and a nickel catalyst.

Impurities such as raw materials used in the synthesis process and by-products are preferably removed from the conjugated polymer used in the present invention. As the method, for example, a silica gel column chromatography method, a Soxhlet extraction method, a filtration method, an ion exchange method, and a chelating method can be used. These methods may be used in combination of two or more of these methods.

(Protecting agent)

In the semiconductor sensor according to the embodiment of the present invention, a semiconducting component may be subjected to treatment with a reagent for preventing approach and attachment of the materials other than the target substance (referred to as "protecting agent"). This allows the target substance to be more selectively detected. The protecting agent may be physically attached to the semiconducting component or may be introduced into somewhere in the semiconducting component through bonds.

Methods for attaching a protective agent to a semiconducting component include: (I) a method in which a semiconducting component is pre-dispersed ultrasonically, etc., and a protective agent is added thereto and mixed; (II) a method in which a protecting agent and a semiconducting component are added to a solvent, and the mixed system is mixed by ultrasonic irradiation; (III) a method in which a semiconducting component applied onto a substrate 1 is immersed in a molten protecting agent; and (IV) a method in which a protecting agent is dissolved in a solvent, and a semiconducting component applied onto a substrate 1 is immersed therein. In the present invention, any of these methods may be used, and is also possible to use any combination of these methods. The methods for attaching the protecting agent to the semiconducting components by using the solid-liquid reaction such as (III) or (IV) are preferable from the viewpoint of detection sensitivity.

The conjugated polymer and the protecting agent may be either different compounds or the same compounds. These compounds, however, are preferably different compounds from the viewpoint of detection sensitivity. Examples of protecting agent include proteins such as bovine serum albumin, casein, and skim milk, polysaccharides such as amylose, cellulose, dextran, and carboxymethyl cellulose, polyalkylene glycols such as a polyethylene glycol, low molecular weight organic compounds such as ethanolamine and mercaptoethanol, and synthetic macromolecules such as polyvinyl alcohol, polyoxyethylene-polyoxytetramethylene monomethacrylate, and poly(2-methacryloyloxyethyl phosphorylcholine).

The order of the process of attaching the conjugated polymer to the semiconducting components and the process of modifying the semiconducting component with the protecting agent are not particularly limited. The order in which the conjugated polymer is attached and thereafter the modification with the protecting agent is carried out is preferable.

(Target substance)

The target substance for the semiconductor sensor according to the embodiment of the present invention is not particularly limited. Examples of the target substance include enzymes, antigens, antibodies, haptens, peptides, oligopeptides, polypeptides (proteins), hormones, nucleic acids, oligonucleotides, saccharides such as sugars, oligo sugars, and polysaccharides, low molecular weight compounds, inorganic materials, and composites thereof, viruses, bacteria, cells, living tissues, and substances constituting these examples. These substances bring about change in the electrical characteristics of the semiconductor layer in the semiconductor sensor according to the embodiment of the

present invention due to interaction with the target recognition molecule.

The semiconductor sensor according to the embodiment of the present invention can also detect low molecular weight compounds. The low molecular weight compound is not particularly limited. Examples of the low molecular weight compound include gaseous compounds at normal temperature and normal pressure such as ammonia and methane and solid compounds such as uric acid released from a living body.

The semiconductor sensor according to the embodiment of the present invention has good affinity with substance originated from living organisms because the semiconductor sensor utilizes a protein or nucleic acid as the target capture body X. Accordingly, the semiconductor sensor according to the embodiment of the present invention is preferably a semiconductor sensor that determines the substance originated from living organisms to be a detection target. Here, viruses are also treated as the substance originated from living organisms.

Examples of the combination of the target capture body X/target substance in the semiconductor sensor according to the embodiment of the present invention include T-PSA-mAb (monoclonal antibody for prostate specific antigen)/PSA (prostate specific antigen), anti-hCG-mAb (anti-human chorionic gonadotropin antibody)/hCG (human chorionic gonadotropin), anti-AFP polyclonal antibody (human tissue immunostaining antibody)/$\alpha$-fetoprotein, anti-troponin T (anti-troponin T antibody)/troponin T, anti-CK-MB (anti-creatinine kinase MB antibody)/CK-MB (creatinine kinase MB), anti-PIVKA-II (anti-protein induced by vitamin K absence or antagonist (PIVKA)-II antibody)/PIVKA-II, anti-CA15-3 (breast C tumor marker) antibody/CA15-3, anti-CEA (anti-carcinoembryonic antigen antibody)/CEA (carcinoembryonic antigen), anti-CYFRA (anti-cytokeratin 19 fragment antibody)/CYFRA (cytokeratin 19 fragment), anti-p53 (anti-p53 protein antibody)/p53 (p53 protein), anti-hemoglobin antibody/hemoglobin, anti-glycated hemoglobin antibody/glycated hemoglobin, anti-natriuretic peptide (BNP) antibody/BNP, glucose dehydrogenase (GDH)/glucose, glucose oxidase/glucose, and deoxyribonucleic acid (DNA)/ribonucleic acid (RNA). The semiconductor sensor according to the embodiment of the present invention can be used for various sensors such as a gas sensor, an ion sensor, a myocardial marker sensor, a tumor marker sensor, a hemoglobin sensor, and a glycated hemoglobin sensor.

In the semiconductor sensor according to the embodiment of the present invention, the target substances that are less likely to cause measurement errors over a wide concentration range are hemoglobin and glycated hemoglobin. Therefore, the semiconductor sensor for which these substances are determined to be detection targets is particularly preferable. In other words, the target capture body X is preferably the substructure of immunoglobulin that selectively interacts with at least one of hemoglobin or glycated hemoglobin.

In addition, the semiconductor sensor according to the embodiment of the present invention is suitable for detecting hemoglobin and glycated hemoglobin and thus may constitute a combined sensor including the semiconductor sensor and a sensor configured to detect glucose. For the combined sensor, glycated hemoglobin, which is one of the target substance, is particularly preferably hemoglobin A1c (hereinafter, abbreviated as HbA1c). The medical condition of diabetes can be effectively monitored by the sensor that measures the concentrations of the substances, glucose and HbA1c, which are indicators of diabetes, in a biological fluid.

(Biological fluid)

The semiconductor sensor according to the embodiment of the present invention can also be used for the purpose of detecting substances in a biological fluid. The biological fluid refers to a liquid that a creature has in some way in its body. All liquids collected from the living body such as blood, a tissue fluid, a body cavity fluid, a digestive fluid, urine, saliva, sweat, tear, snot, semen, a lymphatic fluid, a vaginal fluid, an amniotic fluid, milk, a cerebrospinal fluid, a synovial fluid, and a cell suspension can be used as they are. In addition, the biological fluid may be a sample in which cell components or the like are previously crushed or removed from a biological sample. Of the biological fluids, as the sample for the semiconductor sensor according to the embodiment of the present invention, blood, saliva, sweat, tear, or urine is preferable from the viewpoint of easy availability. Of these fluids, blood is more preferable because blood includes numerous pieces of biological information.

(Method for producing semiconductor sensor)

As the method for producing the semiconductor sensor according to the embodiment of the present invention, a method for producing the semiconductor sensor according to the first embodiment will be described as an example. The method of manufacturing the semiconductor sensor includes the steps of applying a semiconducting component onto a substrate 1 and drying the applied semiconducting component to form a semiconductor layer. The production method, however, is not limited to the following method.

First, the first electrode 2 and the second electrode 3 are formed on the substrate 1. Examples of the method for forming the electrodes include known methods such as vacuum evaporation of metal, a spin coating method, a blade coating method, a slit die coating method, a screen printing method, a bar coater method, a casting method, a transfer printing method, an immersion and withdrawal method, or an inkjet method. In order to form each of the electrodes 2

and 3 in a pattern, the pattern may be directly formed by using, for example, a mask. In addition, the pattern formation of the electrodes is also possible by applying a resist to the substrate 1, exposing and developing the applied resist to process the resist film into a desired pattern, and etching the electrodes.

Subsequently, the semiconductor layer 4 is formed. As the method for producing the semiconductor layer 4, a method including the steps of applying the semiconducting component onto the substrate 1, bonding or attaching a precursor of a target recognition molecule to the semiconducting component, and forming a bond between the precursor of the target recognition molecule and the target capture body X is preferable. Here, the phrase "precursor of a/the target recognition molecule" refers to a part of the target recognition molecule excluding the target capture body X. Forming the bond between the precursor of the target recognition molecule and the target capture body X results in the target recognition molecule used in the present invention.

Examples of the method for bonding or attaching the precursor of the target recognition molecule to the semiconducting component include a method for vacuum-evaporating the precursor of the target recognition molecule in a vacuum, a method for immersing a layer including the semiconducting component into a solution in which the precursor of the target recognition molecule is dissolved, a method for applying the precursor of the target recognition molecule made of a part excluding the target capture body X to the semiconductor layer, and a method for applying a solution into which the precursor of the target recognition molecule is dissolved to the layer including the semiconducting component.

Examples of a method for forming a bond between the precursor of the target recognition molecule and the target capture body X include a method for colliding the target capture body X to a layer including the semiconducting component in a vacuum to cause reaction, a method for immersing the layer including the semiconducting component into a solution into which the target capture body X is dissolved, and a method for applying a solution into which the target capture body X is dissolved to a layer including the semiconducting component.

Hereinafter, the reaction in which the precursor of the target recognition molecule is attached to the semiconducting component, and thereafter a bond is formed between the precursor of the target recognition molecule and the target capture body X to form the target recognition molecule will be described using specific examples. First, a substrate in which CNT serving as the semiconducting component is arranged between two electrodes is prepared. Subsequently, the precursor of the target recognition molecule having a pyrenyl group and a maleimide group is dissolved into an organic solvent such as acetonitrile. Into this solution, the substrate prepared as described above is immersed. Then, as illustrated in FIG. 9A, the precursor of the target recognition molecule is attached onto CNT 19 by the $\pi$-$\pi$ electron interaction between CNT 19 and the pyrene ring. L' in FIG. 9A, however, is a structure excluding the maleimide group part from the structure to be the linking group L after the reaction.

Subsequently, thus obtained substrate on which the precursor of the target recognition molecule is immobilized on CNT 19 is immersed into an aqueous solution into which Fab', which is the substructure of immunoglobulin 20 serving as the target capture body X, is dissolved. Then, the sulfur atom 21 in the hinge region of Fab' forms a bond by reacting with the maleimide group in the precursor of the target recognition molecule to give the target recognition molecule. In other words, as the result of the reaction, a state where the target recognition molecule is immobilized on CNT 19 is formed as illustrated in FIG. 9B.

The arrangement of the semiconducting component onto the substrate 1 and the immobilization of the target recognition molecule may be separately carried out or may be collectively carried out. To carry out the arrangement and the immobilization collectively, for example, a method for forming the semiconductor layer by using the semiconducting component to which target recognition molecules are previously bonded or attached is used.

For the method for producing the semiconductor sensor according to other embodiments, the method described above may be correspondingly applicable. For example, the method for producing the semiconductor sensor according to the second embodiment further includes a step of previously forming the third electrode 5 and the insulating layer 6 on the substrate 1 with respect to the method for producing the semiconductor sensor according to the first embodiment.

(Examples and Comparative Examples)

Hereinafter, specific Examples according to the embodiment of the present invention will be described. The present invention, however, is not limited to Examples described below. The used compounds represented by abbreviations will be listed below.

| | |
|---|---|
| o-DCB: | o-Dichlorobenzene |
| PBS: | Phosphate-buffered saline |
| BSA: | Bovine serum albumin |
| EDTA: | Ethylenediaminetetraacetic acid |
| P3HT: | Poly-3-hexylthiophene |
| DMF: | Dimethylformamide |

HRG: Histidine-rich glycoprotein
NMP: N-Methylpyrrolidone
EDC: Ethyl(dimethylaminopropyl)carbodiimide
NHS: N-Hydroxysuccinimide
PBSE: Pyrenebutyric acid succinimide ester

Example 1

(1) Preparation of semiconducting component solution A

To 15 mL of chloroform, 1.5 mg of CNT (manufactured by Carbon Nanotechnology Inc., single-walled CNT, including 95% by weight of semiconductor-type CNT) and 1.5 mg of poly[(m-phenylenevinylene)-co-(2,5-dioctoxy-p-phenylenevinylene)] (manufactured by Sigma-Aldrich Co. LLC, hereinafter referred to as a conjugated polymer [A]), which was a conjugated polymer not containing a sulfur atom, were added. The resultant mixture was stirred by ultrasonic wave at an output of 250 W for 30 minutes using an ultrasonic homogenizer (VCX-500, manufactured by TOKYO RIKAKIKAI CO, LTD.), while cooling with ice to give a CNT composite dispersion liquid A (a concentration of CNT composite relative to the solvent of 0.06 g/L).

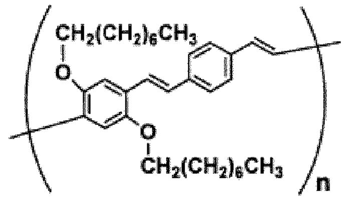

Subsequently, a semiconducting component solution A for forming the semiconductor layer was prepared. The CNT dispersion liquid A described above was filtered using a membrane filter (pore size 10 μm, diameter 25 mm, omnipore membrane filter, manufactured by Millipore corporation) to remove the CNT composite having a length of 10 μm or longer. To 5 mL of the obtained filtrate, 21 mL of o-DCB was added to prepare a semiconducting component solution A (a concentration of CNT composite relative to the solvent of 0.01 g/L).

(2) Preparation of mouse anti-human HbA1c Fab'

Using citric acid monohydrate (manufactured by KANTO CHEMICAL CO., INC.) and 10 N sodium hydroxide aqueous solution (manufactured by Wako Pure Chemical Corporation), 0.1 M sodium citrate buffer solution (pH 3.5) was prepared. Into this buffer solution, pepsin (manufactured by Wako Pure Chemical Corporation) was dissolved so that the resultant solution had a concentration of 1 mg/mL to prepare a pepsin solution. Separately, 40 μL of a mouse anti-human HbA1c antibody (manufactured by BBI Solutions) having a concentration of 7.7 mg/mL was diluted with 269 μL of 0.1 M sodium citrate buffer solution (pH 3.5) to prepare a solution having a concentration of 1 mg/mL. To 300 μL of the obtained solution, 3 μL of the pepsin solution was added and the resultant mixture was allowed to stand at 37°C for 1 hour. Thereafter, 100 μL of 1 M trishydroxymethylaminomethane-hydrochloric acid buffer solution (pH 8.0) was added and the reaction was stopped by neutralization.

10 μL of the obtained solution, 5 μL of pure water, and 5 μL of NuPAGE LDS Sample Buffer (trade name, manufactured by Invitrogen corporation) were mixed and the resultant mixture was heated at 95°C for 5 minutes. Thereafter, polyacrylamide gel electrophoresis (SDS-PAGE) was carried out together with Precision Plus protein two color standard (trade name, manufactured by Bio-Rad Laboratories, Inc.) serving as a molecular weight marker. The electrophoresis tank used in that procedure was EasySeparator (trade name, manufactured by Wako Pure Chemical Corporation), the power supply was My Power II500 (trade name, manufactured by ATTO CORPORATION), the gel was Super Sep Ace, 5% - 20% (trade name, manufactured by Wako Pure Chemical Corporation), and the electrophoresis buffer solution was Protein Running Buffer (trade name, manufactured by Wako Pure Chemical Corporation). The current flowing through the electrophoresis tank using the power supply was set at 20 mA at a constant voltage and electrophoresis was carried out for 70 minutes. It was determined that the Fc region 16 was cut to generate $F(ab')_2$ by staining a gel and an antibody digestion product with Quick CBB Plus (trade name, manufactured by Wako Pure Chemical Corporation), thereafter decolorizing the gel with pure water, and comparing the generated band of the antibody digestion product with the molecular weight marker.

Subsequently, 300 μL of buffer solution of $F(ab')_2$ solution was replaced with 0.1 M sodium phosphate buffer solution including 5 mM EDTA using PD MiniTrap G-25 column. Thereafter, the $F(ab')_2$ solution was concentrated using

Amicon Ultra 0.5, 10 K (trade name, manufactured by Merck Millipore). After concentration, the concentration of the concentrated product was measured using NanoDrop 2000 (trade name, manufactured by Thermo Fisher Scientific Inc.) and the concentration was found to be 2.0 mg/mL. 2-aminoethanethiol hydrochloride (manufactured by Wako Pure Chemical Corporation) was dissolved into 0.1 M sodium phosphate buffer solution including 5 mM EDTA so that the concentration of 2-aminoethanethiol hydrochloride was 0.1 M and 10 μL of the prepared solution was added to 100 μL of the $F(ab')_2$ solution described above. The resultant solution was allowed to stand at 37°C for 90 minutes to promote reduction reaction to prepare Fab'. The obtained solution was passed through PD MiniTrap G-25 column using 0.1 M sodium phosphate buffer solution including 5 mM EDTA as an eluent to remove low molecular weight components. The concentration of the obtained solution was measured by NanoDrop 2000 and the concentration was found to be 0.33 mg/mL. In addition, the molecular weight of the prepared Fab' was measured by MALDI-TOF-MS (manufactured by Bruker Corporation, autoflex speed) and the molecular weight was found to be 48,550.

(3) Formation of electrodes and semiconductor layer

A semiconductor element for use in the application of the semiconductor sensor according to the third embodiment was prepared. Gold was vacuum evaporated on the glass substrate 1 (thickness 0.7 mm) so as to have a film thickness of 50 nm. A photoresist (trade name "LC 100-10 cP", manufactured by Rohm and Haas Company) was applied by spin coating (1000 rpm × 20 seconds) on the gold film, followed by heating and drying the applied photoresist at 100°C for 10 minutes.

The prepared photoresist film was subjected to pattern exposure through a mask using a parallel light mask aligner (PLA-501F, manufactured by Canon Inc.). Thereafter, the patterned photoresist film was subjected to shower development with ELM-D (trade name, manufactured by MITSUBISHI GAS CHEMICAL COMPANY, INC.) being a 2.38% by weight tetramethylammonium hydroxide aqueous solution for 70 seconds using an automatic developing apparatus (AD-2000, manufactured by TAKIZAWA SANGYO K.K.) and then washed with water for 30 seconds. Thereafter, etching treatment was carried out for 5 minutes using AURUM-302 (trade name, manufactured by KANTO CHEMICAL CO., INC.) and then the sample was washed with water for 30 seconds. The resist was peeled off by immersing the resist into AZ Remover 100 (trade name, manufactured by Az Electronic Materials Plc) for 5 minutes and the sample was washed with water for 30 seconds. Thereafter, the sample was heated and dried at 120°C for 20 minutes to form the first electrode 2, the second electrode 3, and the third electrode 7 made of gold.

The width (channel width) of each of the first electrode 2 and the second electrode 3 was set to 300 μm and the interval (channel length) between the first electrode 2 and the second electrode 3 was set to 20 μm. The third electrode 7 was arranged parallel to the second electrode 3 and the interval between the third electrode 7 and the second electrode 3 was set to 5 mm.

Onto the substrate 1 on which the first electrode 2 and the second electrode 3 were formed, 600 pL of the semiconducting component solution A prepared by the method described in (1) was dropped using an ink jet apparatus (manufactured by Cluster Technology Co., Ltd.) to form the semiconductor layer 4. Thereafter, heat treatment was carried out on a hot plate at 150°C for 30 minutes under a nitrogen stream to give a semiconductor element A.

Subsequently, the characteristics of the current (Id) between the first electrode 2 and the second electrode 3/the voltage (Vsd) between the first electrode 2 and the second electrode 3 at the time of changing the voltage (Vg) of the third electrode 7 of the semiconductor element A described above were measured. Measurement was carried out using a semiconductor characteristic evaluation system 4200-SCS type (manufactured by Keithley Instruments, LLC) under 100 μL of 0.01 M PBS (pH 7.4, manufactured by Sigma-Aldrich Co. LLC) (room temperature of 25°C and humidity of 45% at the time of measurement). The minimum value and the maximum value of the drain current Id when Vg was changed from 0 V to -1 V were found to be $5 \times 10^{-11}$ A and $2 \times 10^{-7}$ A, respectively. The on/off ratio, which is the ratio of the maximum value of Id to the minimum value of Id, was found to be $4 \times 10^3$.

(4) Synthesis of precursor of target recognition molecule [A]

Into 143 mL of DMF, 38.32 mg of 1-aminomethylpyrene hydrochloride (manufactured by Funakoshi Co., Ltd.) and 51.09 mg of N-(8-maleimidecapryloxy)succinimide (manufactured by DOJINDO LABORATORIES) were dissolved. To this solution, 46.48 mg of sodium hydrogen carbonate was added and the resultant mixture was stirred for 7 hours. Thereafter, 312 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 60.08 mg of a white solid. The mass spectrum (JMS-Q1000TD, manufactured by JEOL Ltd.) of the obtained white solid was measured and M + 1 was found to be 453. This confirmed that the white solid was the following precursor of the target recognition molecule [A].

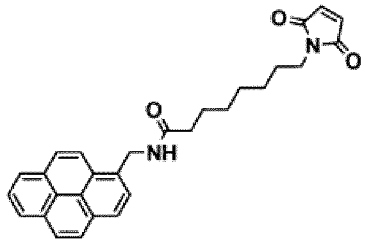

(5) Attachment of precursor of target recognition molecule [A] to semiconducting component and immobilization of Fab'

Subsequently, 10 mg of the precursor of the target recognition molecule [A] synthesized in (4) was dissolved into 10 mL of acetonitrile (manufactured by Wako Pure Chemical Corporation) and the semiconductor layer 4 formed in (3) was immersed into the resultant solution for 4 hours to attach the precursor of the target recognition molecule [A] to the CNT composite serving as the semiconducting component. Thereafter, the semiconductor layer 4 was sufficiently rinsed with acetonitrile and 0.01 M PBS.

Subsequently, the Fab' solution of the mouse anti-human HbA1c antibody prepared in (2) was diluted with PBS so that the concentration of the solution was 0.10 mg/mL and the semiconductor layer 4 was immersed into the diluted Fab' solution at 4°C for 18 hours. This caused the reaction of the mercapto group in the hinge region of Fab' and the maleimide group in the precursor of the target recognition molecule [A] to immobilize Fab' on the semiconductor layer 4. Thus, the semiconductor layer 4 having the CNT composite to which the target recognition molecule in which Fab' of the mouse anti-human HbA1c antibody having a molecular weight of 48550 served as the target capture body X was attached was obtained. In this case, the pyrene ring of the precursor of the target recognition molecule [A] is attached to CNT and thus the number of atoms from the atom bonded to the group attached to the semiconducting component to the atom bonded to the atom originated from Fab' of the mouse anti-human HbA1c antibody serving as the target capture body in the linking group L of the target recognition molecule being the final product is 13.

Moreover, protection with a protecting agent was carried out. More specifically, BSA (manufactured by Wako Pure Chemical Corporation) was dissolved into 0.01 M PBS so that the concentration of the BSA was 10 mg/mL and the semiconductor layer 4 was immersed into the solution at 4°C for 3 hours. Thereafter, the semiconductor layer 4 was sufficiently rinsed with 0.01 M PBS. This provided the semiconductor sensor having the surface of the semiconductor layer 4 in the gap part between Fab's covered with BSA and protecting the surface from nonselective adsorption of proteins.

(6) Evaluation as semiconductor sensor

The semiconductor layer 4 of the prepared semiconductor sensor was immersed into 100 μL of a 0.01 M PBS solution and the current value (Id) flowing between the first electrode 2 and the second electrode 3 was measured. At this time, conditions in which the voltage (Vsd) between the first electrode 2 and the second electrode 3 was -0.2 V and the voltage (Vg) between the first electrode 2 and the third electrode 7 was -0.6 V were set. The current Id (initial current value) at the start of the measurement was 3.74 μA.

To the 0.01 M PBS solution into which the semiconductor layer 4 was immersed, 20 μL of a solution in which BSA was dissolved into 0.01 M PBS solution so that the concentration of the BSA solution was 100 μL/mL, 20 μL of a solution in which avidin (manufactured by Wako Pure Chemical Corporation) was dissolved into 0.01 M PBS solution so that the concentration of the avidin solution was 100 μL/mL, and 20 μL of a solution in which human HbAlc (manufactured by manufactured by BBI Solutions) was dissolved into 0.01 M PBS solution so that the concentration of the human HbAlc solution was 100 μL/mL each were added 3 minutes, 6 minutes, and 9 minutes after the start of the measurement, respectively. In this case, the current value increased by 0.63 μA only when the solution including human HbAlc was added. This result confirmed that this semiconductor sensor selectively detected human HbAlc.

The preparation conditions and measurement results of the semiconductor sensors for quantitatively evaluating the performance as semiconductor sensors are listed in Table 1 and Table 2. Table 1 lists Examples as specific examples of the embodiments described above and Table 2 lists Comparative Examples and Reference Examples for comparison with Examples. Here, as the rate of change in the current value becomes larger, the performance of the semiconductor sensor becomes better when the sample solution into which the target substance is dissolved is added. Therefore, the signal intensity is determined by reading Id before the addition of the sample solution and Id after adding the sample solution and calculating the absolute value of the current value change ratio [%]. That is, the calculation formula of the signal intensity [%] is as follows.

$$\text{Signal intensity [\%]} = 100 \times |(\text{Id after addition of target substance}) - (\text{Id before addition of target substance})| / (\text{Id before addition of target substance})$$

The signal intensity was 16.8%.

Table 1

| | Semiconducting component | Sensor preparation conditions | | | | | Measurement results | | |
|---|---|---|---|---|---|---|---|---|---|
| | | Conjugated polymer | Target recognition molecule | | Number of atom (s) of linking group N | Third electrode | Initial current value [μA] | Change in current value [μA] | Signal intensity [%] |
| | | | Target capture body X | Precursor of target recognition molecule | | | | | |
| Example 1 | CNT | Conjugated polymer [A] | Mouse anti-human HbAlc Fab' | Precursor of target recognition molecule [A] | 13 | Presence | 3.74 | 0.63 | 16.8 |
| Example 2 | CNT | P3HT | Mouse anti-human HbAlc Fab' | Precursor of target recognition molecule [B] | 8 | Presence | 4.10 | 0.81 | 19.8 |
| Example 3 | CNT | P3HT | Mouse anti-human HbAlc Fab' | Precursor of target recognition molecule [C] | 6 | Presence | 4.08 | 0.43 | 10.6 |
| Example 4 | CNT | P3HT | Mouse anti-human HRG Fab' | Precursor of target recognition molecule [A] | 13 | Presence | 4.12 | 0.79 | 19.2 |
| Example 5 | CNT | P3HT | Mouse anti-human HbAlc Fab' | Precursor of target recognition molecule [A] | 13 | Absence | 1.46 | 0.27 | 18.5 |
| Example 6 | CNT | Conjugated polymer [B] | Mouse anti-human HbAlc Fab' | Conjugated polymer [B] | 12 | Presence | 3.60 | 0.53 | 14.7 |
| Example 7 | CNT | - | Mouse anti-human HbAlc Fab' | Precursor of target recognition molecule [A] | 13 | Presence | 3.61 | 0.55 | 15.3 |
| Example 8 | CNT | P3HT | Mouse anti-human HbAlc Fab' | Precursor of target recognition molecule [D] | 9 | Presence | 4.13 | 0.85 | 20.6 |
| Example 9 | CNT | P3HT | Mouse anti-human HbAlc Fab' | Precursor of target recognition molecule [A] | 13 | Presence | 4.15 | 0.87 | 21.0 |

(continued)

| | Semiconducting component | Sensor preparation conditions | | | | | Measurement results | | |
|---|---|---|---|---|---|---|---|---|---|
| | | Conjugated polymer | Target recognition molecule | | Number of atom (s) of linking group N | Third electrode | Initial current value [μA] | Change in current value [μA] | Signal intensity [%] |
| | | | Target capture body X | Precursor of target recognition molecule | | | | | |
| Example 10 | CNT | Conjugated polymer [B] | Mouse anti-human HbAlc Fab' | Conjugated polymer [B]/ Precursor of target recognition molecule [A] | 12/13 | Presence | 4.17 | 0.88 | 21.2 |
| Example 11 | CNT | P3HT | Mouse anti-human HbAlc Fab' | Precursor of target recognition molecule [E] | 16 | Presence | 4.15 | 0.85 | 20.4 |
| Example 12 | CNT | P3HT | Mouse anti-human HbAlc Fab' | Precursor of target recognition molecule [F] | 5 | Presence | 4.09 | 0.42 | 10.2 |
| Example 13 | Graphene | - | Mouse anti-human HbAlc Fab' | Precursor of target recognition molecule [A] | 13 | Presence | 6.29 | 0.71 | 11.3 |
| Example 14 | CNT | P3HT | Mouse anti-human HbAlc Fab' | Precursor of target recognition molecule [G] | 14 | Presence | 4.13 | 0.83 | 20.2 |
| Example 15 | CNT | P3HT | Mouse anti-human HbAlc Fab' | Precursor of target recognition molecule [H] | 26 | Presence | 4.10 | 0.45 | 10.9 |
| Example 16 | CNT | P3HT | Mouse anti-human HbAlc Fab' | Precursor of target recognition molecule [I] | 30 | Presence | 4.09 | 0.43 | 10.4 |
| Example 17 | CNT | P3HT | Mouse anti-human HbAlc Fab' | Precursor of target recognition molecule [J] | 17 | Presence | 4.11 | 0.65 | 15.8 |

Table 2

| | Semiconducting component | Sensor preparation conditions | | | | | Measurement results | | |
|---|---|---|---|---|---|---|---|---|---|
| | | Conjugated polymer | Target recognition molecule | | Number of atom(s) of linking group N | Third electrode | Initial current value [μA] | Change in current value [μA] | Signal intensity [%] |
| | | | Target capture body X | Precursor of target recognition molecule | | | | | |
| Comparative Example 1 | CNT | P3HT | Mouse anti-human HbAlc Fab' | Physical adsorption | - | Presence | 4.03 | 0.16 | 4.0 |
| Comparative Example 2 | CNT | P3HT | Mouse anti-human HbAlc Fab' | PBSE | 4 | Presence | 4.06 | 0.17 | 4.3 |
| Comparative Example 3 | CNT | P3HT | Mouse anti-human HbAlc whole antibody | PBSE | 4 | Presence | 4.04 | 0.11 | 2.7 |
| Comparative Example 4 | Graphene | - | Mouse anti-human HbAlc Fab' | PBSE | 4 | Presence | 5.32 | 0.22 | 4.1 |
| Comparative Example 5 | CNT | P3HT | Mouse anti-human HbAlc Fab' | Precursor of target recognition molecule [K] | 31 | Presence | 4.05 | 0.17 | 4.2 |
| Comparative Example 6 | CNT | P3HT | Target recognition molecule [L] | | 4 | Presence | 4.01 | 0.14 | 3.4 |
| Comparative Example 7 | CNT | P3HT | Target recognition molecule [M] | | 5 | Presence | 4.03 | 0.15 | 3.6 |
| Comparative Example 8 | CNT | P3HT | Target recognition molecule [N] | | 13 | Presence | 4.02 | 0.14 | 3.5 |
| Comparative Example 9 | CNT | P3HT | Target recognition molecule [O] | | 30 | Presence | 4.02 | 0.14 | 3.5 |
| Comparative Example 10 | CNT | P3HT | Target recognition molecule [P] | | 31 | Presence | 4.03 | 0.15 | 3.6 |
| Reference Example 1 | CNT | P3HT | Mouse anti-human HbAlc Fab' | N-(1-Pyrenyl) maleimide | 4 | Presence | 4.08 | 0.31 | 7.6 |

(continued)

| | Semiconducting component | Sensor preparation conditions | | | | | Measurement results | | |
|---|---|---|---|---|---|---|---|---|---|
| | | Conjugated polymer | Target recognition molecule | | Number of atom(s) of linking group N | Third electrode | Initial current value [μA] | Change in current value [μA] | Signal intensity [%] |
| | | | Target capture body X | Precursor of target recognition molecule | | | | | |
| Reference Example 2 | CNT | P3HT | Mouse anti-human HbAlc Fab' | Precursor of target recognition molecule [Q] | 31 | Presence | 4.08 | 0.30 | 7.3 |

Example 2

(1) Preparation of semiconducting component solution B

To 15 mL of chloroform, 1.5 mg of CNT (manufactured by Carbon Nanotechnology Inc., single-walled CNT, including 95% by weight of semiconductor-type CNT) and 1.5 mg of P3HT were added. The resultant mixture was stirred by ultrasonic wave at an output of 250 W for 30 minutes using an ultrasonic homogenizer (VCX-500, manufactured by TOKYO RIKAKIKAI CO, LTD.) while cooling with ice to give a CNT composite dispersion liquid B (a concentration of CNT composite relative to the solvent of 0.1 g/L).

Subsequently, a semiconducting component solutions B for forming the semiconductor layer 4 was prepared. The CNT dispersion liquid B described above was filtered using a membrane filter (pore size 10 μm, diameter 25 mm, omnipore membrane filter, manufactured by Millipore corporation) to remove the CNT composite having a length of 10 μm or longer. To 5 mL of the obtained filtrate, 45 mL of o-DCB was added to prepare a semiconducting component solution B (a concentration of CNT composite relative to the solvent of 0.01 g/L).

(2) Formation of electrodes and semiconductor layer

The first electrode 2, the second electrode 3, the third electrode 7, and the semiconductor layer 4 were formed to obtain the semiconductor element B by the same method as the method in Example 1 (3) except that the semiconducting component solution B prepared in (1) was used instead of the semiconducting component solution A. The characteristics of the current (Id) between the first electrode 2 and the second electrode 3/the voltage (Vsd) between the first electrode 2 and the second electrode 3 at the time of changing the voltage (Vg) of the third electrode 7 of the semiconductor element B described above were measured under the same conditions as Example 1 (3). The on/off ratio when Vg was changed from 0 V to -1 V was $6 \times 10^3$.

(3) Synthesis of precursor of target recognition molecule [B]

Into 1,110 mL of DMF, 40.65 mg of 1-aminopyrene (manufactured by Tokyo Chemical Industry Co., Ltd.) and 57.54 mg of N-(4-maleimidebutyryloxy)succinimide (manufactured by DOJINDO LABORATORIES) were dissolved and the resultant mixture was stirred for 7 hours. Thereafter, 400 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 48.69 mg of a white solid. The mass spectrum (JMS-Q1000TD, manufactured by JEOL Ltd.) of the obtained white solid was measured and M + 1 was found to be 383. This confirmed that the white solid was the following precursor of the target recognition molecule [B].

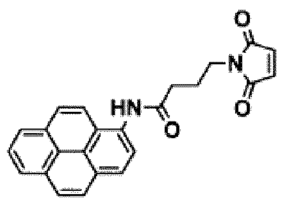

(4) Attachment of precursor of target recognition molecule [B] to semiconducting component and immobilization of Fab'

The precursor of the target recognition molecule [B] was attached to the CNT composite serving as the semiconducting component by the same method as the method in Example 1 (5) except that the precursor of the target recognition molecule [B] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [B] is attached to CNT and thus the number of atoms from the atom bonded to the group attached to the semiconducting component to the atom bonded to the atom originated from Fab' of the mouse anti-human HbAlc antibody serving as the target capture body in the linking group L of the target recognition molecule being the final product is 8. Moreover, the surface was protected with BSA by the same method as the method in Example 1 (5) to give a semiconductor sensor.

(5) Evaluation as semiconductor sensor

The obtained semiconductor sensor was evaluated as a semiconductor sensor by the same method as the method in Example 1 (6). The current Id at the start of the measurement was 4.10 μA and the current value was increased by 0.81 μA only when human HbAlc was added. This result confirmed that this semiconductor sensor selectively detected human HbAlc. The signal intensity was 19.8%.

Example 3

(1) Synthesis of precursor of target recognition molecule [C]

Into 100 mL of DMF, 54.83 mg of 1-aminopyrene (manufactured by Tokyo Chemical Industry Co., Ltd.) and 63.54 mg of N-succinimidyl maleimide acetate (manufactured by Tokyo Chemical Industry Co., Ltd.) were dissolved and the resultant mixture was stirred for 7 hours. Thereafter, 500 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 21.33 mg of a white solid. The mass spectrum (JMS-Q1000TD, manufactured by JEOL Ltd.) of the obtained white solid was measured and M + 1 was found to be 355. This confirmed that the white solid was the following precursor of the target recognition molecule [C].

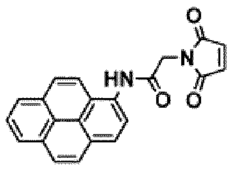

(2) Attachment of precursor of target recognition molecule [C] to semiconducting component and immobilization of Fab'

The precursor of the target recognition molecule [C] was attached to the CNT composite serving as the semiconducting component by the same method as the method in Example 1 (5) except that the precursor of the target recognition molecule [C] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [C] is attached to CNT and thus the number of atoms from the atom bonded to the group attached to the semiconducting component to the atom bonded to the atom originated from Fab' of the mouse anti-human HbAlc antibody serving as the target capture body in the linking group L of the target recognition molecule being the final product is 6. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.08 μA and the current value was increased by 0.43 μA only when human HbAlc was added. This result confirmed that this semiconductor sensor selectively detected human HbAlc. The signal intensity was 10.6%.

Example 4

(1) Preparation of mouse anti-human HRG Fab'

A mouse anti-human HRG Fab' solution was obtained by carrying out the same treatment as the treatment in Example 1 (2) to a mouse anti-human HRG antibody (manufactured by USCN Business Co., Ltd.). The concentration of the obtained solution was measured by NanoDrop 2000 and the concentration was found to be 0.32 mg/mL.

Electrophoresis was carried out to 10 μL of the obtained solution by the same method as the method carried out in Example 1 (2) for F $(ab')_2$. Moreover, gel and mouse anti-human HRG Fab' were stained with Quick CBB Plus (trade name, manufactured by Wako Pure Chemical Corporation). Thereafter, the gel was decolorized with pure water and the band of the mouse anti-human HRG Fab' was compared with the molecular weight marker. As a result, the band of the mouse anti-human HRG Fab' existed in the region between a band of 37,000 and a band of 50,000 of the

molecular weight marker. In addition, existence of no defects such as smiling in the electrophoresis was confirmed. In such a case, it has been known that a good correlation is found between the molecular weight estimated by electrophoresis and the molecular weight determined by MALDI-TOF-MS measurement. Therefore, it is considered that any value between 37,000 and 50,000 can be obtained even in the case where the molecular weight of the obtained mouse anti-human HRG Fab' is determined using MALDI-TOF-MS.

(2) Preparation of semiconductor sensor

The precursor of the target recognition molecule [A] was attached to the CNT composite serving as the semiconducting component by the same method as the method in Example 1 (5) for the semiconductor element B prepared in Example 2 (2). Subsequently, Fab' was immobilized on the semiconductor layer by the same method as the method in Example 1 (5) except that anti-human HRG Fab' was used instead of anti-human HbAlc Fab'. Moreover, the surface was protected with BSA by the same method as the method in Example 1 (5) to give a semiconductor sensor.

(3) Evaluation as semiconductor sensor

The semiconductor sensor was evaluated in the same method as the method in Example 1 (6) except that human HRG (manufactured by PeproTech, Inc.) was used instead of human HbAlc. The current Id at the start of the measurement was 4.12 μA and the current value was increased by 0.79 μA only when human HRG was added. This result confirmed that this semiconductor sensor selectively detected human HRG. The signal intensity was 19.2%.

Example 5

(1) Preparation of semiconductor sensor

The electrodes were formed in the same method as the method in Example 1 (3) except that the third electrode 7 was not formed. In addition, the semiconductor layer was formed by the same method as the method in Example 1 (3) except that the semiconducting component solution B prepared in Example 2 (1) was used instead of using the semiconducting component solution A. Subsequently, the precursor of the target recognition molecule [A] was attached to the CNT composite serving as the semiconducting component by the same method as the method in Example 1 (5) and, in addition, mouse anti-human HbAlc Fab' was immobilized on the semiconductor layer by the same method as the method in Example 1 (5). Moreover, the surface was protected with BSA by the same method as the method in Example 1 (5) to give a semiconductor sensor.

(2) Evaluation as semiconductor sensor

The obtained semiconductor sensor was evaluated as a semiconductor sensor by the same method as the method in Example 1 (6). The current Id at the start of the measurement was 1.46 μA and the current value was increased by 0.27 μA only when human HbAlc was added. This result confirmed that this semiconductor sensor selectively detected human HbAlc. The signal intensity was 18.5%.

Example 6

(1) Synthesis of conjugated polymer [B]

Into 10 mL of a 10 mM acetate buffer solution (manufactured by GE Healthcare Co., Ltd.) in which pH was adjusted to 4.5, 10 mg of poly[3-(5-carboxypentyl)thiophene-2,5-diyl] (manufactured by Rieke Metals Inc.) was dissolved. To the resultant mixture, 1 mL of an aqueous solution of 75 mg/mL EDC (manufactured by GE Healthcare Co., Ltd.) and 1 mL of an aqueous solution of 11.5 mg/mL NHS (manufactured by GE Healthcare Co., Ltd.) were added and the resultant mixture was stirred at room temperature for 1 hour. To this mixture, 17.8 mg of N-(2-aminoethyl)maleimide hydrochloride (manufactured by Tokyo Chemical Industry Co., Ltd.) was added and the resultant mixture was further stirred for 1 hour. The solid precipitated by ice cooling was collected by filtration with a Kiriyama funnel and dried under reduced pressure to give 0.54 mg of a conjugated polymer [B].

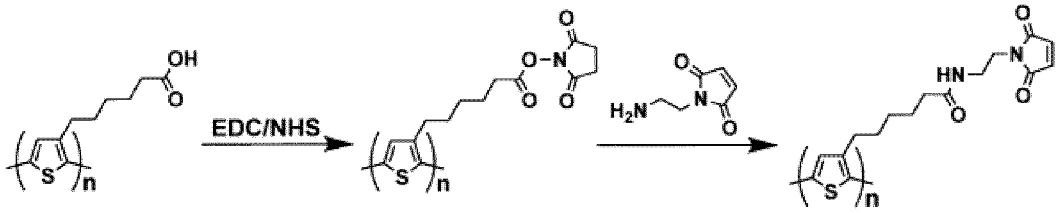


(2) Preparation of semiconducting component solution C

To 15 mL of chloroform, 1.5 mg of CNT (manufactured by Carbon Nanotechnology Inc., single-walled CNT, including 95% by weight of semiconductor-type CNT) and 1.5 mg of the conjugated polymer [B] prepared in (1) were added. The resultant mixture was stirred by ultrasonic wave at an output of 250 W for 30 minutes using an ultrasonic homogenizer (VCX-500, manufactured by TOKYO RIKAKIKAI CO, LTD.) while cooling with ice to give a CNT composite dispersion liquid C (a concentration of CNT composite relative to solvent of 0.08 g/L).

Subsequently, a semiconducting component solution for forming the semiconductor layer was prepared. The CNT dispersion liquid A described above was filtered using a membrane filter (pore size 10 μm, diameter 25 mm, omnipore membrane filter, manufactured by Millipore corporation) to remove the CNT composite having a length of 10 μm or longer. To 5 mL of the obtained filtrate, 27 mL of o-DCB was added to prepare a semiconducting component solution C (a concentration of CNT composite relative to the solvent of 0.01 g/L).

(3) Formation of electrodes and semiconductor layer

The first electrode 2, the second electrode 3, the third electrode 7, and the semiconductor layer 4 were formed to obtain the semiconductor element C by the same method as the method in Example 1 (3) except that the semiconducting component solution C prepared in (1) was used instead of the semiconducting component solution A. The characteristics of the current (Id) between the first electrode 2 and the second electrode 3/the voltage (Vsd) between the first electrode 2 and the second electrode 3 at the time of changing the voltage (Vg) of the third electrode 7 of the semiconductor element C described above were measured under the same conditions as Example 1 (3). The on/off ratio when the gate voltage Vg of the third electrode 7 was changed from 0 V to -1 V was $5 \times 10^3$.

(4) Immobilization of Fab' and surface protection treatment

The Fab' solution of the mouse anti-human HbAlc antibody prepared in Example 1 (2) was diluted with PBS so that the concentration of the solution was 0.10 mg/mL and the semiconductor layer of the semiconductor element C was immersed into the diluted Fab' solution at 4°C for 18 hours. This caused the reaction of the mercapto group in the hinge region of Fab' and the maleimide group of the conjugated polymer [B] to immobilize Fab' on the semiconductor layer. In this case, the thiophene ring of the target recognition molecule is attached to CNT and thus the number of atoms from the atom bonded to the atom originated from $Ar^2$ to the atom bonded to the atom originated from Fab' of the mouse anti-human HbAlc antibody serving as the target capture body in the linking group L is 12. Moreover, the surface was protected with BSA by the same method as the method in Example 1 (5) to give a semiconductor sensor.

(5) Evaluation as semiconductor sensor

The obtained semiconductor sensor was evaluated as a semiconductor sensor by the same method as the method in Example 1 (6). The current Id at the start of the measurement was 3.60 μA and the current value was increased by 0.53 μA only when human HbAlc was added. This result confirmed that this semiconductor sensor selectively detected human HbAlc. The signal intensity was 14.7%.

Example 7

(1) Preparation of semiconducting component solution D

To 15 mL of NMP, 1.5 mg of CNT (manufactured by Carbon Nanotechnology Inc., single-walled CNT, including 95% by weight of semiconductor-type CNT) was added. The resultant mixture was stirred by ultrasonic wave at an output of 250 W for 1 hour using an ultrasonic homogenizer while cooling with ice to give a semiconducting component solution D.

(2) Formation of electrodes and semiconductor layer

The electrodes and the semiconductor layer were formed to obtain semiconductor element D by the same method as the method in Example 1 (3) except that 2 μL of the semiconducting component solution D was dropped into the channel part by using Pipetman instead of dropping the semiconducting component solution A using the inkjet device. The characteristics of the current (Id) between the first electrode 2 and the second electrode 3/the voltage (Vsd) between the first electrode 2 and the second electrode 3 at the time of changing the voltage (Vg) of the third electrode 7 of the semiconductor element D were measured under the same conditions as Example 1 (3). The on/off ratio when Vg was changed from 0 V to -1 V was $1 \times 10^3$.

(3) Preparation of semiconductor sensor

The precursor of the target recognition molecule [A] was attached to CNT serving as the semiconducting component by the same method as the method in Example 1 (5) for the semiconductor element D. Moreover, anti-human HbAlc Fab' was immobilized on the semiconductor layer by the same method as the method in Example 1 (5). Moreover, the surface was protected with BSA by the same method as the method in Example 1 (5) to give a semiconductor sensor.

(4) Evaluation as semiconductor sensor

The obtained semiconductor sensor was evaluated as a semiconductor sensor by the same method as the method in Example 1 (6). The current Id at the start of the measurement was 3.61 μA and the current value was increased by 0.55 μA only when human HbA1c was added. This result confirmed that this semiconductor sensor selectively detected human HbAlc. The signal intensity was 15.3%.

Example 8

(1) Synthesis of precursor of target recognition molecule [D]

Into 93.4 mL of DMF, 50 mg of 1-aminomethylpyrene hydrochloride (manufactured by Funakoshi Co., Ltd.) and 57.57 mg of N-(4-maleimidebutyryloxy)succinimide (manufactured by DOJINDO LABORATORIES) were dissolved. To this solution, 55.46 mg of sodium hydrogen carbonate was added and the resultant mixture was stirred for 6 hours. Thereafter, 408 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 53.31 mg of a white solid. The mass spectrum (JMS-Q1000TD, manufactured by JEOL Ltd.) of the obtained white solid was measured and M + 1 was found to be 397. This confirmed that the white solid was the following precursor of the target recognition molecule [D].

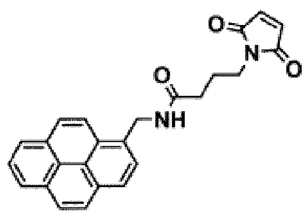

(2) Preparation of semiconductor sensor

The precursor of the target recognition molecule [D] was attached to the CNT composite serving as the semiconducting component by the same method as the method in Example 2 (4) except that the precursor of the target recognition molecule [D] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [D] is attached to CNT and thus the number of atoms from the atom bonded to the group attached to the semiconducting component to the atom bonded to the atom originated from Fab' of the mouse anti-human HbAlc antibody serving as the target capture body in the linking group L of the target recognition molecule being the final product is 9. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.13 μA and the current value was increased by 0.85 μA only when human HbAlc was added. This result confirmed that this semiconductor sensor selectively detected human HbAlc. The signal intensity was 20.6%.

Example 9

(1) Preparation of semiconductor sensor

The precursor of the target recognition molecule [A] was attached to the CNT composite serving as the semiconducting component by the same method as the method in Example 2 (4) except that the precursor of the target recognition molecule [A] was used instead of the precursor of the target recognition molecule [B]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [A] is attached to CNT and thus the number of atoms from the atom bonded to the group attached to the semiconducting component to the atom bonded to the atom originated from Fab' of the mouse anti-human HbAlc antibody serving as the target capture body in the linking group L of the target recognition molecule being the final product is 13. Moreover, the surface was protected with BSA by the same method as the method in Example 1 (5) to give a semiconductor sensor.

(2) Evaluation as semiconductor sensor

The obtained semiconductor sensor was evaluated as a semiconductor sensor by the same method as the method in Example 1 (6). The current Id at the start of the measurement was 4.15 μA and the current value was increased by 0.87 μA only when human HbAlc was added. This result confirmed that this semiconductor sensor selectively detected human HbAlc. The signal intensity was 21.0%.

Example 10

(1) Preparation of semiconductor sensor

The precursor of the target recognition molecule [A] was attached to the CNT composite serving as the semiconducting component by the same method as the method in Example 1 (5) for the semiconductor element C prepared in Example 6. Moreover, mouse anti-human HbAlc Fab' was immobilized on the semiconductor layer by the same method as the method in Example 1 (5). Moreover, the surface was protected with BSA by the same method as the method in Example 1 (5) to give a semiconductor sensor.

(2) Evaluation as semiconductor sensor

The obtained semiconductor sensor was evaluated as a semiconductor sensor by the same method as the method in Example 1 (6). The current Id at the start of the measurement was 4.17 μA and the current value was increased by 0.88 μA only when human HbAlc was added. This result confirmed that this semiconductor sensor selectively detected human HbAlc. The signal intensity was 21.2%.

Example 11

(1) Synthesis of precursor of target recognition molecule [E]

Into 93.4 mL of DMF, 50 mg of 1-aminomethylpyrene hydrochloride (manufactured by Funakoshi Co., Ltd.) and 78.56 mg of N-(11-maleimideundecanoyloxy)succinimide (manufactured by DOJINDO LABORATORIES) were dissolved. To this solution, 48.33 mg of sodium hydrogen carbonate was added and the resultant mixture was stirred for 6 hours. Thereafter, 250 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 76.14 mg of a white solid. The mass spectrum (JMS-Q1000TD, manufactured by JEOL Ltd.) of the obtained white solid was measured and M + 1 was found to be 495. This confirmed that the white solid was the following precursor of the target recognition molecule [E].

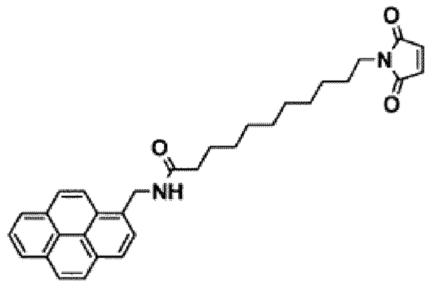

(2) Preparation of semiconductor sensor

The precursor of the target recognition molecule [E] was attached to the CNT composite serving as the semiconducting component by the same method as the method in Example 2 (4) except that the precursor of the target recognition molecule [E] was used instead of the precursor of the target recognition molecule [B]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [E] is attached to CNT and thus the number of atoms from the atom bonded to the group attached to the semiconducting component to the atom bonded to the atom originated from Fab' of the mouse anti-human HbAlc antibody serving as the target capture body in the linking group L of the target recognition molecule being the final product is 16. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.15 μA and the current value was increased by 0.85 μA only when human HbAlc was added. This result confirmed that this semiconductor sensor selectively detected human HbAlc. The signal intensity was 20.4%.

Example 12

(1) Synthesis of precursor of target recognition molecule [F]

Into 500 mL of diethyl ether, 1.01 g of 2-(4-bromophenyl)ethylamine (manufactured by Tokyo Chemical Industry Co., Ltd.) and 497.6 mg of maleic anhydride (manufactured by Tokyo Chemical Industry Co., Ltd.) were dissolved and the resultant mixture was stirred for 24 hours. The precipitated solid was collected by filtration with a Kiriyama funnel and dried under reduced pressure and the weight of the solid was found to be 867.8 mg. The obtained solid and 283.4 mg of sodium acetate (manufactured by Wako Pure Chemical Corporation) were added into 350 mL of acetic anhydride (manufactured by Wako Pure Chemical Corporation) and stirred at 80°C for 1 hour. The reaction solution was cooled with ice to precipitate a solid. Thereafter, the precipitate was collected with a Kiriyama funnel, washed with pure water, and dried under reduced pressure. Recrystallization was carried out using heptane to give 392.7 mg of a solid. The mass spectrum (JMS-Q1000TD, manufactured by JEOL Ltd.) of the obtained solid was measured and M + 1 was found to be 280 and 282 in a ratio of 1:1. This confirmed that the obtained solid was N-[2-(4-bromophenyl)ethyl]maleimide.

Subsequently, into a 100 ml three-necked flask, 350.8 mg of the obtained solid, 338.4 mg of 1-pyreneboronic acid (manufactured by Tokyo Chemical Industry Co., Ltd.), and 168.6 mg of sodium tert-butoxide were placed. To the resultant mixture, 15 mL of o-xylene was added and the inside of the container was purged with nitrogen. To this mixture liquid, 14.6 mg of tetrakis(triphenylphosphine) palladium (0) was added and heated under reflux in an oil bath at 90°C for 3 hours. After completion of the reaction, the reaction mixture was cooled with ice. The precipitated solid was collected by filtration with a Kiriyama funnel, washed with pure water, and dried under reduced pressure. Recrystallization was carried out using heptane to give 309.6 mg of a solid. The mass spectrum (JMS-Q1000TD, manufactured by JEOL Ltd.) of the obtained solid was measured and M + 1 was found to be 402. This confirmed that the white solid was the following precursor of the target recognition molecule [F].

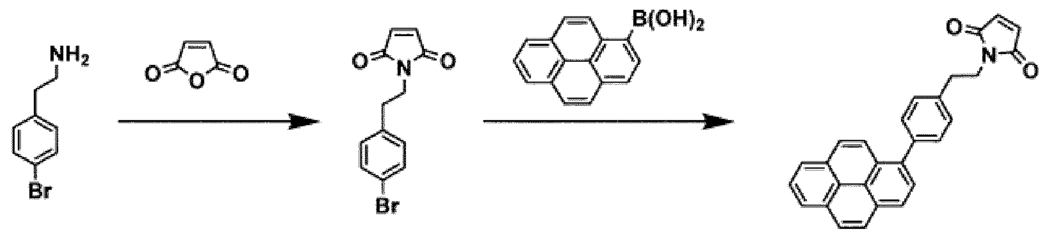

(2) Attachment of precursor of target recognition molecule [F] to semiconducting component and immobilization of Fab'

The precursor of the target recognition molecule [F] was attached to the CNT composite serving as the semiconducting component by the same method as the method in Example 1 (5) except that the precursor of the target recognition molecule [F] was used instead of the precursor of the target recognition molecule [A]. Moreover, Fab' of the mouse anti-human HbAlc antibody prepared in Example 1 (2) was immobilized on the semiconductor layer 4 by the same method as the method in Example 1 (5). In this case, the conjugated systems of the benzene ring and the pyrene ring of the precursor of the target recognition molecule [F] are linked and thus this pyrenylphenyl group can be regarded as $Ar^1$ attached to CNT. Consequently, the number of atom(s) $N^1$ from the atom bonded to the atom originated from $Ar^1$ to the atom bonded to the atom originated from Fab' of the mouse anti-human HbAlc antibody serving as the target capture body in the linking group L of the target recognition molecule being the final product is 5. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.09 μA and the current value was increased by 0.42 μA only when human HbA1c was added. This result confirmed that this semiconductor sensor selectively detected human HbAlc. The signal intensity was 10.2%.

Example 13

(1) Preparation of semiconductor sensor

The silicon wafer (manufactured by Silicon Technology Co., Ltd.) surface on which an oxide film was formed was treated with a UV-ozone generator (manufactured by SEN LIGHTS Corporation) for 30 minutes. Thereafter, the silicon wafer was immersed into a solution of 3-aminopropyltriethoxysilane (manufactured by Wako Pure Chemical Corporation) diluted 10 times with ethanol for 1 hour. The silicon wafer was washed with ethanol and thereafter heated at 120°C for 30 minutes.

Subsequently, the silicon wafer was immersed into a single layer graphene oxide aqueous dispersion liquid (manufactured by Graphene Laboratories Inc.) for 1 hour. Thereafter, the immersed silicon wafer was washed with pure water and dried by nitrogen blowing to give a graphene oxide film. In order to reduce the obtained graphene oxide film, the silicon wafer was placed in a tube furnace (manufactured by Koyo Thermo Systems Co., Ltd.) and treated at 1,100°C for 60 minutes in a mixed gas containing 97% of argon and 3% of hydrogen. The first electrode 2 and the second electrode 3 were formed by the photolithography method, which is the same as the method in Example 1 (3), to the surface of the obtained silicon wafer on which the reduced graphene was formed to prepare a semiconductor element E.

The part between electrodes of the prepared semiconductor element E was immersed into 100 μL of a 0.01 M PBS solution and an Ag/AgCl electrode was inserted into the PBS solution to form a third electrode 7. The characteristics of the current (Id) between the first electrode 2 and the second electrode 3/the voltage (Vsd) between the first electrode 2 and the second electrode 3 at the time of changing the voltage (Vg) of the third electrode 7 of the prepared semiconductor element E were measured. The on/off ratio when Vg was changed from 0 V to -1 V was $3.8 \times 10^3$.

(2) Immobilization of mouse anti-human HbAlc Fab'

The precursor of the target recognition molecule [A] was attached to graphene serving as the semiconducting component by the same method as the method in Example 1 (5). Moreover, Fab' was immobilized on the semiconductor layer by the same method as the method in Example 1 (5). Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 6.29 μA and the current value was increased by 0.71 μA only when human HbA1c was added. This result confirmed that this semiconductor sensor selectively detected human HbAlc. The signal intensity was 11.3%.

Example 14

(1) Synthesis of precursor of target recognition molecule [G]

Into 100 mL of PBS (pH 7.4), 26.78 mg of 1-aminomethylpyrene hydrochloride (manufactured by Funakoshi Co., Ltd.) and 59.58 mg of Sulfo-$AC_5$-SPDP (manufactured by DOJINDO LABORATORIES) were dissolved and the resultant mixture was stirred for 4 hours. Thereafter, the reaction container was cooled with ice. Thereafter, the white precipitate was collected with a Kiriyama funnel, washed with pure water, and dried under reduced pressure to give 43.30 mg of a white solid. The mass spectrum (JMS-Q1000TD, manufactured by JEOL Ltd.) of the obtained white solid was measured and M + 1 was found to be 556. This confirmed that the white solid was the following precursor of the target recognition molecule [G].

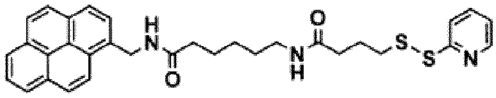

(2) Attachment of precursor of target recognition molecule [G] to semiconducting component and immobilization of Fab'

The precursor of the target recognition molecule [G] was attached to the CNT composite serving as the semiconducting component by the same method as the method in Example 1 (5) except that the precursor of the target recognition molecule [G] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). This caused the reaction of the mercapto group in the hinge region of Fab' and the pyridyldisulfide group in the precursor of the target recognition molecule [G] to eliminate pyridine-2(1H)-thione and, at the same time, to immobilize Fab' on the semiconductor layer. In this case, the pyrene ring of the precursor of the target recognition molecule [G] is attached to CNT and thus the number of atoms from the atom bonded to the group attached to the semiconducting component to the atom bonded to the atom originated from Fab' of the mouse anti-human HbAlc antibody serving as the target capture body in the linking group $L^2$ of the target recognition molecule being the final product is 14. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.13 μA and the current value was increased by 0.83 μA only when human HbA1c was added. This result confirmed that this semiconductor sensor selectively detected human HbAlc. The signal intensity was 20.2%.

Example 15

(1) Synthesis of precursor of target recognition molecule [H]

Into 100 mL of DMF, 26.78 mg of 1-aminomethylpyrene hydrochloride (manufactured by Funakoshi Co., Ltd.) and 54.47 mg of dithiobis(succinimidyl octanoate) (manufactured by DOJINDO LABORATORIES) were dissolved. To this solution, 39.50 mg of sodium hydrogen carbonate was added and the resultant mixture was stirred for 6 hours. Thereafter, 500 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 51.55 mg of a white solid. The mass spectrum (JMS-Q1000TD, manufactured by JEOL Ltd.) of the obtained white solid was measured and M + 1 was found to be 661. This confirmed that the white solid was the following intermediate [A].

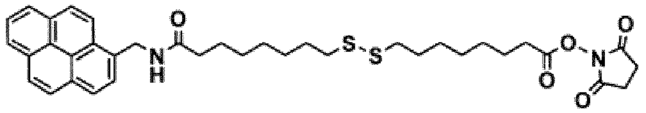

Subsequently, 49.57 mg of the obtained solid and 136.02 mg of N-(2-aminoethyl)maleimide hydrochloride (manufactured by Tokyo Chemical Industry Co., Ltd.) were dissolved into 70 mL of DMF. To the resultant mixture, 27.66 mg of sodium hydrogen carbonate was added and the resultant mixture was stirred for 6 hours. Thereafter, 350 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 38.43 mg of a white solid. The mass spectrum (JMS-Q1000TD, manufactured by JEOL Ltd.) of the obtained white solid was measured and M + 1 was found to be 686. This confirmed that the white solid was the following precursor of the target recognition molecule [H].

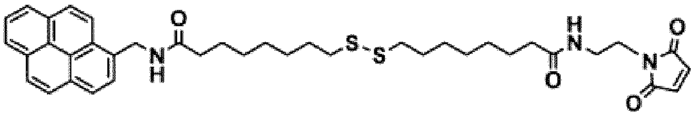

(2) Attachment of precursor of target recognition molecule [H] to semiconducting component and immobilization of Fab'

The precursor of the target recognition molecule [H] was attached to the CNT composite serving as the semiconducting component by the same method as the method in Example 1 (5) except that the precursor of the target recognition molecule [H] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [H] is attached to CNT and thus the number of atoms from the atom bonded to the group attached to the semiconducting component to the atom bonded to the atom originated from Fab' of the mouse anti-human HbAlc antibody serving as the target capture body in the linking group $L^2$ of the target recognition molecule being the final product is 26. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.10 μA and the current value was increased by 0.45 μA only when human HbA1c was added. This result confirmed that this semiconductor sensor selectively detected human HbAlc. The signal intensity was 10.9%.

Example 16

(1) Synthesis of precursor of target recognition molecule [I]

Into 100 mL of DMF, 66.03 mg of the intermediate [A] obtained by the method in Example 15 (1) and 25.61 mg of N-(6-aminohexyl)maleimide hydrochloride (manufactured by Carbosynth Ltd.) were dissolved. To this solution, 39.51 mg of sodium hydrogen carbonate was added and the resultant mixture was stirred for 6 hours. Thereafter, 500 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 56.38 mg of a white solid. The mass spectrum (JMS-Q1000TD, manufactured by JEOL Ltd.) of the obtained white solid was measured and M + 1 was found to be 742. This confirmed that the white solid was the following precursor of the target recognition molecule [I].

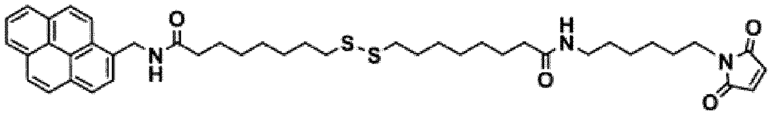

(2) Attachment of precursor of target recognition molecule [I] to semiconducting component and immobilization of Fab'

The precursor of the target recognition molecule [I] was attached to the CNT composite serving as the semiconducting component by the same method as the method in Example 1 (5) except that the precursor of the target recognition molecule [I] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [I] is attached to CNT and thus the number of atoms from the atom bonded to the group attached to the semiconducting component to the atom bonded to the atom originated from Fab' of the mouse anti-human HbAlc antibody serving as the target capture body in the linking group $L^2$ of the target recognition molecule being the final product is 30. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.09 μA and the current value was increased by 0.43 μA only when human HbA1c was added. This result confirmed that this semiconductor sensor selectively detected human HbAlc. The signal intensity was 10.4%.

Example 17

(1) Synthesis of precursor of target recognition molecule [J]

By mixing water and tert-butyl alcohol (manufactured by Tokyo Chemical Industry Co., Ltd.) at a volume ratio of 1:2, 500 mL of a mixed solvent was prepared. To this mixed solvent, 113.20 mg of 1-ethynylpyrene (manufactured by Tokyo Chemical Industry Co., Ltd.), 109.13 mg of 11-azido-3,6,9-trioxaundecan-1-amine (manufactured by Tokyo Chemical Industry Co., Ltd.), 1.27 mg of copper (II) sulfate pentahydrate (manufactured by Wako Pure Chemical Corporation), and 9.92 mg of sodium L-ascorbate (manufactured by Tokyo Chemical Industry Co., Ltd.) were added and the resultant mixture was stirred at room temperature for 18 hours. Thereafter, the reaction container was cooled with ice for 1 hour to sufficiently generate white precipitate. Thereafter, the white precipitate was collected with a Kiriyama funnel, washed with pure water, and dried under reduced pressure to give 190.98 mg of a white solid. The mass spectrum (JMS-Q1000TD, manufactured by JEOL Ltd.) of the obtained white solid was measured and M + 1 was found to be 445. In this reaction using the copper catalyst, one isomer is selectively produced from two possible regioisomers. Therefore, the obtained solid was confirmed to be the following intermediate [B].

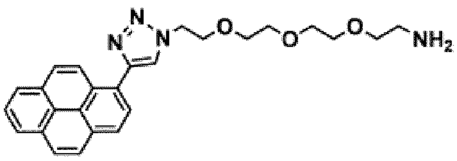

Into 50 mL of diethyl ether, 111.10 mg of the obtained intermediate [B] and 24.52 mg of maleic anhydride (manufactured by Tokyo Chemical Industry Co., Ltd.) were dissolved and the resultant mixture was stirred for 24 hours. The precipitated solid was collected by filtration with a Kiriyama funnel and dried under reduced pressure and the weight of the solid was found to be 118.52 mg. The obtained solid and 15.16 mg of sodium acetate (manufactured by Wako Pure Chemical Corporation) were added into 20 mL of acetic anhydride (manufactured by Wako Pure Chemical Corporation) and the resultant mixture was stirred at 80°C for 1 hour. The reaction solution was cooled with ice to precipitate a solid. Thereafter, the precipitate was collected with a Kiriyama funnel, washed with pure water, and dried under reduced pressure. Recrystallization was carried out using heptane to give 48.78 mg of a solid. The mass spectrum (JMS-Q1000TD, manufactured by JEOL Ltd.) of the obtained white solid was measured and M + 1 was found to be 525. This confirmed that the white solid was the following precursor of the target recognition molecule [J].

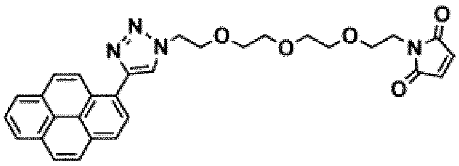

(2) Attachment of precursor of target recognition molecule [J] to semiconducting component and immobilization of Fab'

The precursor of the target recognition molecule [J] was attached to the CNT composite serving as the semiconducting component by the same method as the method in Example 1 (5) except that the precursor of the target recognition molecule [J] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [J] is attached to CNT and thus the number of atoms from the atom bonded to the group attached to the semiconducting component to the atom bonded to the atom originated from Fab' of the mouse anti-human HbAlc antibody serving as the target capture body in the linking group $L^2$ of the target recognition molecule being the final product is 17. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.11 μA and the current value was increased by 0.65 μA only when human HbA1c was added. This result confirmed that this semiconductor sensor selectively detected human HbAlc. The signal intensity was 15.8%.

Comparative Example 1

(1) Preparation of semiconductor sensor

The semiconductor layer of the semiconductor element B prepared in Example 2 (2) was immersed for 18 hours at 4°C into a solution prepared by diluting the Fab' solution of the mouse anti-human HbAlc antibody prepared in Example 1 (2) with PBS so that the concentration of the solution was 0.10 mg/mL. This resulted in physically adsorbing the mouse antihuman HbAlc Fab' to the CNT composite serving as the semiconducting component not through the linking group. Moreover, the surface was protected with BSA by the same method as the method in Example 1 (5) to give a semiconductor sensor.

(2) Evaluation as semiconductor sensor

The obtained semiconductor sensor was evaluated as a semiconductor sensor by the same method as the method in Example 1 (6). The current Id at the start of the measurement was 4.03 μA and the current value was increased by 0.16 μA only when human HbA1c was added. The signal intensity was 4.0%.

The reasons why the signal intensity of Comparative Example 1 is smaller than the signal intensities of Examples 2, 3, 8, 9, 11 and 12 are considered to be as follows. (I) At the time of physically adsorbing the target capture body on CNT, the shape of the target capture body is deformed by the interaction with CNT to lose the target capture ability and (II) even when the target capture ability remains, the target capture body has no mobility and thus many target capture bodies that cannot capture the target exist. As the number of targets to be captured decreases, the signal intensity also decreases accordingly.

No significant difference is recognized in the initial current value of Comparative Example 1 compared with Examples 2, 3, 8, 9, 11, and 12. This result is presumably because the semiconducting component CNT to which P3HT, which is the same semiconducting component as the semiconducting component in Examples, is attached is used, and thus no significant difference in conductivity is generated.

Comparative Example 2

(1) Preparation of semiconductor sensor

PBSE was attached to the CNT composite serving as the semiconducting component of the semiconductor

element B by the same method as the method in Example 2 (4) except that PBSE (manufactured by Sigma-Aldrich Co. LLC) was used instead of the precursor of the target recognition molecule [B]. Moreover, Fab' of the mouse anti-human HbAlc antibody prepared in Example 1 (2) was immobilized by the same method as the method in Example 1 (5). In this case, the number of atoms from the atom bonded to the atom originated from Ar to the atom bonded to the atom originated from X in the linking group L is 4.

Moreover, the surface was protected with BSA by the same method as the method in Example 1 (5) to give a semiconductor sensor.

(2) Evaluation as semiconductor sensor

The obtained semiconductor sensor was evaluated as a semiconductor sensor by the same method as the method in Example 1 (6). The current Id at the start of the measurement was 4.06 μA and the current value was increased by 0.17 μA only when human HbAlc was added. The signal intensity was 4.3%.

The signal intensity is larger than that of Comparative Example 1. This is presumably because the target capture body that lost the target capture ability due to physical adsorption to the CNT and deformation in Comparative Example 1 acquired the target capture ability by immobilizing the target capture body through PBSE in Comparative Example 2. The signal intensity, however, is still small as compared with Examples 2, 3, 8, 9, 11 and 12. This is presumably because the number of atoms from the atom bonded to the group attached to the CNT to the atom bonded to the atom originated from the target capture body is 4, which is insufficient for the target capture body to exhibit the target capture ability.

Comparative Example 3

(1) Preparation of semiconductor sensor

The antibody was immobilized on the semiconductor layer by the same method as the method in Comparative Example 2 except that a mouse anti-human HbAlc whole antibody not subjected to Fab' treatment was used instead of anti-HbAlc Fab'. Moreover, the surface was protected with BSA by the same method as the method in Example 1 (5) to give a semiconductor sensor.

(2) Evaluation as semiconductor sensor

The obtained semiconductor sensor was evaluated as a semiconductor sensor by the same method as the method in Example 1 (6). The current Id at the start of the measurement was 4.04 μA and the current value was increased by 0.11 μA only when human HbAlc was added. The signal intensity was 2.7%.

The signal intensity of Comparative Example 3 was smaller than that of Comparative Example 2. This is presumably because the whole antibody with a high molecular weight was used as it was without reducing the size by Fab' forming treatment as the target capture body. In other words, at the time of capturing the target with the target capture body, the distance from the CNT to the target substance is longer than in the case where Fab' is used and the electric field that affects CNT is weakened.

Comparative Example 4

(1) Preparation of semiconductor sensor

A semiconductor sensor was prepared by the same method as the method in Example 13 except that PBSE was used instead of the precursor of the target recognition molecule [A].

(2) Evaluation as semiconductor sensor

The obtained semiconductor sensor was evaluated as a semiconductor sensor by the same method as the method in Example 1 (6). The current Id at the start of the measurement was 5.32 μA and the current value was increased by 0.22 μA only when human HbAlc was added. The signal intensity was 4.1%.

In the measurement result of Example 4, the signal intensity is smaller than that of the measurement result of Comparative Example 13. The reason for this is considered to be the same as the reason why the signal intensity of Comparative Example 1 is smaller than those of Examples 2, 3, 8, 9, 11 and 12.

Comparative Example 5

(1) Synthesis of precursor of target recognition molecule [K]

Into 100 mL of DMF, 62.88 mg of dithiobis(succinimidyl undecanoate) (manufactured by DOJINDO LABORATORIES) and 7.72 mg of 2-aminoethanethiol (manufactured by Tokyo Chemical Industry Co., Ltd.) were dissolved and the resultant mixture was stirred for 4 hours. Thereafter, 500 mL of pure water was added dropwise. The resultant white precipitate was collected by a Kiriyama funnel and washed with pure water. The white precipitate was dried under reduced pressure to give 53.77 mg of a white solid. Subsequently, 44.32 mg of the obtained white solid and 22.30 mg of N-(1-pyrenyl)maleimide (manufactured by Tokyo Chemical Industry Co., Ltd.) were dissolved into 75 mL of DMF and the resultant mixture was stirred for 8 hours. After distilling off the solvent of the reaction solution to concentrate, the reaction product, unreacted substances, and impurities were separated by silica gel column chromatography using toluene as an eluent. The solvent of the fraction containing the reaction product was distilled off and the residue was dried in a vacuum to give 47.82 mg of a white solid. The mass spectrum (JMS-Q1000TD, manufactured by JEOL Ltd.) of the obtained white solid was measured and M + 1 was found to be 888. This confirmed that the white solid was the following precursor of the target recognition molecule [K].

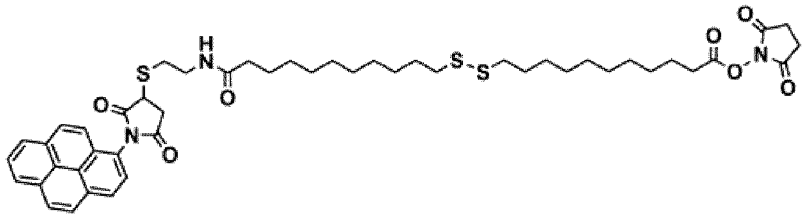

(2) Attachment of precursor of target recognition molecule [K] to semiconducting component and immobilization of Fab'

The precursor of the target recognition molecule [K] was attached to the CNT composite serving as the semiconducting component by the same method as the method in Example 1 (5) except that the precursor of the target recognition molecule [K] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, Fab' of the mouse anti-human HbAlc antibody does not react with the hinge region of Fab' of the mouse anti-human HbAlc antibody but randomly reacts with a plurality of amino groups possessed by Fab' of the mouse anti-human HbAlc antibody. The pyrene ring of the precursor of the target recognition molecule [K] is attached to CNT and thus the number of atoms from the atom bonded to the group attached to the semiconducting component to the atom bonded to the atom originated from Fab' of the mouse anti-human HbAlc antibody serving as the target capture body in the linking group in the target recognition molecule being the final product is 31. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Example 1 (6). The current Id at the start of the measurement was 4.05 μA and the current value was increased by 0.17 μA only when human HbAlc was added. This result confirmed that this semiconductor sensor selectively detected human HbAlc. The signal intensity was 4.2%.

Comparative Example 6

(1) Synthesis of 1-pyrenepropylamine

After the atmosphere in the reaction container was purged with nitrogen, 2.20 g of 1-pyrenebutyric acid (manufactured by Tokyo Chemical Industry Co., Ltd.) was dissolved into a mixed solvent of 70 mL of dichloromethane and 100 μL of DMF. Subsequently, to the resultant mixture, 750 μL of oxalyl chloride (manufactured by Tokyo Chemical Industry Co., Ltd.) was added dropwise with stirring and the resultant mixture was stirred as it was at room temperature for 40 minutes. Thereafter, the solvent of the reaction solution was distilled off and the residue was dried in a vacuum. The obtained solid was dissolved into 10 mL of acetone and the resultant solution was added dropwise to 2 mL of a 0.3

g/mL sodium azide aqueous solution at 0°C with stirring. After completion of the dropwise addition, the temperature of the reaction solution was gradually raised to room temperature and the reaction solution was stirred as it was at room temperature for 1 hour. Thereafter, the reaction solution was poured into 30 mL of pure water. The resultant precipitate was collected by filtration with a Kiriyama funnel, washed with pure water, and dried in a vacuum. The obtained solid was suspended in 10 mL of benzene and the temperature of the resultant suspension was raised to 80°C with stirring, followed by stirring the suspension as it was for 90 minutes. After termination of gas generation was confirmed, the reaction solution was cooled to room temperature. The solvent was distilled off and the residue was dried in a vacuum. Subsequently, the obtained oily product was dissolved into 10 mL of tetrahydrofuran and thereafter the resultant solution was heated to 60°C. Concentrated hydrochloric acid (manufactured by Wako Pure Chemical Corporation) was added dropwise to the solution with stirring until no gas was generated. After the solution was further stirred for 30 minutes, a 10% sodium hydroxide aqueous solution (manufactured by Wako Pure Chemical Corporation) was added until the solution turned into basic. The mixture was extracted with chloroform using a separating funnel and the obtained chloroform solution was dried over anhydrous magnesium sulfate (manufactured by Wako Pure Chemical Corporation). Thereafter, the solvent was distilled off and the residue was dried in a vacuum. The mass spectrum (JMS-Q1000TD, manufactured by JEOL Ltd.) of the obtained solid was measured and M + 1 was found to be 260. This confirmed that the solid was 1-pyrenepropylamine.

(2) Synthesis of target recognition molecule [L]

In the target recognition molecule [L], biotin is the target capture body. Biotin is a compound having the following structure and having a molecular weight of 244.31. Avidin being a protein recognizes the structure of biotin and bonds to biotin.

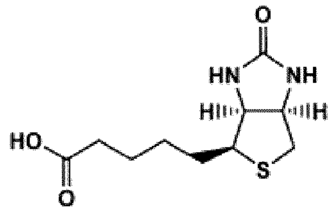

Into 100 mL of DMF, 25.92 mg of 1-pyrenepropylamine synthesized by the method in Comparative Example 6 (1) and 34.14 mg of Biotin-OSu (trade name, manufactured by DOJINDO LABORATORIES) were dissolved. To this solution, 39.50 mg of sodium hydrogen carbonate was added and the resultant mixture was stirred for 6 hours. Thereafter, 500 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 40.57 mg of a white solid. The mass spectrum (JMS-Q1000TD, manufactured by JEOL Ltd.) of the obtained white solid was measured and M + 1 was found to be 486. This confirmed that the white solid was the following target recognition molecule [L].

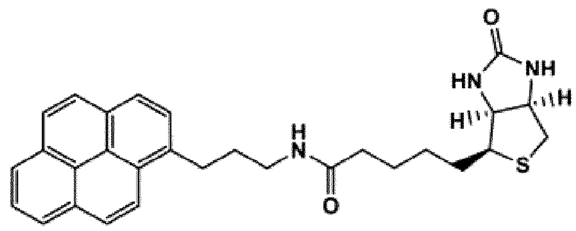

(3) Immobilization of target recognition molecule [L] to semiconducting component

Subsequently, 10 mg of the target recognition molecule [L] synthesized in (2) was dissolved into 10 mL of acetonitrile (manufactured by Wako Pure Chemical Corporation) and the semiconductor layer of the semiconductor element B formed in Example 2 (2) was immersed into the resultant solution for 4 hours to attach the target recognition molecule [L] to the CNT composite serving as the semiconducting component. Thereafter, the semiconductor layer was sufficiently rinsed with acetonitrile and 0.01 M PBS.

Accordingly, a semiconductor layer having a CNT composite to which the target recognition molecule in which a biotin structure having an alkyl group moiety to a carbonyl group and having a molecular weight of 227 acted as the

target capture body was attached was obtained. In this case, the pyrene ring of the target recognition molecule [L] is attached to CNT and thus the number of atoms from the atom bonded to the group attached to the semiconducting component to the atom bonded to the atom originated from biotin serving as the target capture body in the linking group L of the target recognition molecule [L] is 4. Moreover, the surface was protected with BSA by the same method as the method in Example 1 (5) to give a semiconductor sensor.

(4) Evaluation as semiconductor sensor

The obtained semiconductor sensor was evaluated as a semiconductor sensor by the same measurement conditions as the measurement conditions in Example 1 (6). In other words, the semiconductor layer of the prepared semiconductor sensor was immersed into 100 μL of a 0.01 M PBS solution to measure the current value (Id) flowing between the first electrode 2 and the second electrode 3. At this time, conditions in which the voltage (Vsd) between the first electrode 2 and the second electrode 3 was -0.2 V and the voltage (Vg) between the first electrode 2 and the third electrode 7 was -0.6 V were set. The current Id (initial current value) at the start of the measurement was 4.01 μA.

To the 0.01 M PBS solution into which the semiconductor layer 4 was immersed, 20 μL of a solution in which BSA was dissolved into a 0.01 M PBS solution so that the concentration of the BSA solution was 100 μL/mL, 20 μL of a solution in which human HbAlc (manufactured by manufactured by BBI Solutions) was dissolved into a 0.01 M PBS solution so that the concentration of the human HbAlc solution was 100 μL/mL, and 20 μL of a solution in which avidin (manufactured by Wako Pure Chemical Corporation) was dissolved into a 0.01 M PBS solution so that the concentration of the avidin solution was 100 μL/mL each were added 3 minutes, 6 minutes, and 9 minutes after the start of the measurement, respectively. Then, the current value decreased by 0.14 μA only when the solution including avidin was added. This result confirmed that this semiconductor sensor selectively detected avidin. The signal intensity was 3.4%.

Here, the current value increases when human HbAlc is detected whereas the current value decreases when avidin is detected. This is due to the isoelectric point of each of the target substances. In other words, human HbAlc is negatively charged in a neutral PBS solution because the isoelectric point of human HbAlc is on the acidic side, whereas avidin is positively charged in a neutral PBS solution because the isoelectric point of avidin is on the basic side. Consequently, the trend of the increase and decrease of the current values when human HbAlc is detected by the semiconductor sensor is opposite to the trend when avidin is detected.

Comparative Example 7

(1) Synthesis of target recognition molecule [M]

Into 100 mL of DMF, 27.34 mg of 1-pyrenebutylamine (manufactured by SANTA CRUZ BIOTECHNOLOGY, Inc.) and 34.14 mg of Biotin-OSu (trade name, manufactured by DOJINDO LABORATORIES) were dissolved. To this solution, 39.52 mg of sodium hydrogen carbonate was added and the resultant mixture was stirred for 6 hours. Thereafter, 500 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 42.04 mg of a white solid. The mass spectrum (JMS-Q1000TD, manufactured by JEOL Ltd.) of the obtained white solid was measured and M + 1 was found to be 500. This confirmed that the white solid was the following target recognition molecule [M].

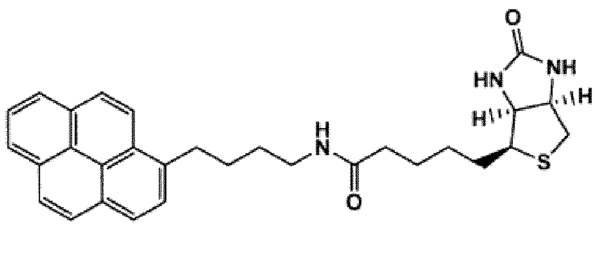

(2) Immobilization of target recognition molecule [M] to semiconducting component

The target recognition molecule [M] was attached to the CNT composite serving as the semiconducting component by the same method as the method in Comparative Example 6 (3) except that the target recognition molecule [M] was used instead of the target recognition molecule [L]. In this case, the pyrene ring of the target recognition molecule [M] is attached to CNT and thus the number of atoms from the atom bonded to the group attached to the semiconducting component to the atom bonded to the atom originated from biotin serving as the target capture body in the linking group

in the target recognition molecule is 5. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Comparative Example 6 (4). The current Id at the start of the measurement was 4.03 μA and the current value was decreased by 0.15 μA only when avidin was added. This result confirmed that this semiconductor sensor selectively detected avidin. The signal intensity was 3.6%.

Comparative Example 8

(1) Synthesis of target recognition molecule [N]

Into 100 mL of DMF, 27.34 mg of 1-pyrenebutylamine (manufactured by SANTA CRUZ BIOTECHNOLOGY, Inc.) and 60.67 mg of Biotin-SS-Sulfo-OSu (trade name, manufactured by DOJINDO LABORATORIES) were dissolved. To this solution, 39.53 mg of sodium hydrogen carbonate was added and the resultant mixture was stirred for 6 hours. Thereafter, 500 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 56.96 mg of a white solid. The mass spectrum (JMS-Q1000TD, manufactured by JEOL Ltd.) of the obtained white solid was measured and M + 1 was found to be 663. This confirmed that the white solid was the following target recognition molecule [N].

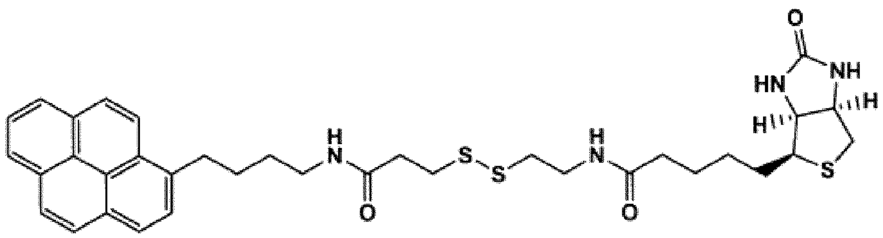

(2) Immobilization of target recognition molecule [N] to semiconducting component

The target recognition molecule [N] was attached to the CNT composite serving as the semiconducting component by the same method as the method in Comparative Example 6 (3) except that the target recognition molecule [N] was used instead of the target recognition molecule [L]. In this case, the pyrene ring of the target recognition molecule [N] is attached to CNT and thus the number of atoms from the atom bonded to the group attached to the semiconducting component to the atom bonded to the atom originated from biotin serving as the target capture body in the linking group in the target recognition molecule is 13. Moreover, the surface was protected with BSA by the same method as the method in Example 1 (5) to give a semiconductor sensor.

(3) Evaluation as semiconductor sensor

The obtained semiconductor sensor was evaluated as a semiconductor sensor by the same method as the method in Comparative Example 6 (4). The current Id at the start of the measurement was 4.02 μA and the current value was decreased by 0.14 μA only when avidin was added. This result confirmed that this semiconductor sensor selectively detected avidin. The signal intensity was 3.5%.

Comparative Example 9

(1) Synthesis of intermediate [C]

Into 100 mL of DMF, 29.73 mg of N-(1-pyrenyl)maleimide (manufactured by Tokyo Chemical Industry Co., Ltd.) and 26.38 mg of 11-amino-1-undecanethiol hydrochloride (manufactured by DOJINDO LABORATORIES) were dissolved. The resultant mixture was stirred for 12 hours with cooling with ice. Thereafter, the solvent of the reaction solution was distilled off to concentrate. Thereafter, the reaction product, unreacted substances, and impurities were separated by silica gel column chromatography using toluene as an eluent. The solvent of the fraction containing the reaction

product was distilled off and the residue was dried in a vacuum to give 33.79 mg of a white solid. The mass spectrum (JMS-Q1000TD, manufactured by JEOL Ltd.) of the obtained white solid was measured and M + 1 was found to be 501. This confirmed that the white solid was the following intermediate [C].

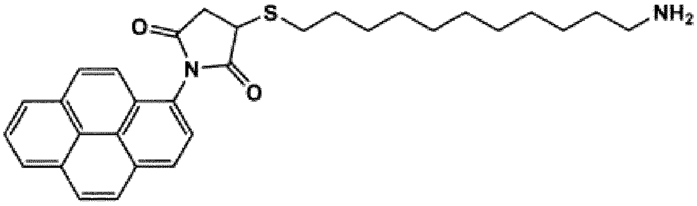

(2) Synthesis of target recognition molecule [O]

Into 50 mL of DMF, 25.01 mg of the intermediate [C] synthesized in (1) described above and 28.39 mg of Biotin-$(AC_5)_2$-OSu (trade name, manufactured by DOJINDO LABORATORIES) were dissolved. To this solution, 19.96 mg of sodium hydrogen carbonate was added and the resultant mixture was stirred for 6 hours. Thereafter, 250 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 41.90 mg of a white solid. The mass spectrum (JMS-Q1000TD, manufactured by JEOL Ltd.) of the obtained white solid was measured and M + 1 was found to be 953. This confirmed that the white solid was the following target recognition molecule [O].

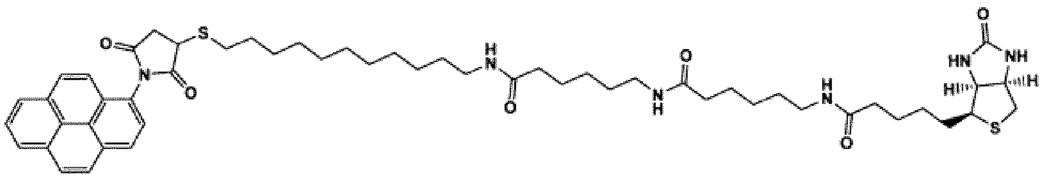

(3) Immobilization of target recognition molecule [O] to semiconducting component

The target recognition molecule [O] was attached to the CNT composite serving as the semiconducting component by the same method as the method in Comparative Example 6 (3) except that the target recognition molecule [O] was used instead of the target recognition molecule [L]. In this case, the pyrene ring of the target recognition molecule [O] is attached to CNT and thus the number of atoms from the atom bonded to the group attached to the semiconducting component to the atom bonded to the atom originated from biotin serving as the target capture body in the linking group in the target recognition molecule is 30. Moreover, the surface was protected with BSA by the same method as the method in Example 1 (5) to give a semiconductor sensor.

(4) Evaluation as semiconductor sensor

The obtained semiconductor sensor was evaluated as a semiconductor sensor by the same method as the method in Comparative Example 6 (4). The current Id at the start of the measurement was 4.02 μA and the current value was decreased by 0.14 μA only when avidin was added. This result confirmed that this semiconductor sensor selectively detected avidin. The signal intensity was 3.5%.

Comparative Example 10

(1) Synthesis of intermediate [D]

Into 100 mL of DMF, 29.73 mg of N-(1-pyrenyl)maleimide (manufactured by Tokyo Chemical Industry Co., Ltd.) and 23.92 mg of 11-amino-1-dodecanethiol (manufactured by MOLBASE) were dissolved. The resultant mixture was stirred for 12 hours with cooling with ice. Thereafter, the solvent of the reaction solution was distilled off to concentrate. Thereafter, the reaction product, unreacted substances, and impurities were separated by silica gel column chromatography using toluene as an eluent. The solvent of the fraction containing the reaction product was distilled off and the residue was dried in a vacuum to give 34.76 mg of a white solid. The mass spectrum (JMS-Q1000TD, manufactured by JEOL Ltd.) of the obtained white solid was measured and M + 1 was found to be 515. This confirmed that the white solid

was the following intermediate [D].

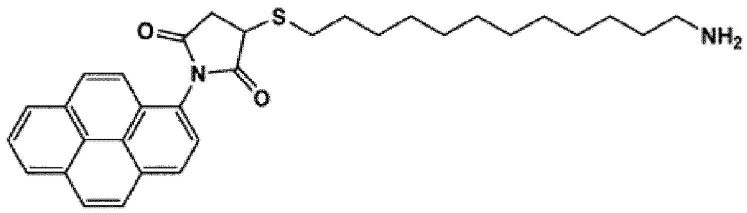

(2) Synthesis of target recognition molecule [P]

Into 50 mL of DMF, 25.71 mg of the intermediate [D] synthesized in Comparative Example 10 (1) described above and 28.39 mg of Biotin-$(AC_5)_2$-OSu (trade name, manufactured by DOJINDO LABORATORIES) were dissolved. To this solution, 19.95 mg of sodium hydrogen carbonate was added and the resultant mixture was stirred for 6 hours. Thereafter, 250 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 42.52 mg of a white solid. The mass spectrum (JMS-Q1000TD, manufactured by JEOL Ltd.) of the obtained white solid was measured and M + 1 was found to be 967. This confirmed that the white solid was the following target recognition molecule [P].

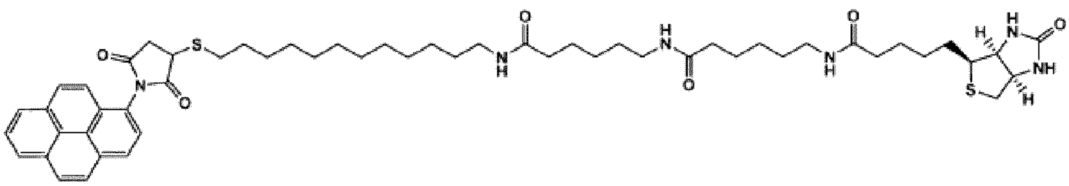

(3) Immobilization of target recognition molecule [P] to semiconducting component

The target recognition molecule [P] was attached to the CNT composite serving as the semiconducting component by the same method as the method in Comparative Example 6 (3) except that the target recognition molecule [P] was used instead of the target recognition molecule [L]. In this case, the pyrene ring of the target recognition molecule [L] is attached to CNT and thus the number of atoms from the atom bonded to the group attached to the semiconducting component to the atom bonded to the atom originated from biotin serving as the target capture body in the linking group in the target recognition molecule [P] is 31. Moreover, the surface was protected with BSA by the same method as the method in Example 1 (5) to give a semiconductor sensor.

(4) Evaluation as semiconductor sensor

The obtained semiconductor sensor was evaluated as a semiconductor sensor by the same method as the method in Comparative Example 6 (4). The current Id at the start of the measurement was 4.03 μA and the current value was decreased by 0.15 μA only when avidin was added. This result confirmed that this semiconductor sensor selectively detected avidin. The signal intensity was 3.6%.

From Comparative Examples 6 to 10, it is found that in the case where the target recognition molecule to be immobilized is a low molecule such as biotin, the effect of improving the sensitivity due to the number of atom(s) N of the linking group as observed in the present invention is not observed.

Reference Example 1

(1) Attachment of N-(1-pyrenyl)maleimide to semiconducting component and immobilization of Fab'

N-(1-Pyrenyl)maleimide (manufactured by Tokyo Chemical Industry Co., Ltd.) was attached to the CNT composite serving as the semiconducting component by the same method as the method in Example 1 (5) except that N-(1-pyrenyl)maleimide was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of N-(1-pyrenyl)maleimide is attached to CNT and thus the number of atoms from the atom bonded to the group attached to the semiconducting component to the atom bonded to

the atom originated from Fab' of the mouse anti-human HbAlc antibody serving as the target capture body in the linking group $L^2$ of the target recognition molecule being the final product is 4. Moreover, the surface was protected with BSA by the same method as the method in Example 1 (5) to give a semiconductor sensor.

(2) Evaluation as semiconductor sensor

The obtained semiconductor sensor was evaluated as a semiconductor sensor by the same method as the method in Example 1 (6). The current Id at the start of the measurement was 4.08 μA and the current value was increased by 0.31 μA only when human HbAlc was added. This result confirmed that this semiconductor sensor selectively detected human HbAlc. The signal intensity was 7.6%.

Reference Example 2

(1) Synthesis of intermediate [E]

Into 100 mL of PBS (pH 7.4), 21.73 mg of 1-aminopyrene (manufactured by Tokyo Chemical Industry Co., Ltd.) and 62.88 mg of dithiobis(succinimidylundecanoate) (manufactured by DOJINDO LABORATORIES) were dissolved and the resultant mixture was stirred for 6 hours. Thereafter, the reaction container was cooled with ice. Thereafter, the white precipitate was collected with a Kiriyama funnel, washed with pure water, and dried under reduced pressure to give 598.87 mg of a white solid. The mass spectrum (JMS-Q1000TD, manufactured by JEOL Ltd.) of the obtained white solid was measured and M + 1 was found to be 731. This confirmed that the white solid was the following intermediate [E].

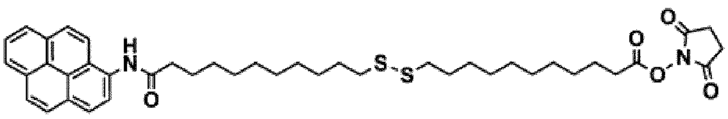

(2) Synthesis of precursor of target recognition molecule [Q]

Into 50 mL of DMF, 36.52 mg of the intermediate [E] obtained by the method in Reference Example 2 (1) and 8.83 mg of N-(2-aminoethyl)maleimide hydrochloride (manufactured by Tokyo Chemical Industry Co., Ltd.) were dissolved. To this solution, 19.96 mg of sodium hydrogen carbonate was added and the resultant mixture was stirred for 6 hours. Thereafter, 250 mL of 1 M hydrochloric acid was added dropwise. The generated white precipitate was collected by filtration with a Kiriyama funnel, washed with a saturated sodium hydrogen carbonate aqueous solution and pure water separately, and dried under reduced pressure to give 32.73 mg of a white solid. The mass spectrum (JMS-Q1000TD, manufactured by JEOL Ltd.) of the obtained white solid was measured and M + 1 was found to be 756. This confirmed that the white solid was the following target recognition molecule [Q].

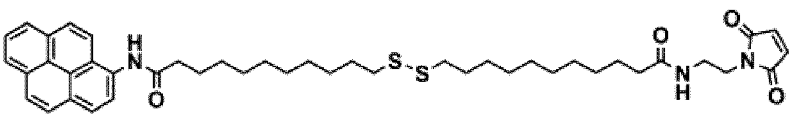

(3) Attachment of precursor of target recognition molecule [Q] to semiconducting component and immobilization of Fab'

The precursor of the target recognition molecule [Q] was attached to the CNT composite serving as the semiconducting component by the same method as the method in Example 1 (5) except that the precursor of the target recognition molecule [Q] was used instead of the precursor of the target recognition molecule [A]. Moreover, Fab' of the mouse anti-human HbAlc antibody prepared in Example 1 (2) was immobilized on the semiconductor layer by the same method as the method in Example 1 (5). In this case, the pyrene ring of the precursor of the target recognition molecule [Q] is attached to CNT and thus the number of atoms from the atom bonded to the group attached to the semiconducting component to the atom bonded to the atom originated from Fab' of the mouse anti-human HbAlc antibody serving as the target capture body in the linking group $L^2$ of the target recognition molecule being the final product is 31. Moreover, the surface was protected with BSA by the same method as the method in Example 1 (5) to give a semiconductor sensor.

(4) Evaluation as semiconductor sensor

The obtained semiconductor sensor was evaluated as a semiconductor sensor by the same method as the

method in Example 1 (6). The current Id at the start of the measurement was 4.08 μA and the current value was increased by 0.30 μA only when human HbAlc was added. This result confirmed that this semiconductor sensor selectively detected human HbAlc. The signal intensity was 7.3%.

Industrial Applicability

As described above, the semiconductor sensor, the method for manufacturing the same, and the combined sensor according to the present invention are suitable for various sensors such as a gas sensor, an ion sensor, a myocardial marker sensor, a tumor marker sensor, a hemoglobin sensor, or a glycated hemoglobin sensor, which exhibit high detection selectivity and high detection sensitivity.

Reference Signs List

1 Substrate
2 First Electrode
3 Second Electrode
4 Semiconductor Layer
5, 7 Third Electrode
6 Insulating Layer
8 Covering Member
9 Inner Space
10 Immunoglobulin
11 Heavy Chain
12 Light Chain
13 Binding Site
14 Hinge Region
15 Fab Region
16 Fc Region
17 Substructure (Fab')
18 Substructure (Reduced Immunoglobulin Half-molecule)
19 CNT
20 Substructure of Immunoglobulin
21 Sulfur Atom in Hinge Region of Substructure of Immunoglobulin

## Claims

**1.** A semiconductor sensor comprising:

a substrate;
a first electrode;
a second electrode; and
a semiconductor layer located between the first electrode and the second electrode, wherein
the semiconductor layer comprises a semiconducting component and a substructure of immunoglobulin, and
the substructure of immunoglobulin is bonded or attached to the semiconducting component through a linking group $L^1$ in a hinge region of a heavy chain.

**2.** The semiconductor sensor according to claim 1, wherein
the hinge region of the substructure of immunoglobulin contains a sulfur atom, and
the sulfur atom forms a bond to the linking group $L^1$.

**3.** The semiconductor sensor according to claim 2, wherein the linking group $L^1$ and the hinge region of the substructure of immunoglobulin forms a thioether bond through the sulfur atom.

**4.** The semiconductor sensor according to any one of claims 1 to 3, wherein the linking group $L^1$ has a substituted or unsubstituted aromatic hydrocarbon group and/or aromatic heterocyclic group.

**5.** The semiconductor sensor according to any one of claims 1 to 4, wherein
the linking group $L^1$ has a substituted or unsubstituted aromatic hydrocarbon group, and
number of carbon atoms of the aromatic hydrocarbon group not containing a substituent is 14 or more and 22 or less.

**6.** A semiconductor sensor comprising:

a substrate;
a first electrode;
a second electrode; and
a semiconductor layer located between the first electrode and the second electrode, wherein
the semiconductor layer includes a semiconducting component to which target recognition molecules are bonded or attached,
the target recognition molecule includes at least a target capture body X and a linking group $L^2$,
the target capture body X is a protein or nucleic acid having a molecular weight of 20,000 or higher and 200,000 or lower, and
number of atom(s) N from the atom bonded to the semiconducting component or from the atom bonded to the group attached to the semiconducting component to the atom bonded to the atom originated from the target capture body X in the linking group $L^2$ is 5 or more and 30 or less.

**7.** The semiconductor sensor according to claim 6, wherein
the target recognition molecule is a compound represented by a general formula (1) or a macromolecular compound having a structure represented by a general formula (2) as a repeating unit:

$$Ar^1\text{-}L^2\text{-}X \qquad (1)$$

wherein, in the general formula (1), $Ar^1$ is a substituted or unsubstituted aromatic heterocyclic group or a substituted or unsubstituted aromatic hydrocarbon group;
$L^2$ is the linking group $L^2$ and has number of atom(s) $N^1$ from the atom bonded to the atom originated from $Ar^1$ to the atom bonded to the atom originated from X of 5 or more and 30 or less; and
X is the target capture body X and is a protein or nucleic acid having a molecular weight of 20,000 or higher and 200,000 or lower,

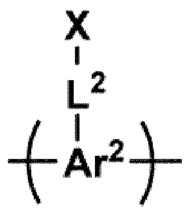

(2)

wherein, in the general formula (2), $Ar^2$ is a substituted or unsubstituted aromatic heterocyclic group or a substituted or unsubstituted aromatic hydrocarbon group;
$L^2$ is the linking group $L^2$ and has number of atom(s) $N^2$ from the atom bonded to the atom originated from $Ar^2$ to the atom bonded to the atom originated from X of 5 or more and 30 or less; and
X is the target capture body X and is a protein or nucleic acid having a molecular weight of 20,000 or higher and 200,000 or lower.

**8.** The semiconductor sensor according to claim 7, wherein
the target recognition molecule is the compound represented by the general formula (1),
$Ar^1$ is the substituted or unsubstituted aromatic hydrocarbon group in the general formula (1), and
number of carbon atoms of the aromatic hydrocarbon group not containing a substituent is 14 or more and 22 or less.

**9.** The semiconductor sensor according to claim 6, wherein the number of atom(s) N is 8 or more and 16 or less.

**10.** The semiconductor sensor according to claim 7 or 8, wherein the number of atom(s) $N^1$ and/or $N^2$ is 8 or more and 16 or less.

**11.** The semiconductor sensor according to any one of claims 6 to 10, wherein the target capture body X is immunoglobulin

or a substructure of immunoglobulin.

**12.** The semiconductor sensor according to any one of claims 6 to 11, wherein the target capture body X is a substructure of immunoglobulin.

**13.** The semiconductor sensor according to any one of claims 6 to 12, wherein the substructure of immunoglobulin forms a bond to the linking group $L^2$ in a hinge region of a heavy chain.

**14.** The semiconductor sensor according to any one of claims 1 to 5, 12, and 13, wherein the substructure of immunoglobulin is a substructure of immunoglobulin that selectively interacts with at least one of hemoglobin or glycated hemoglobin.

**15.** The semiconductor sensor according to any one of claims 1 to 5 and 12 to 14, wherein the molecular weight of the substructure of immunoglobulin is 20,000 or higher and 120,000 or lower.

**16.** The semiconductor sensor according to any one of claims 1 to 15, wherein the linking group $L^1$ and/or $L^2$ contains at least one structure selected from the group consisting of an ether bond, an amide bond, and an imide bond.

**17.** The semiconductor sensor according to any one of claims 1 to 16, wherein the linking group $L^1$ and/or $L^2$ contains a five-membered ring structure.

**18.** The semiconductor sensor according to any one of claims 1 to 17, wherein the semiconducting component is at least one component selected from the group consisting of fullerene, carbon nanotube, graphene, and carbon nanohorn.

**19.** The semiconductor sensor according to any one of claims 1 to 18, wherein a conjugated polymer is attached to at least a part of the semiconducting component.

**20.** The semiconductor sensor according to any one of claims 1 to 19, further comprising a third electrode.

**21.** The semiconductor sensor according to any one of claims 1 to 20, wherein a substance originated from a living organism is a detection target.

**22.** A combined sensor comprising:

the semiconductor sensor according to any one of claims 1 to 21; and
a sensor configured to detect glucose.

**23.** A method for producing a semiconductor sensor that comprises a substrate, a first electrode, a second electrode, and a semiconductor layer located between the first electrode and the second electrode, the method comprising:

a step of forming the semiconductor layer, wherein
the step of forming the semiconductor layer comprises a step of applying a semiconducting component between the first electrode and the second electrode.

FIG.1A

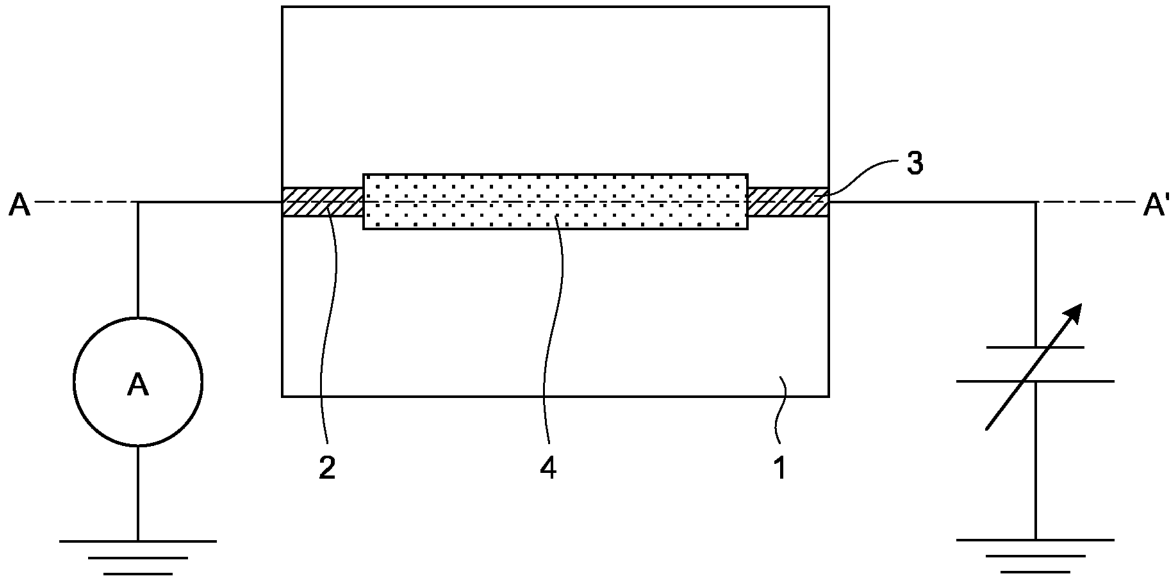


FIG.1B

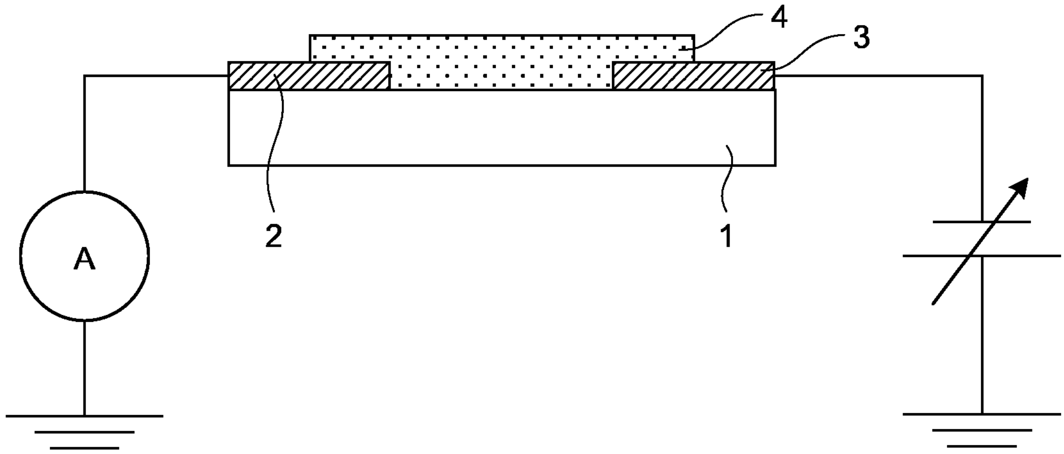

FIG.2A

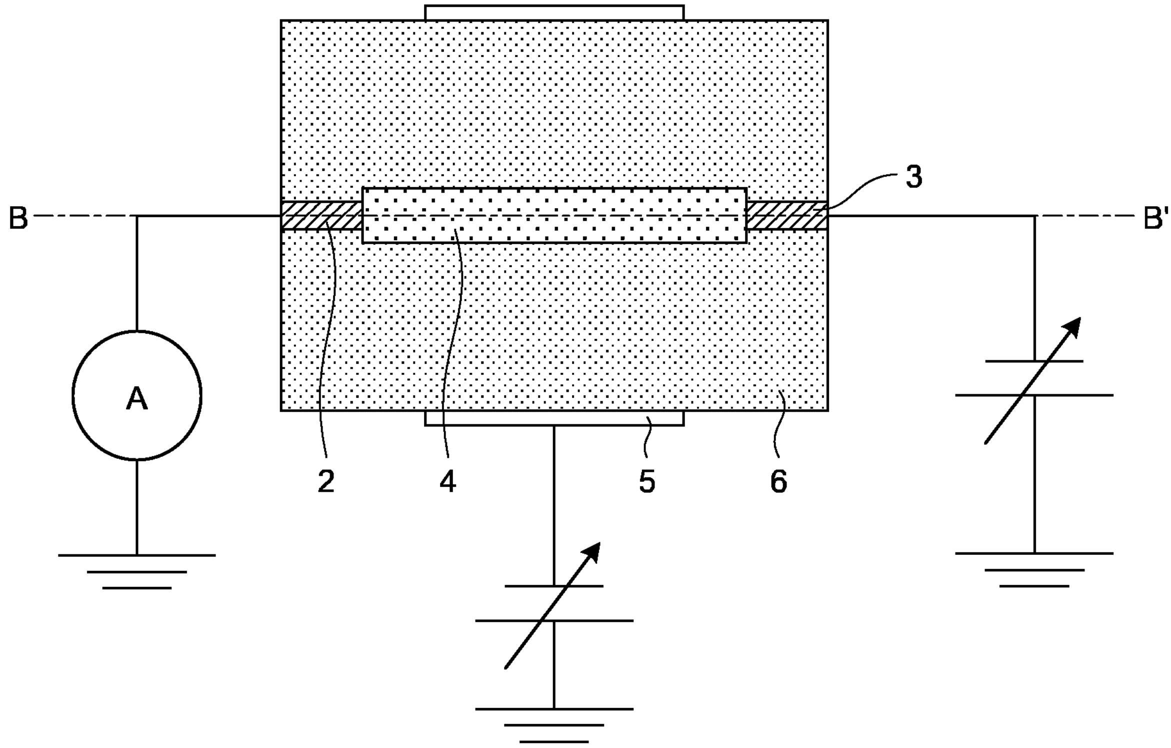


FIG.2B

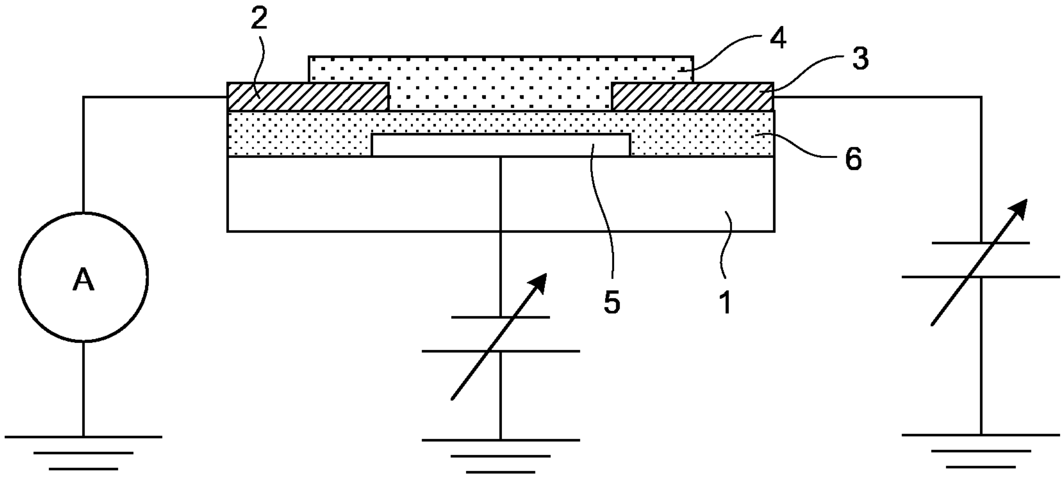

FIG.3

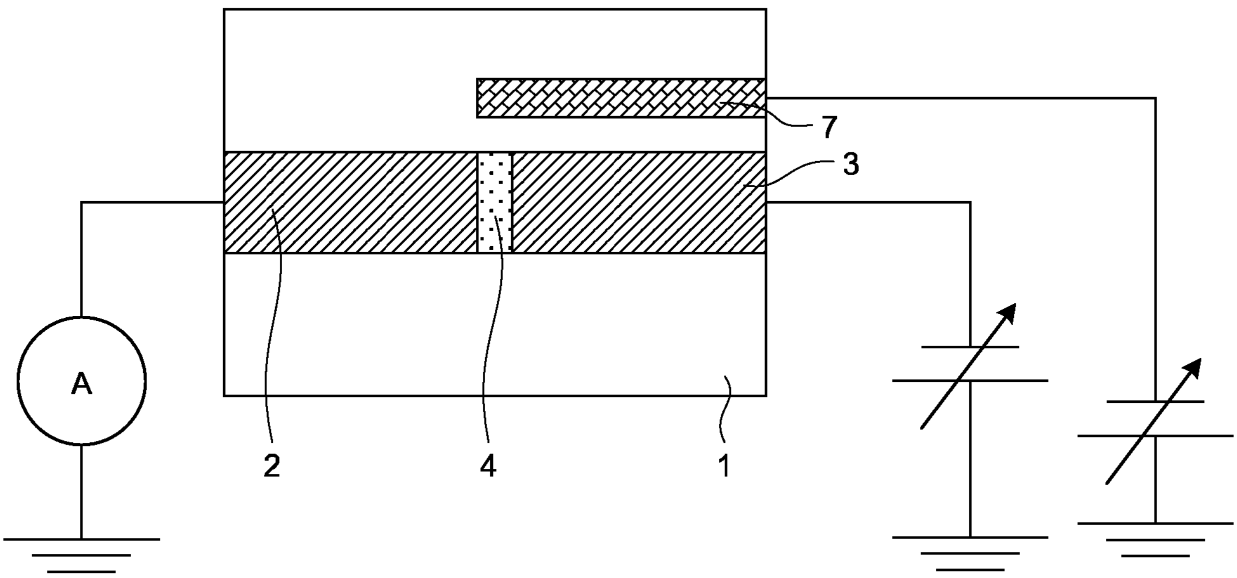

FIG.4A

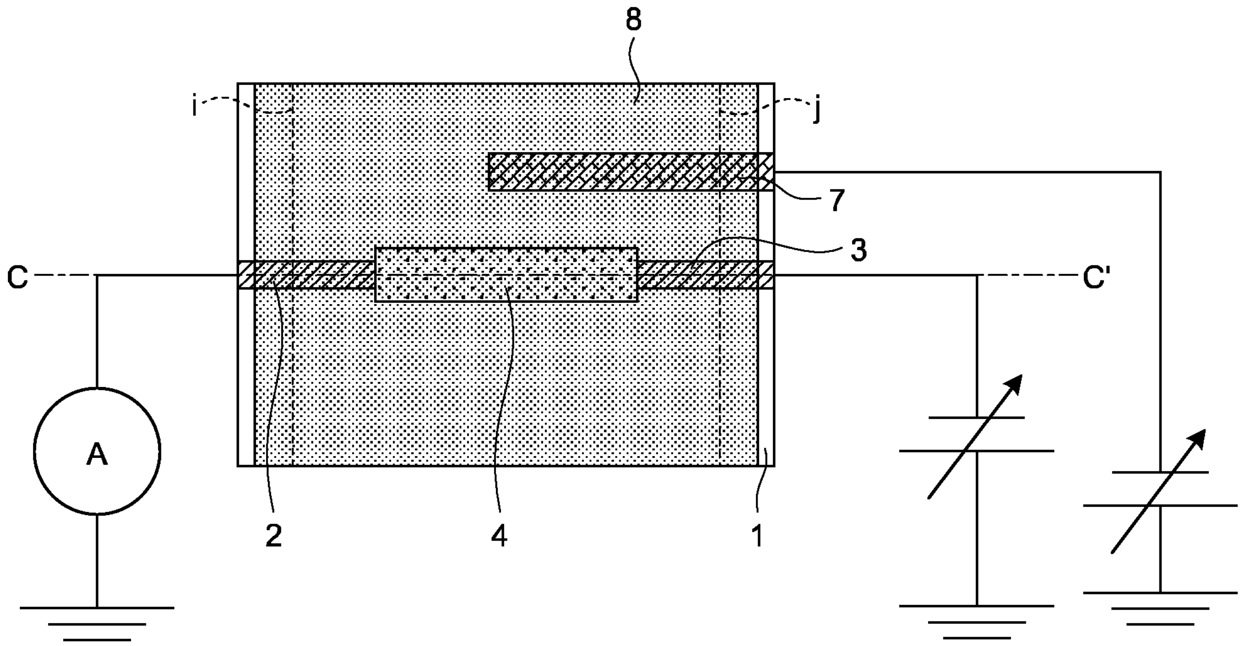


FIG.4B

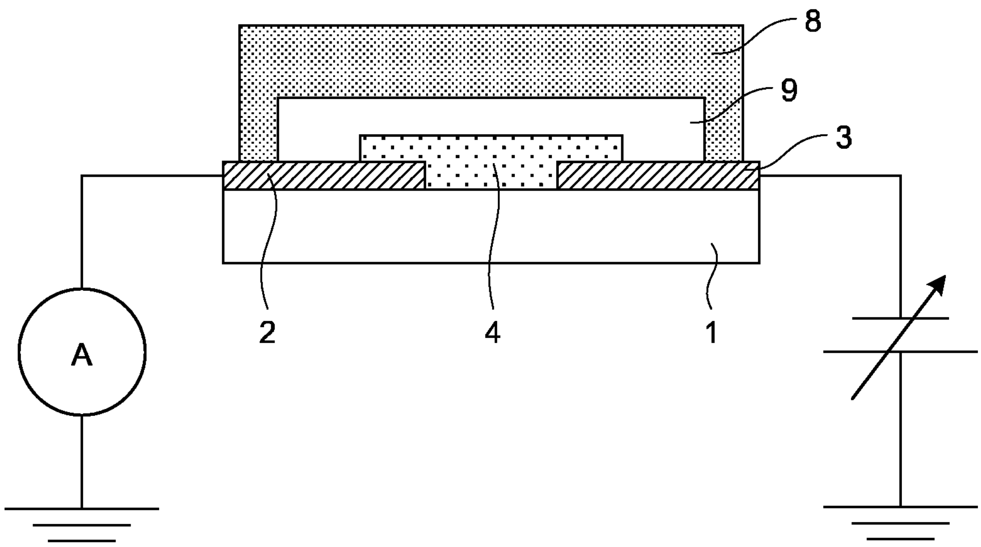

FIG.5A

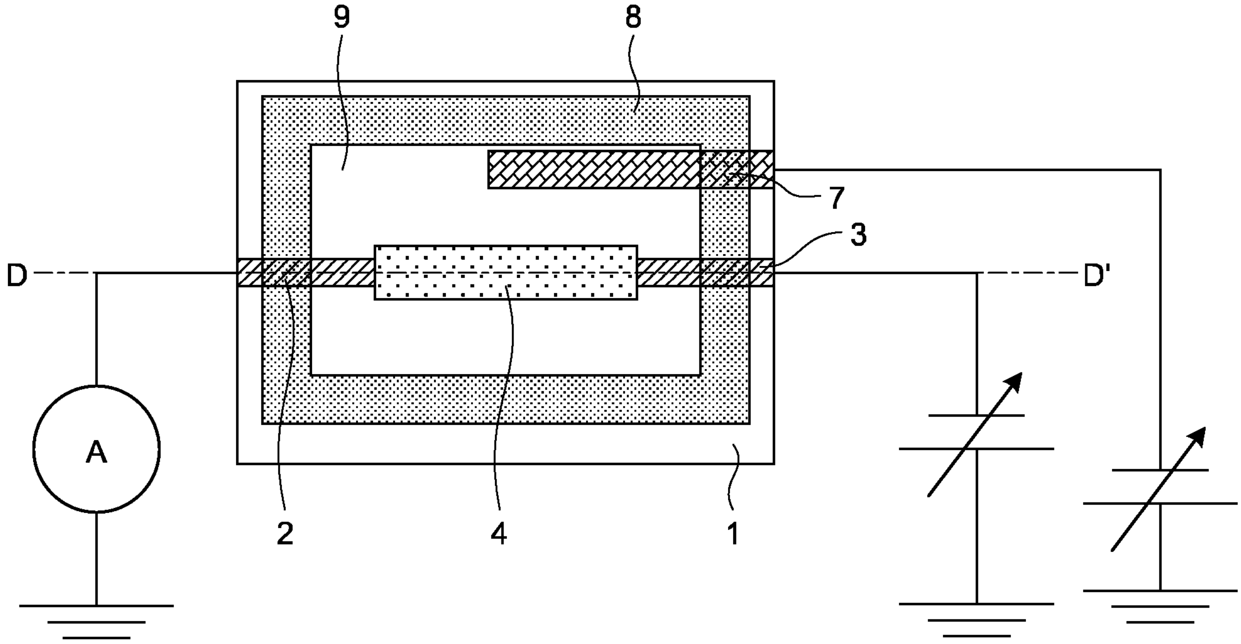


FIG.5B

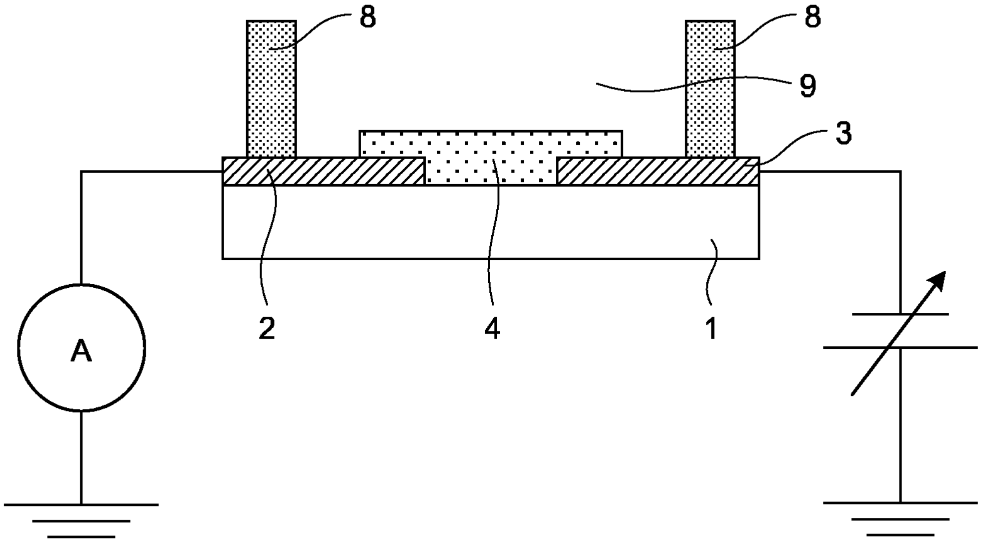

FIG.6A

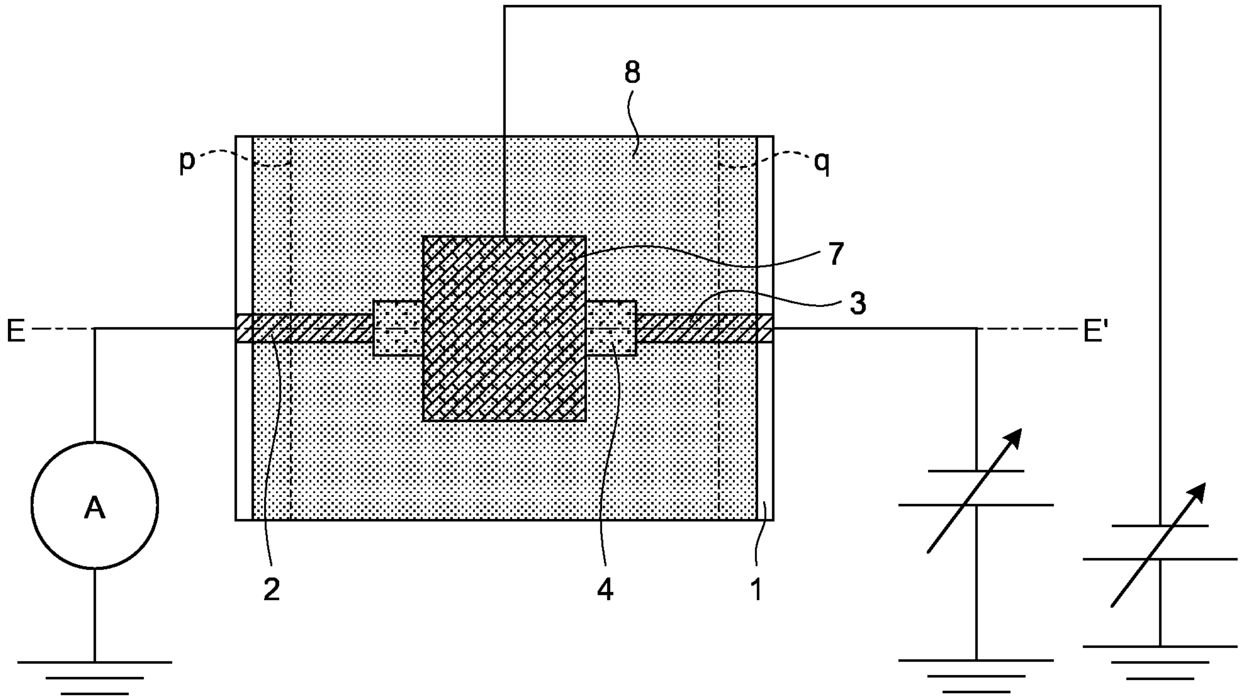


FIG.6B

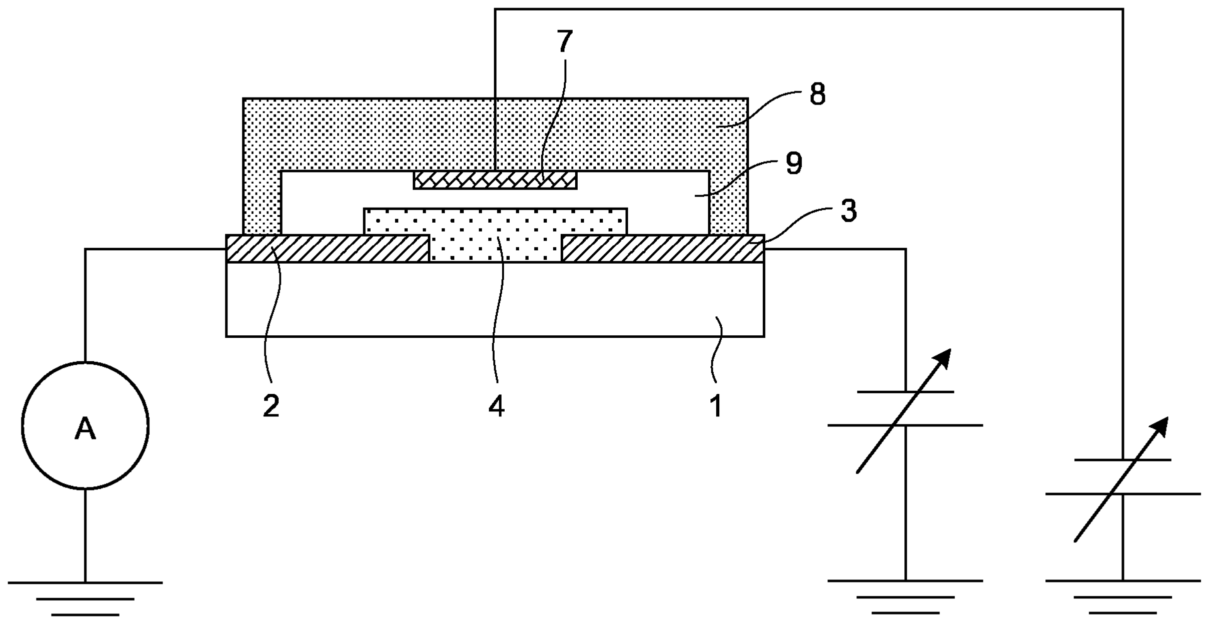

FIG.7A

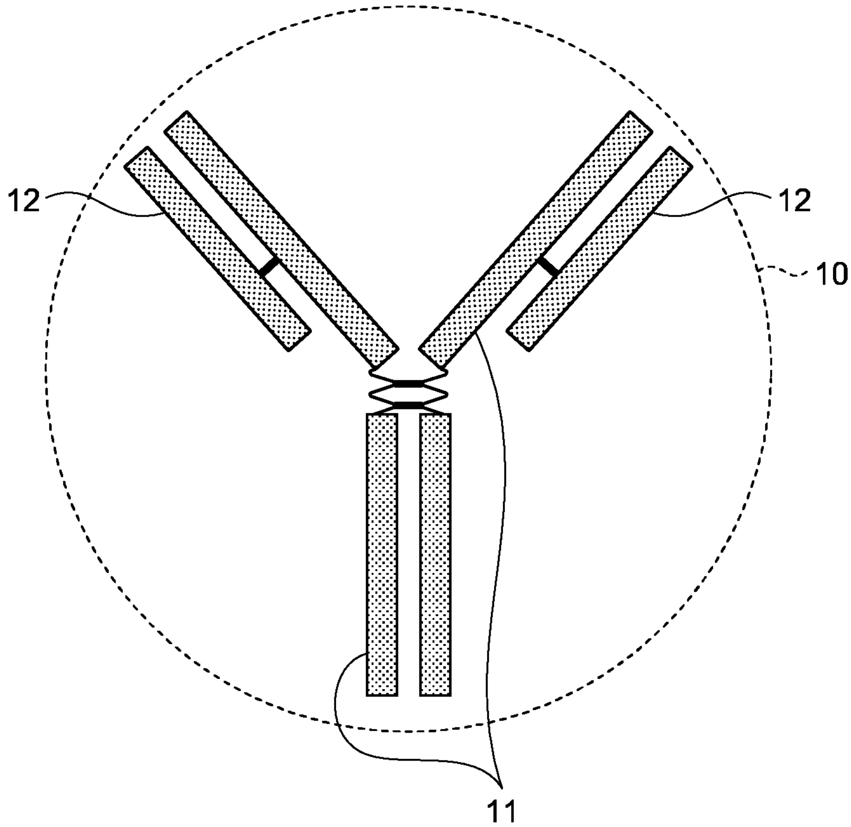


FIG.7B

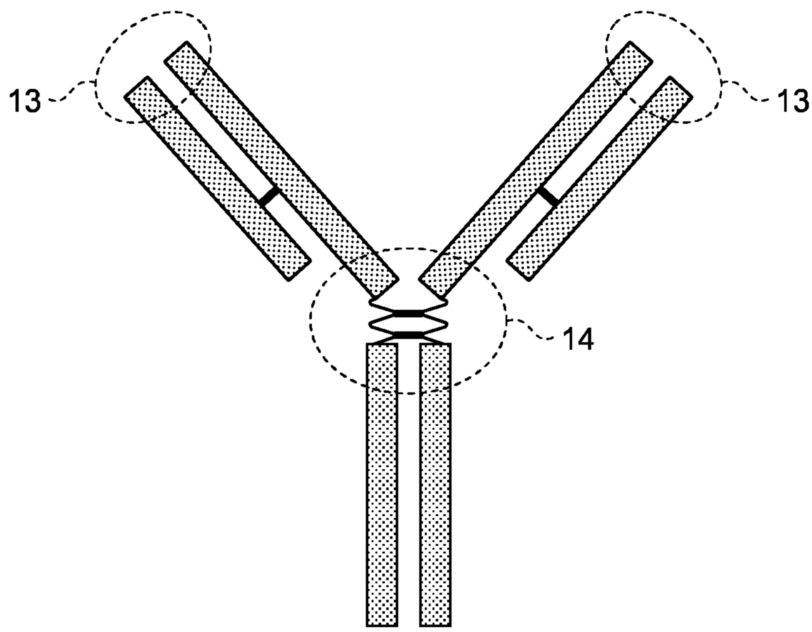

FIG.7C

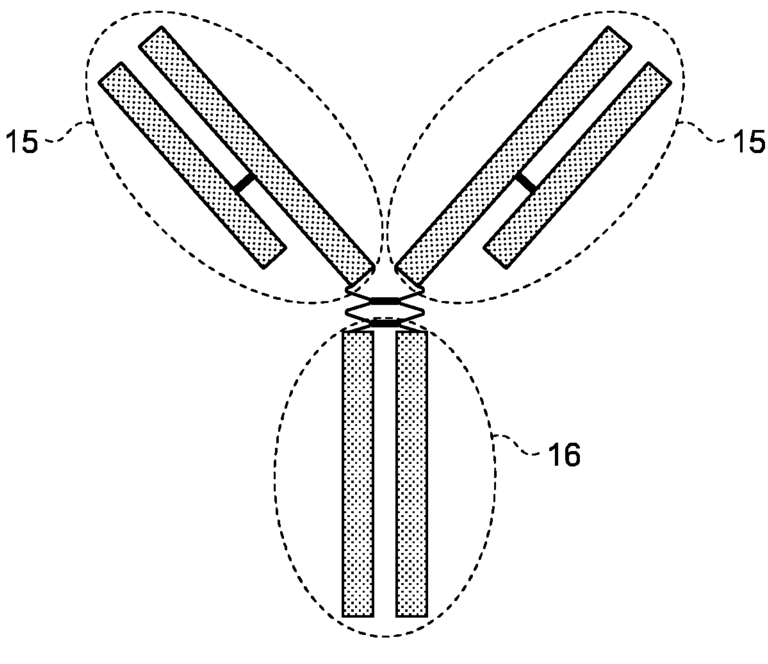

FIG.8A

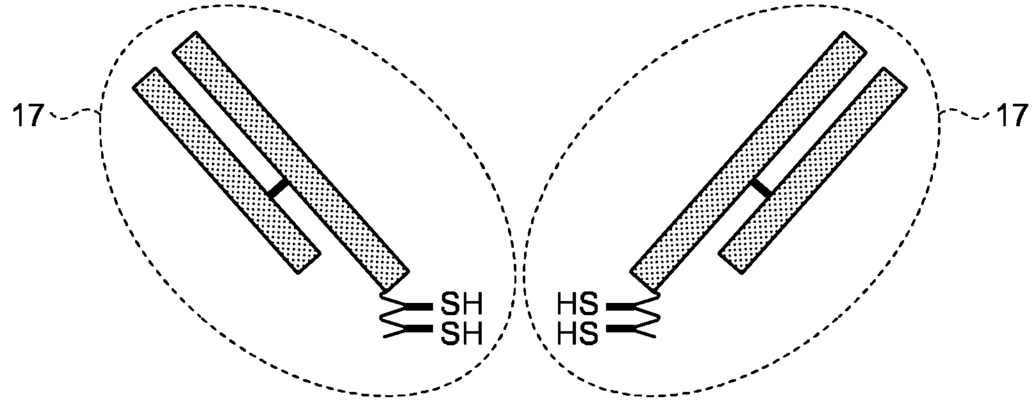


FIG.8B

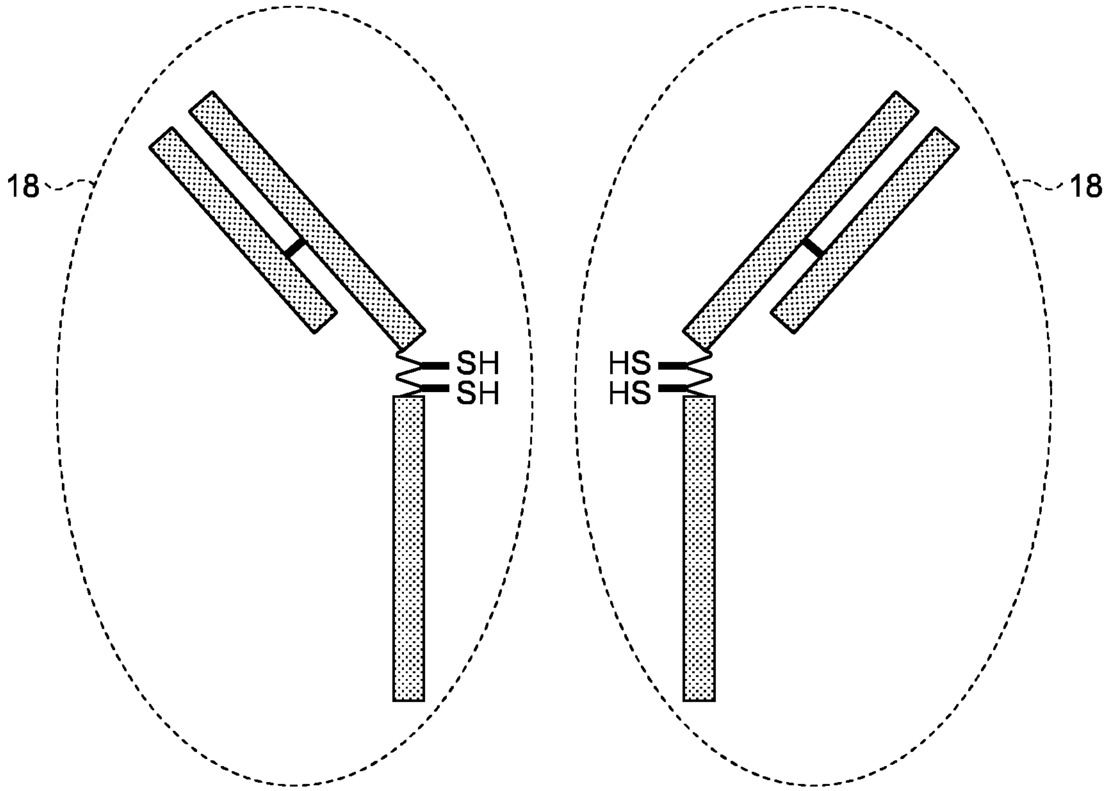

FIG.9A

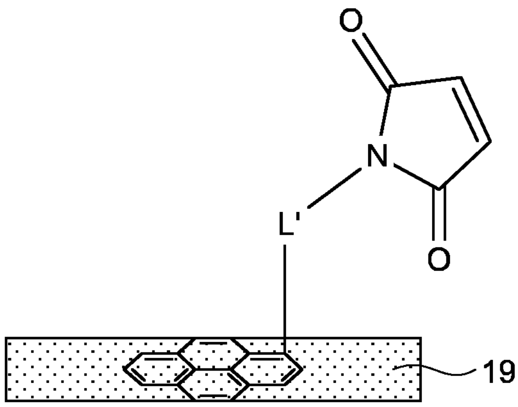

FIG.9B

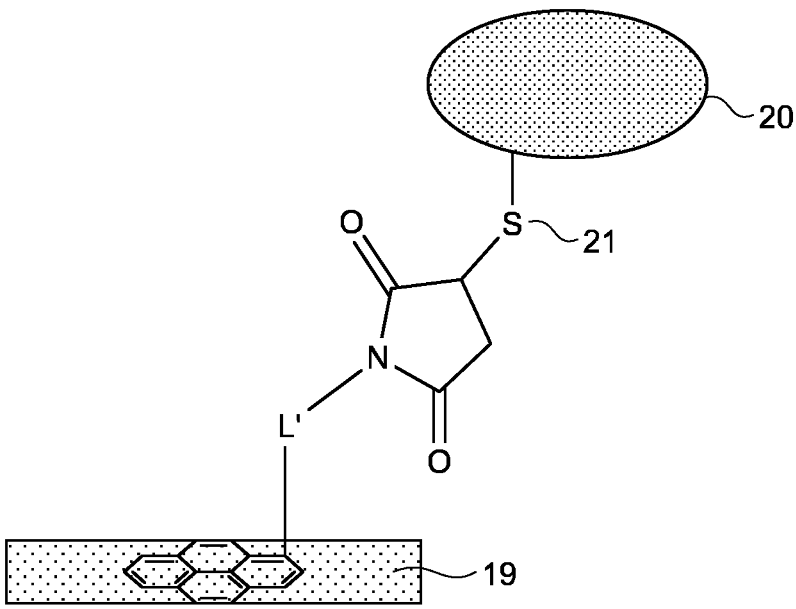

## INTERNATIONAL SEARCH REPORT

International application No. PCT/JP2017/037226

A. CLASSIFICATION OF SUBJECT MATTER
Int.Cl. G01N33/543(2006.01)i, G01N27/327(2006.01)i, G01N27/414(2006.01)i

According to International Patent Classification (IPC) or to both national classification and IPC

B. FIELDS SEARCHED

Minimum documentation searched (classification system followed by classification symbols)
Int.Cl. G01N33/543, G01N27/327, G01N27/414

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
PubMed

C. DOCUMENTS CONSIDERED TO BE RELEVANT

| Category* | Citation of document, with indication, where appropriate, of the relevant passages | Relevant to claim No. |
|---|---|---|
| Y | WO 2016/021693 A1 (NIPPON KAYAKU KK) 11 February 2016, paragraphs [0018]-[0133], [0162]-[0174], fig. 1A-18 (Family: none) | 1-8, 11-18, 20-23 |
| Y | NIWAYAMA, S. et al., A pyrene maleimide with a flexible linker for sampling of longer inter-thiol distances by excimer formation, PloS ONE, 20 October 2011, Vol. 6/No. 10/e26691, pp. 1-6 | 1-8, 11-18, 20-23 |
| Y | JP 2010-107207 A (PANASONIC CORPORATION) 13 May 2010, paragraphs [0004]-[0019], [0042], [0043] (Family: none) | 1-8, 11-18, 20-23 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 27 December 2017 | 16 January 2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2017/037226

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2009-192228 A (FUJIFILM CORPORATION) 27 August 2009, paragraphs [0015]-[0024] & US 2009/0203155 A1, paragraphs [0041]-[0050] | 1-8, 11-18, 20-23 |
| Y | WO 2011/129357 A1 (EIKEN CHEM CO., LTD.) 20 October 2011, paragraphs [0041]-[0049] & US 2013/0230897 A1, paragraphs [0052]-[0056] & EP 2560003 A1 & CN 102893151 A & KR 10-2013-0081206 A | 1-8, 11-18, 20-23 |
| X<br>Y | WO 2015/012186 A1 (TORAY INDUSTRIES) 09 December 2015, paragraphs [0015]-[0122], all drawings & US 2016/0155948 A1, paragraphs [0022]-[0133], all drawings & EP 3026014 A1 & CN 105408245 A & KR 10-2016-0033118 A | 6-7, 9-12, 14-23<br>13 |
| A | WO 2016/129444 A1 (KONICA MINOLTA, INC.) 02 February 2016 (Family: none) | 1-23 |
| A | WO 2016/111237 A1 (TOPPAN PRINTING CO., LTD.) 14 July 2016 (Family: none) | 1-23 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H2501860 PCT
- JP 2017009612 A
- WO 2006103872 A


### Non-patent literature cited in the description


- *Japanese Journal of Applied Physics (Japan),* 2012, vol. 51, 06FD08-1, 06FD08-4